# NEW EUROPEAN PATENT SPECIFICATION

(11) **EP 0 792 363 B2**
(45) Date of publication and mention of the opposition decision: **26.09.2012**
(45) Mention of the grant of the patent: 17.12.2003
(21) Application number: 95935394.7
(22) Date of filing: 27.09.1995
(51) Int. Cl.: C12N 15/32, C07K 14/325, C12N 15/62, C12Q 1/68, C12N 15/82, A01N 63/00, A01H 5/00, C12N 1/21, G01N 33/00, C07K 16/12

(54) **NOVEL PESTICIDAL PROTEINS AND STRAINS**
PESTIZID-PROTEINE UND STÄMME
NOUVELLES PROTEINES ET SOUCHES PESTICIDES

(30) Priority: 28.09.1994 US 314594; 05.06.1995 US 463483
(43) Date of publication of application: 03.09.1997
(60) Divisional application: 03022998.3
(73) Proprietor: Syngenta Participations AG, 4058 Basel (CH)
(72) Inventor: WARREN, Gregory, Wayne, Cary, NC 27513 (US); KOZIEL, Michael, Gene, Clive,IA 50325 (US); MULLINS, Martha, Alice, Cary,NC 27511 (US); NYE, Gordon, James, Raleigh,NC 27606 (US); CARR, Brian, Cary,NC 27511 (US); DESAI, Nalini, Manoj, Cary, NC 27511 (US); KOSTICHKA, Kristy, Durham, NC 27713 (US); DUCK, Nicholas, Brendan, Cary, NC 27511 (US); ESTRUCH, Juan, Jose, Durham, NC 27713 (US)
(74) Representative: Schaberg, Ulf Günther
(86) International application number: PCT/EP1995/003826
(87) International publication number: WO 1996/010083

(56) References cited:
- WO-A-88/08880
- WO-A-90/13651
- WO-A-91/16434
- WO-A-94/21795
- WO-A-95/15383
- TAYABALI A F ET AL: 'Semiautomated quantification of cytotoxic damage induced in cultured insect cells exposed to commercial Bacillus thuringiensis biopesticides.' JOURNAL OF APPLIED TOXICOLOGY vol. 15, no. 5, pages 365 - 373, XP008089640
- SEKAR V: 'THE INSECTICIDAL CRYSTAL PROTEIN GENE IS EXPRESSED IN VEGETATIVE CELLS OF BACILLUS - THURINGIENSIS -VAR-TENEBRIONIS.' CURR MICROBIOL vol. 17, no. 6, pages 347 - 350, XP008027416
- WALTHER, COREY J. ET AL: 'Analysis of mosquito larvicidal potential exhibited by vegetative cells of Bacillus thuringiensis subsp. israelensis' APPL. ENVIRON. MICROBIOL. vol. 52, no. 4, 1986, pages 650 - 653, XP008027417
- WARD E S ET AL: 'BACILLUS - THURINGIENSIS -VAR-ISRAELENSIS DELTA ENDOTOXIN CLONING AND EXPRESSION OF THE TOXIN IN SPOROGENIC AND ASPOROGENIC STRAINS OF BACILLUS -SUBTILIS.' J MOL BIOL vol. 191, no. 1, 1986, pages 13 - 22, XP009005020
- M.G. KOZIEL ET AL.: 'Field performance of elite transgenic maize plants expressing an insecticidal protein derived from Bacillus thuringiensis' BIOTECHNOLOGY vol. 11, pages 194 - 200, XP002025246
- H. YOSHISUE: 'Effects of Bacillus thuringiensis var. israelensis 20-kDa protein on production of Bti 130-kDa crystal protein in Escherichia coli.' BIOSCIENCE, BIOTECHNOLOGY AND BIOCHEMISTRY vol. 56, no. 9, pages 1429 - 1433, XP000322466
- A.S. SHIVAKUMAR ET AL.: 'Cloned crystal protein genes express vegetatively in Bacillus subtilis.' PLASMID vol. 16, no. 3, page 230, XP008090749
- T. THANABALU ET AL: 'Proteolytic processing of the mosquitocidal toxin from Bacillus sphaericus SSII-1' JOURNAL OF BACTERIOLOGY vol. 174, no. 15, pages 5051 - 5056, XP000764305
- T. THANABALU ET AL: 'Cytotoxicity and ADP-Ribosylating activity of the mosquitocidal toxin from Bacillus sphaericus SSII-1: possible roles of the 27- and 70-kilodalton peptides.' JOURNAL OF BACTERIOLOGY vol. 175, no. 8, pages 2314 - 2320, XP000764304
- CRICKMORE N. ET AL: 'Revision of the nomenclature for Bacillus thuringiensis pesticidal crystal proteins' MICROBIOLOGY AND MOLEC.BIOL.REVIEW vol. 62, no. 3, 01 September 1998, pages 807 - 813
- THANABALU ET AL: 'Cloning, sequencing, and expression of a gene encoding a 100-Kilodalton mosquitocidal toxin from Bacillus sphaericus SSII-1' JOURNAL OF BACTERIOLOGY vol. 173, no. 9, 01 May 1991, pages 2776 - 2785
- FAUST, BULLA: 'E. Kursmak Ed. Marcel Decker Pub.' MICROBIAL AND VIRAL PESTIDICES 01 January 1982, NEW YORK, pages 108 - 112
- BOSSÉ M. ET AL: 'Nucleotide sequence of a novel crystal protein gene isolated from Bacillus thuringiensis subspecies kenyae' NUCLEIC ACIDS RESEARCH vol. 18, no. 4, 01 January 1990, page 7443
- DARDENNE F. ET AL: 'Nucleotide sequence and deduced amino acid sequence of a cryIA(c) gene variant from Bacillus thuringiensis' NUCLEIC ACIDS RESEARCH vol. 18, no. 18, 01 January 1990, page 5546
- HAIDER M.Z. AND ELLAR D.J.: 'Nucleotide sequence of a Bacillus thuringiensis aizawai IC1 entomocidal crystal protein gene.' NUCLEIC ACIDS RESEARCH vol. 16, no. 22, 01 January 1998, page 10927
- Sequence Alignment Global DNA alignment against reference molecule

### SEQUENCE LISTING

(1) GENERAL INFORMATION:
   (A) NAME: SYNGENTA PARTICIPATIONS AG
   (B) STREET:
   (C) CITY: Basel
   (E) COUNTRY: Switzerland
   (F) POSTAL CODE (ZIP): 4002
   (G) TELEPHONE:
   (H) TELEFAX: + 41 61 323 50 44
   (I) TELEX:

   (ii) TITLE OF INVENTION: Novel Pesticidal Proteins and Strains
   (iii) NUMBER OF SEQUENCES: 7
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30B
(2) INFORMATION FOR SEQ ID NO:1:
   (i) SEQUENCE CHARACTERISTICS:
      (A) LENGTH: 2378 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (ix) FEATURE:
      (A) NAME/KEY: CDS
      (B) LOCATION: 9..2375
      (D) OTHER INFORMATION: /note= "Native DNA sequence encoding VIP3A(a) protein from AB88 as contained in pCIB7104"
   (xi) SEQUENCE DESCRIPTION: SEQ ID NO:1:
   AGATGAAC ATG AAC AAG AAT AAT ACT AAA TTA AGC ACA AGA GCC TTA CCA        50
            Met Asn Lys Asn Asn Thr Lys Leu Ser Thr Arg Ala Leu Pro
              1               5                  10
   AGT TTT ATT GAT TAT TTT AAT GGC ATT TAT GGA TTT GCC ACT GGT ATC         98
   Ser Phe Ile Asp Tyr Phe Asn Gly Ile Tyr Gly Phe Ala Thr Gly Ile
    15                  20                  25                  30
   AAA GAC ATT ATG AAC ATG ATT TTT AAA ACG GAT ACA GGT GGT GAT CTA        146
   Lys Asp Ile Met Asn Met Ile Phe Lys Thr Asp Thr Gly Gly Asp Leu
                   35                  40                  45
   ACC CTA GAC GAA ATT TTA AAG AAT CAG CAG TTA CTA AAT GAT ATT TCT        194
   Thr Leu Asp Glu Ile Leu Lys Asn Gln Gln Leu Leu Asn Asp Ile Ser
               50                  55                  60
   GGT AAA TTG GAT GGG GTG AAT GGA AGC TTA AAT GAT CTT ATC GCA CAG        242
   Gly Lys Leu Asp Gly Val Asn Gly Ser Leu Asn Asp Leu Ile Ala Gln
           65                  70                  75
   GGA AAC TTA AAT ACA GAA TTA TCT AAG GAA ATA TTA AAA ATT GCA AAT        290
   Gly Asn Leu Asn Thr Glu Leu Ser Lys Glu Ile Leu Lys Ile Ala Asn
       80                  85                  90
   GAA CAA AAT CAA GTT TTA AAT GAT GTT AAT AAC AAA CTC GAT GCG ATA        338
   Glu Gln Asn Gln Val Leu Asn Asp Val Asn Asn Lys Leu Asp Ala Ile
    95                 100                 105                 110
   AAT ACG ATG CTT CGG GTA TAT CTA CCT AAA ATT ACC TCT ATG TTG AGT        386
   Asn Thr Met Leu Arg Val Tyr Leu Pro Lys Ile Thr Ser Met Leu Ser
                   115                 120                 125
   GAT GTA ATG AAA CAA AAT TAT GCG CTA AGT CTG CAA ATA GAA TAC TTA        434
   Asp Val Met Lys Gln Asn Tyr Ala Leu Ser Leu Gln Ile Glu Tyr Leu
               130                 135                 140
   AGT AAA CAA TTG CAA GAG ATT TCT GAT AAG TTG GAT ATT ATT AAT GTA        482
   Ser Lys Gln Leu Gln Glu Ile Ser Asp Lys Leu Asp Ile Ile Asn Val
           145                 150                 155
   AAT GTA CTT ATT AAC TCT ACA CTT ACT GAA ATT ACA CCT GCG TAT CAA        530
   Asn Val Leu Ile Asn Ser Thr Leu Thr Glu Ile Thr Pro Ala Tyr Gln
       160                 165                 170
   AGG ATT AAA TAT GTG AAC GAA AAA TTT GAG GAA TTA ACT TTT GCT ACA        578
   Arg Ile Lys Tyr Val Asn Glu Lys Phe Glu Glu Leu Thr Phe Ala Thr
   175                 180                 185                 190
   GAA ACT AGT TCA AAA GTA AAA AAG GAT GGC TCT CCT GCA GAT ATT CTT        626
   Glu Thr Ser Ser Lys Val Lys Lys Asp Gly Ser Pro Ala Asp Ile Leu
                   195                 200                 205
   GAT GAG TTA ACT GAG TTA ACT GAA CTA GCG AAA AGT GTA ACA AAA AAT        674
   Asp Glu Leu Thr Glu Leu Thr Glu Leu Ala Lys Ser Val Thr Lys Asn
               210                 215                 220
   GAT GTG GAT GGT TTT GAA TTT TAC CTT AAT ACA TTC CAC GAT GTA ATG        722
   Asp Val Asp Gly Phe Glu Phe Tyr Leu Asn Thr Phe His Asp Val Met
           225                 230                 235
   GTA GGA AAT AAT TTA TTC GGG CGT TCA GCT TTA AAA ACT GCA TCG GAA        770
   Val Gly Asn Asn Leu Phe Gly Arg Ser Ala Leu Lys Thr Ala Ser Glu
       240                 245                 250
   TTA ATT ACT AAA GAA AAT GTG AAA ACA AGT GGC AGT GAG GTC GGA AAT        818
   Leu Ile Thr Lys Glu Asn Val Lys Thr Ser Gly Ser Glu Val Gly Asn
   255                 260                 265                 270
   GTT TAT AAC TTC TTA ATT GTA TTA ACA GCT CTG CAA GCC CAA GCT TTT        866
   Val Tyr Asn Phe Leu Ile Val Leu Thr Ala Leu Gln Ala Gln Ala Phe
                   275                 280                 285
   CTT ACT TTA ACA ACA TGC CGA AAA TTA TTA GGC TTA GCA GAT ATT GAT        914
   Leu Thr Leu Thr Thr Cys Arg Lys Leu Leu Gly Leu Ala Asp Ile Asp
               290                 295                 300
   TAT ACT TCT ATT ATG AAT GAA CAT TTA AAT AAG GAA AAA GAG GAA TTT        962
   Tyr Thr Ser Ile Met Asn Glu His Leu Asn Lys Glu Lys Glu Glu Phe
               305                 310                 315
   AGA GTA AAC ATC CTC CCT ACA CTT TCT AAT ACT TTT TCT AAT CCT AAT     1010
   Arg Val Asn Ile Leu Pro Thr Leu Ser Asn Thr Phe Ser Asn Pro Asn
       320                 325                 330
   TAT GCA AAA GTT AAA GGA AGT GAT GAA GAT GCA AAG ATG ATT GTG GAA     1058
   Tyr Ala Lys Val Lys Gly Ser Asp Glu Asp Ala Lys Met Ile Val Glu
   335                 340                 345                 350
   GCT AAA CCA GGA CAT GCA TTG ATT GGG TTT GAA ATT AGT AAT GAT TCA     1106
   Ala Lys Pro Gly His Ala Leu Ile Gly Phe Glu Ile Ser Asn Asp Ser
                   355                 360                 365
   ATT ACA GTA TTA AAA GTA TAT GAG GCT AAG CTA AAA CAA AAT TAT CAA     1154
   Ile Thr Val Leu Lys Val Tyr Glu Ala Lys Leu Lys Gln Asn Tyr Gln
               370                 375                 380
   GTC GAT AAG GAT TCC TTA TCG GAA GTT ATT TAT GGT GAT ATG GAT AAA     1202
   Val Asp Lys Asp Ser Leu Ser Glu Val Ile Tyr Gly Asp Met Asp Lys
           385                 390                 395
   TTA TTG TGC CCA GAT CAA TCT GAA CAA ATC TAT TAT ACA AAT AAC ATA     1250
   Leu Leu Cys Pro Asp Gln Ser Glu Gln Ile Tyr Tyr Thr Asn Asn Ile
       400                 405                 410
   GTA TTT CCA AAT GAA TAT GTA ATT ACT AAA ATT GAT TTC ACT AAA AAA     1298
   Val Phe Pro Asn Glu Tyr Val Ile Thr Lys Ile Asp Phe Thr Lys Lys
   415                 420                 425                 430
   ATG AAA ACT TTA AGA TAT GAG GTA ACA GCG AAT TTT TAT GAT TCT TCT     1346
   Met Lys Thr Leu Arg Tyr Glu Val Thr Ala Asn Phe Tyr Asp Ser Ser
                   435                 440                 445
   ACA GGA GAA ATT GAC TTA AAT AAG AAA AAA GTA GAA TCA AGT GAA GCG     1394
   Thr Gly Glu Ile Asp Leu Asn Lys Lys Lys Val Glu Ser Ser Glu Ala
               450                 455                 460
   GAG TAT AGA ACG TTA AGT GCT AAT GAT GAT GGG GTG TAT ATG CCG TTA     1442
   Glu Tyr Arg Thr Leu Ser Ala Asn Asp Asp Gly Val Tyr Met Pro Leu
           465                 470                 475
   GGT GTC ATC AGT GAA ACA TTT TTG ACT CCG ATT AAT GGG TTT GGC CTC     1490
   Gly Val Ile Ser Glu Thr Phe Leu Thr Pro Ile Asn Gly Phe Gly Leu
       480                 485                 490
   CAA GCT GAT GAA AAT TCA AGA TTA ATT ACT TTA ACA TGT AAA TCA TAT     1538
   Gln Ala Asp Glu Asn Ser Arg Leu Ile Thr Leu Thr Cys Lys Ser Tyr
   495                 500                 505                 510
   TTA AGA GAA CTA CTG CTA GCA ACA GAC TTA AGC AAT AAA GAA ACT AAA     1586
   Leu Arg Glu Leu Leu Leu Ala Thr Asp Leu Ser Asn Lys Glu Thr Lys
                   515                 520                 525
   TTG ATC GTC CCG CCA AGT GGT TTT ATT AGC AAT ATT GTA GAG AAC GGG     1634
   Leu Ile Val Pro Pro Ser Gly Phe Ile Ser Asn Ile Val Glu Asn Gly
               530                 535                 540
   TCC ATA GAA GAG GAC AAT TTA GAG CCG TGG AAA GCA AAT AAT AAG AAT      1682
   Ser Ile Glu Glu Asp Asn Leu Glu Pro Trp Lys Ala Asn Asn Lys Asn
           545                 550                 555
   GCG TAT GTA GAT CAT ACA GGC GGA GTG AAT GGA ACT AAA GCT TTA TAT      1730
   Ala Tyr Val Asp His Thr Gly Gly Val Asn Gly Thr Lys Ala Leu Tyr
       560                 565                 570
   GTT CAT AAG GAC GGA GGA ATT TCA CAA TTT ATT GGA GAT AAG TTA AAA      1778
   Val His Lys Asp Gly Gly Ile Ser Gln Phe Ile Gly Asp Lys Leu Lys
   575                 580                 585                 590
   CCG AAA ACT GAG TAT GTA ATC CAA TAT ACT GTT AAA GGA AAA CCT TCT      1826
   Pro Lys Thr Glu Tyr Val Ile Gln Tyr Thr Val Lys Gly Lys Pro Ser
                   595                 600                 605
   ATT CAT TTA AAA GAT GAA AAT ACT GGA TAT ATT CAT TAT GAA GAT ACA      1874
   Ile His Leu Lys Asp Glu Asn Thr Gly Tyr Ile His Tyr Glu Asp Thr
               610                 615                 620
   AAT AAT AAT TTA GAA GAT TAT CAA ACT ATT AAT AAA CGT TTT ACT ACA      1922
   Asn Asn Asn Leu Glu Asp Tyr Gln Thr Ile Asn Lys Arg Phe Thr Thr
           625                 630                 635
   GGA ACT GAT TTA AAG GGA GTG TAT TTA ATT TTA AAA AGT CAA AAT GGA      1970
   Gly Thr Asp Leu Lys Gly Val Tyr Leu Ile Leu Lys Ser Gln Asn Gly
       640                 645                 650
   GAT GAA GCT TGG GGA GAT AAC TTT ATT ATT TTG GAA ATT AGT CCT TCT      2018
   Asp Glu Ala Trp Gly Asp Asn Phe Ile Ile Leu Glu Ile Ser Pro Ser
   655                 660                 665                 670
   GAA AAG TTA TTA AGT CCA GAA TTA ATT AAT ACA AAT AAT TGG ACG AGT      2066
   Glu Lys Leu Leu Ser Pro Glu Leu Ile Asn Thr Asn Asn Trp Thr Ser
                   675                 680                 685
   ACG GGA TCA ACT AAT ATT AGC GGT AAT ACA CTC ACT CTT TAT CAG GGA      2114
   Thr Gly Ser Thr Asn Ile Ser Gly Asn Thr Leu Thr Leu Tyr Gln Gly
               690                 695                 700
   GGA CGA GGG ATT CTA AAA CAA AAC CTT CAA TTA GAT AGT TTT TCA ACT      2162
   Gly Arg Gly Ile Leu Lys Gln Asn Leu Gln Leu Asp Ser Phe Ser Thr
           705                 710                 715
   TAT AGA GTG TAT TTT TCT GTG TCC GGA GAT GCT AAT GTA AGG ATT AGA      2210
   Tyr Arg Val Tyr Phe Ser Val Ser Gly Asp Ala Asn Val Arg Ile Arg
       720                 725                 730
   AAT TCT AGG GAA GTG TTA TTT GAA AAA AGA TAT ATG AGC GGT GCT AAA      2258
   Asn Ser Arg Glu Val Leu Phe Glu Lys Arg Tyr Met Ser Gly Ala Lys
   735                 740                 745                 750
   GAT GTT TCT GAA ATG TTC ACT ACA AAA TTT GAG AAA GAT AAC TTT TAT      2306
   Asp Val Ser Glu Met Phe Thr Thr Lys Phe Glu Lys Asp Asn Phe Tyr
                   755                 760                 765
   ATA GAG CTT TCT CAA GGG AAT AAT TTA TAT GGT GGT CCT ATT GTA CAT      2354
   Ile Glu Leu Ser Gln Gly Asn Asn Leu Tyr Gly Gly Pro Ile Val His
               770                 775                 780
   TTT TAC GAT GTC TCT ATT AAG TAA                                      2378
   Phe Tyr Asp Val Ser Ile Lys
           785
(2) INFORMATION FOR SEQ ID NO:2:
   (i) SEQUENCE CHARACTERISTICS:
      (A) LENGTH: 789 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Asn Lys Asn Asn Thr Lys Leu Ser Thr Arg Ala Leu Pro Ser Phe
    1               5                  10                  15
   Ile Asp Tyr Phe Asn Gly Ile Tyr Gly Phe Ala Thr Gly Ile Lys Asp
               20                  25                  30
   Ile Met Asn Met Ile Phe Lys Thr Asp Thr Gly Gly Asp Leu Thr Leu
           35                  40                  45
   Asp Glu Ile Leu Lys Asn Gln Gln Leu Leu Asn Asp Ile Ser Gly Lys
       50                  55                  60
   Leu Asp Gly Val Asn Gly Ser Leu Asn Asp Leu Ile Ala Gln Gly Asn
   65                  70                  75                  80
   Leu Asn Thr Glu Leu Ser Lys Glu Ile Leu Lys Ile Ala Asn Glu Gln
                   85                  90                  95
   Asn Gln Val Leu Asn Asp Val Asn Asn Lys Leu Asp Ala Ile Asn Thr
               100                 105                 110
   Met Leu Arg Val Tyr Leu Pro Lys Ile Thr Ser Met Leu Ser Asp Val
           115                 120                 125
   Met Lys Gln Asn Tyr Ala Leu Ser Leu Gln Ile Glu Tyr Leu Ser Lys
       130                 135                 140
   Gln Leu Gln Glu Ile Ser Asp Lys Leu Asp Ile Ile Asn Val Asn Val
   145                 150                 155                 160
   Leu Ile Asn Ser Thr Leu Thr Glu Ile Thr Pro Ala Tyr Gln Arg Ile
                   165                 170                 175
   Lys Tyr Val Asn Glu Lys Phe Glu Glu Leu Thr Phe Ala Thr Glu Thr
   180 185 190
   Ser Ser Lys Val Lys Lys Asp Gly Ser Pro Ala Asp Ile Leu Asp Glu
   195 200 205
   Leu Thr Glu Leu Thr Glu Leu Ala Lys Ser Val Thr Lys Asn Asp Val
   210 215 220
   Asp Gly Phe Glu Phe Tyr Leu Asn Thr Phe His Asp Val Met Val Gly
   225 230 235 240
   Asn Asn Leu Phe Gly Arg Ser Ala Leu Lys Thr Ala Ser Glu Leu Ile
   245 250 255
   Thr Lys Glu Asn Val Lys Thr Ser Gly Ser Glu Val Gly Asn Val Tyr
   260 265 270
   Asn Phe Leu Ile Val Leu Thr Ala Leu Gln Ala Gln Ala Phe Leu Thr
   275 280 285
   Leu Thr Thr Cys Arg Lys Leu Leu Gly Leu Ala Asp Ile Asp Tyr Thr
   290 295 300
   Ser Ile Met Asn Glu His Leu Asn Lys Glu Lys Glu Glu Phe Arg Val
   305 310 315 320
   Asn Ile Leu Pro Thr Leu Ser Asn Thr Phe Ser Asn Pro Asn Tyr Ala
   325 330 335
   Lys Val Lys Gly Ser Asp Glu Asp Ala Lys Met Ile Val Glu Ala Lys
   340 345 350
   Pro Gly His Ala Leu Ile Gly Phe Glu Ile Ser Asn Asp Ser Ile Thr
   355 360 365
   Val Leu Lys Val Tyr Glu Ala Lys Leu Lys Gln Asn Tyr Gln Val Asp
   370 375 380
   Lys Asp Ser Leu Ser Glu Val Ile Tyr Gly Asp Met Asp Lys Leu Leu
   385 390 395 400
   Cys Pro Asp Gln Ser Glu Gln Ile Tyr Tyr Thr Asn Asn Ile Val Phe
   405 410 415
   Pro Asn Glu Tyr Val Ile Thr Lys Ile Asp Phe Thr Lys Lys Met Lys
   420 425 430
   Thr Leu Arg Tyr Glu Val Thr Ala Asn Phe Tyr Asp Ser Ser Thr Gly
   435 440 445
   Glu Ile Asp Leu Asn Lys Lys Lys Val Glu Ser Ser Glu Ala Glu Tyr
   450 455 460
   Arg Thr Leu Ser Ala Asn Asp Asp Gly Val Tyr Met Pro Leu Gly Val
   465                 470                 475                 480
   Ile Ser Glu Thr Phe Leu Thr Pro Ile Asn Gly Phe Gly Leu Gln Ala
                   485                 490                 495
   Asp Glu Asn Ser Arg Leu Ile Thr Leu Thr Cys Lys Ser Tyr Leu Arg
               500                 505                 510
   Glu Leu Leu Leu Ala Thr Asp Leu Ser Asn Lys Glu Thr Lys Leu Ile
           515                 520                 525
   Val Pro Pro Ser Gly Phe Ile Ser Asn Ile Val Glu Asn Gly Ser Ile
       530                 535                 540
   Glu Glu Asp Asn Leu Glu Pro Trp Lys Ala Asn Asn Lys Asn Ala Tyr
   545                 550                 555                 560
   Val Asp His Thr Gly Gly Val Asn Gly Thr Lys Ala Leu Tyr Val His
                   565                 570                 575
   Lys Asp Gly Gly Ile Ser Gln Phe Ile Gly Asp Lys Leu Lys Pro Lys
               580                 585                 590
   Thr Glu Tyr Val Ile Gln Tyr Thr Val Lys Gly Lys Pro Ser Ile His
           595                 600                 605
   Leu Lys Asp Glu Asn Thr Gly Tyr Ile His Tyr Glu Asp Thr Asn Asn
       610                 615                 620
   Asn Leu Glu Asp Tyr Gln Thr Ile Asn Lys Arg Phe Thr Thr Gly Thr
   625                 630                 635                 640
   Asp Leu Lys Gly Val Tyr Leu Ile Leu Lys Ser Gln Asn Gly Asp Glu
                   645                 650                 655
   Ala Trp Gly Asp Asn Phe Ile Ile Leu Glu Ile Ser Pro Ser Glu Lys
               660                 665                 670
   Leu Leu Ser Pro Glu Leu Ile Asn Thr Asn Asn Trp Thr Ser Thr Gly
           675                 680                 685
   Ser Thr Asn Ile Ser Gly Asn Thr Leu Thr Leu Tyr Gln Gly Gly Arg
       690                 695                 700
   Gly Ile Leu Lys Gln Asn Leu Gln Leu Asp Ser Phe Ser Thr Tyr Arg
   705                 710                 715                 720
   Val Tyr Phe Ser Val Ser Gly Asp Ala Asn Val Arg Ile Arg Asn Ser
                   725                 730                 735
   Arg Glu Val Leu Phe Glu Lys Arg Tyr Met Ser Gly Ala Lys Asp Val
               740                 745                 750
   Ser Glu Met Phe Thr Thr Lys Phe Glu Lys Asp Asn Phe Tyr Ile Glu
   755 760 765
   Leu Ser Gln Gly Asn Asn Leu Tyr Gly Gly Pro Ile Val His Phe Tyr
   770 775 780
   Asp Val Ser Ile Lys
   785
(2) INFORMATION FOR SEQ ID NO:3:
   (i) SEQUENCE CHARACTERISTICS:
      (A) LENGTH: 2403 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "Synthetic DNA"
   (iii) HYPOTHETICAL: NO
   (ix) FEATURE:
      (A) NAME/KEY: misc_feature
      (B) LOCATION: 11..2389
      (D) OTHER INFORMATION: /note= "maize optimized DNA sequence encoding VIP3A(a)"
   (xi) SEQUENCE DESCRIPTION: SEQ ID NO:3:
   GGATCCACCA ATGAACATGA ACAAGAACAA CACCAAGCTG AGCACCCGCG CCCTGCCGAG 60
   CTTCATCGAC TACTTCAACG GCATCTACGG CTTCGCCACC GGCATCAAGG ACATCATGAA 120
   CATGATCTTC AAGACCGACA CCGGCGGCGA CCTGACCCTG GACGAGATCC TGAAGAACCA 180
   GCAGCTGCTG AACGACATCA GCGGCAAGCT GGACGGCGTG AACGGCAGCC TGAACGACCT 240
   GATCGCCCAG GGCAACCTGA ACACCGAGCT GAGCAAGGAG ATCCTTAAGA TCGCCAACGA 300
   GCAGAACCAG GTGCTGAACG ACGTGAACAA CAAGCTGGAC GCCATCAACA CCATGCTGCG 360
   CGTGTACCTG CCGAAGATCA CCAGCATGCT GAGCGACGTG ATGAAGCAGA ACTACGCCCT 420
   GAGCCTGCAG ATCGAGTACC TGAGCAAGCA GCTGCAGGAG ATCAGCGACA AGCTGGACAT 480
   CATCAACGTG AACGTCCTGA TCAACAGCAC CCTGACCGAG ATCACCCCGG CCTACCAGCG 540
   CATCAAGTAC GTGAACGAGA AGTTCGAAGA GCTGACCTTC GCCACCGAGA CCAGCAGCAA 600
   GGTGAAGAAG GACGGCAGCC CGGCCGACAT CCTGGACGAG CTGACCGAGC TGACCGAGCT 660
   GGCCAAGAGC GTGACCAAGA ACGACGTGGA CGGCTTCGAG TTCTACCTGA ACACCTTCCA 720
   CGACGTGATG GTGGGCAACA ACCTGTTCGG CCGCAGCGCC CTGAAGACCG CCAGCGAGCT 780
   GATCACCAAG GAGAACGTGA AGACCAGCGG CAGCGAGGTG GGCAACGTGT ACAACTTCCT 840
   GATCGTGCTG ACCGCCCTGC AGGCCCAGGC CTTCCTGACC CTGACCACCT GTCGCAAGCT 900
   GCTGGGCCTG GCCGACATCG ACTACACCAG CATCATGAAC GAGCACTTGA ACAAGGAGAA 960
   GGAGGAGTTC CGCGTGAACA TCCTGCCGAC CCTGAGCAAC ACCTTCAGCA ACCCGAACTA 1020
   CGCCAAGGTG AAGGGCAGCG ACGAGGACGC CAAGATGATC GTGGAGGCTA AGCCGGGCCA 1080
   CGCGTTGATC GGCTTCGAGA TCAGCAACGA CAGCATCACC GTGCTGAAGG TGTACGAGGC 1140
   CAAGCTGAAG CAGAACTACC AGGTGGACAA GGACAGCTTG AGCGAGGTGA TCTACGGCGA 1200
   CATGGACAAG CTGCTGTGTC CGGACCAGAG CGAGCAAATC TACTACACCA ACAACATCGT 1260
   GTTCCCGAAC GAGTACGTGA TCACCAAGAT CGACTTCACC AAGAAGATGA AGACCCTGCG 1320
   CTACGAGGTG ACCGCCAACT TCTACGACAG CAGCACCGGC GAGATCGACC TGAACAAGAA 1380
   GAAGGTGGAG AGCAGCGAGG CCGAGTACCG CACCCTGAGC GCGAACGACG ACGGCGTCTA 1440
   CATGCCACTG GGCGTGATCA GCGAGACCTT CCTGACCCCG ATCAACGGCT TTGGCCTGCA 1500
   GGCCGACGAG AACAGCCGCC TGATCACCCT GACCTGTAAG AGCTACCTGC GCGAGCTGCT 1560
   GCTAGCCACC GACCTGAGCA ACAAGGAGAC CAAGCTGATC GTGCCACCGA GCGGCTTCAT 1620
   CAGCAACATC GTGGAGAACG GCAGCATCGA GGAGGACAAC CTGGAGCCGT GGAAGGCCAA 1680
   CAACAAGAAC GCCTACGTGG ACCACACCGG CGGCGTGAAC GGCACCAAGG CCCTGTACGT 1740
   GCACAAGGAC GGCGGCATCA GCCAGTTCAT CGGCGACAAG CTGAAGCCGA AGACCGAGTA 1800
   CGTGATCCAG TACACCGTGA AGGGCAAGCC ATCGATTCAC CTGAAGGACG AGAACACCGG 1860
   CTACATCCAC TACGAGGACA CCAACAACAA CCTGGAGGAC TACCAGACCA TCAACAAGCG 1920
   CTTCACCACC GGCACCGACC TGAAGGGCGT GTACCTGATC CTGAAGAGCC AGAACGGCGA 1980
   CGAGGCCTGG GGCGACAACT TCATCATCCT GGAGATCAGC CCGAGCGAGA AGCTGCTGAG 2040
   CCCGGAGCTG ATCAACACCA ACAACTGGAC CAGCACCGGC AGCACCAACA TCAGCGGCAA 2100
   CACCCTGACC CTGTACCAGG GCGGCCGCGG CATCCTGAAG CAGAACCTGC AGCTGGACAG 2160
   CTTCAGCACC TACCGCGTGT ACTTCAGCGT GAGCGGCGAC GCCAACGTGC GCATCCGCAA 2220
   CAGCCGCGAG GTGCTGTTCG AGAAGAGGTA CATGAGCGGC GCCAAGGACG TGAGCGAGAT 2280
   GTTCACCACC AAGTTCGAGA AGGACAACTT CTACATCGAG CTGAGCCAGG GCAACAACCT 2340
   GTACGGCGGC CCGATCGTGC ACTTCTACGA CGTGAGCATC AAGTTAACGT AGAGCTCAGA 2400
   TCT 2403
(2) INFORMATION FOR SEQ ID NO:4:
   (i) SEQUENCE CHARACTERISTICS:
      (A) LENGTH: 2612 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (ix) FEATURE:
      (A) NAME/KEY: CDS
      (B) LOCATION: 118..2484
      (D) OTHER INFORMATION: /note= "Native DNA sequence encoding VIP3A(b) from AB424"
   (xi) SEQUENCE DESCRIPTION: SEQ ID NO:4:
   ATTGAAATTG ATAAAAAGTT ATGAGTGTTT AATAATCAGT AATTACCAAT AAAGAATTAA          60
   GAATACAAGT TTACAAGAAA TAAGTGTTAC AAAAAATAGC TGAAAAGGAA GATGAAC            117
   ATG AAC AAG AAT AAT ACT AAA TTA AGC ACA AGA GCC TTA CCA AGT TTT           165
   Met Asn Lys Asn Asn Thr Lys Leu Ser Thr Arg Ala Leu Pro Ser Phe
   790                 795                 800                 805
   ATT GAT TAT TTC AAT GGC ATT TAT GGA TTT GCC ACT GGT ATC AAA GAC           213
   Ile Asp Tyr Phe Asn Gly Ile Tyr Gly Phe Ala Thr Gly Ile Lys Asp
                   810                 815                 820
   ATT ATG AAC ATG ATT TTT AAA ACG GAT ACA GGT GGT GAT CTA ACC CTA           261
   Ile Met Asn Met Ile Phe Lys Thr Asp Thr Gly Gly Asp Leu Thr Leu
               825                 830                 835
   GAC GAA ATT TTA AAG AAT CAG CAG CTA CTA AAT GAT ATT TCT GGT AAA           309
   Asp Glu Ile Leu Lys Asn Gln Gln Leu Leu Asn Asp Ile Ser Gly Lys
           840                 845                 850
   TTG GAT GGG GTG AAT GGA AGC TTA AAT GAT CTT ATC GCA CAG GGA AAC           357
   Leu Asp Gly Val Asn Gly Ser Leu Asn Asp Leu Ile Ala Gln Gly Asn
       855                 860                 865
   TTA AAT ACA GAA TTA TCT AAG GAA ATA TTA AAA ATT GCA AAT GAA CAA           405
   Leu Asn Thr Glu Leu Ser Lys Glu Ile Leu Lys Ile Ala Asn Glu Gln
   870                 875                 880                 885
   AAT CAA GTT TTA AAT GAT GTT AAT AAC AAA CTC GAT GCG ATA AAT ACG           453
   Asn Gln Val Leu Asn Asp Val Asn Asn Lys Leu Asp Ala Ile Asn Thr
                   890                 895                 900
   ATG CTT CGG GTA TAT CTA CCT AAA ATT ACC TCT ATG TTG AGT GAT GTA      501
   Met Leu Arg Val Tyr Leu Pro Lys Ile Thr Ser Met Leu Ser Asp Val
               905                 910                 915
   ATG AAA CAA AAT TAT GCG CTA AGT CTG CAA ATA GAA TAC TTA AGT AAA      549
   Met Lys Gln Asn Tyr Ala Leu Ser Leu Gln Ile Glu Tyr Leu Ser Lys
           920                 925                 930
   CAA TTG CAA GAG ATT TCT GAT AAG TTG GAT ATT ATT AAT GTA AAT GTA      597
   Gln Leu Gln Glu Ile Ser Asp Lys Leu Asp Ile Ile Asn Val Asn Val
       935                 940                 945
   CTT ATT AAC TCT ACA CTT ACT GAA ATT ACA CCT GCG TAT CAA AGG ATT      645
   Leu Ile Asn Ser Thr Leu Thr Glu Ile Thr Pro Ala Tyr Gln Arg Ile
   950                 955                 960                 965
   AAA TAT GTG AAC GAA AAA TTT GAG GAA TTA ACT TTT GCT ACA GAA ACT      693
   Lys Tyr Val Asn Glu Lys Phe Glu Glu Leu Thr Phe Ala Thr Glu Thr
                   970                 975                 980
   AGT TCA AAA GTA AAA AAG GAT GGC TCT CCT GCA GAT ATT CGT GAT GAG      741
   Ser Ser Lys Val Lys Lys Asp Gly Ser Pro Ala Asp Ile Arg Asp Glu
               985                 990                 995
   TTA ACT GAG TTA ACT GAA CTA GCG AAA AGT GTA ACA AAA AAT GAT GTG      789
   Leu Thr Glu Leu Thr Glu Leu Ala Lys Ser Val Thr Lys Asn Asp Val
           1000                1005                1010
   GAT GGT TTT GAA TTT TAC CTT AAT ACA TTC CAC GAT GTA ATG GTA GGA      837
   Asp Gly Phe Glu Phe Tyr Leu Asn Thr Phe His Asp Val Met Val Gly
       1015                1020                1025
   AAT AAT TTA TTC GGG CGT TCA GCT TTA AAA ACT GCA TCG GAA TTA ATT      885
   Asn Asn Leu Phe Gly Arg Ser Ala Leu Lys Thr Ala Ser Glu Leu Ile
   1030                1035                1040                1045
   ACT AAA GAA AAT GTG AAA ACA AGT GGC AGT GAG GTC GGA AAT GTT TAT      933
   Thr Lys Glu Asn Val Lys Thr Ser Gly Ser Glu Val Gly Asn Val Tyr
                   1050                1055                1060
   AAC TTC CTA ATT GTA TTA ACA GCT CTG CAA GCA AAA GCT TTT CTT ACT      981
   Asn Phe Leu Ile Val Leu Thr Ala Leu Gln Ala Lys Ala Phe Leu Thr
               1065                1070                1075
   TTA ACA CCA TGC CGA AAA TTA TTA GGC TTA GCA GAT ATT GAT TAT ACT     1029
   Leu Thr Pro Cys Arg Lys Leu Leu Gly Leu Ala Asp Ile Asp Tyr Thr
           1080                1085                1090
   TCT ATT ATG AAT GAA CAT TTA AAT AAG GAA AAA GAG GAA TTT AGA GTA     1077
   Ser Ile Met Asn Glu His Leu Asn Lys Glu Lys Glu Glu Phe Arg Val
       1095                1100                1105
   AAC ATC CTC CCT ACA CTT TCT AAT ACT TTT TCT AAT CCT AAT TAT GCA      1125
   Asn Ile Leu Pro Thr Leu Ser Asn Thr Phe Ser Asn Pro Asn Tyr Ala
   1110                1115                1120                1125
   AAA GTT AAA GGA AGT GAT GAA GAT GCA AAG ATG ATT GTG GAA GCT AAA      1173
   Lys Val Lys Gly Ser Asp Glu Asp Ala Lys Met Ile Val Glu Ala Lys
               1130                1135                1140
   CCA GGA CAT GCA TTG ATT GGG TTT GAA ATT AGT AAT GAT TCA ATT ACA      1221
   Pro Gly His Ala Leu Ile Gly Phe Glu Ile Ser Asn Asp Ser Ile Thr
           1145                1150                1155
   GTA TTA AAA GTA TAT GAG GCT AAG CTA AAA CAA AAT TAT CAA GTC GAT      1269
   Val Leu Lys Val Tyr Glu Ala Lys Leu Lys Gln Asn Tyr Gln Val Asp
       1160                1165                1170
   AAG GAT TCC TTA TCG GAA GTT ATT TAT GGC GAT ATG GAT AAA TTA TTG      1317
   Lys Asp Ser Leu Ser Glu Val Ile Tyr Gly Asp Met Asp Lys Leu Leu
   1175                1180                1185
   TGC CCA GAT CAA TCT GGA CAA ATC TAT TAT ACA AAT AAC ATA GTA TTT      1365
   Cys Pro Asp Gln Ser Gly Gln Ile Tyr Tyr Thr Asn Asn Ile Val Phe
   1190                1195                1200                1205
   CCA AAT GAA TAT GTA ATT ACT AAA ATT GAT TTC ACT AAA AAA ATG AAA      1413
   Pro Asn Glu Tyr Val Ile Thr Lys Ile Asp Phe Thr Lys Lys Met Lys
               1210                1215                1220
   ACT TTA AGA TAT GAG GTA ACA GCG AAT TTT TAT GAT TCT TCT ACA GGA      1461
   Thr Leu Arg Tyr Glu Val Thr Ala Asn Phe Tyr Asp Ser Ser Thr Gly
           1225                1230                1235
   GAA ATT GAC TTA AAT AAG AAA AAA GTA GAA TCA AGT GAA GCG GAG TAT      1509
   Glu Ile Asp Leu Asn Lys Lys Lys Val Glu Ser Ser Glu Ala Glu Tyr
       1240                1245                1250
   AGA ACG TTA AGT GCT AAT GAT GAT GGG GTG TAT ATG CCG TTA GGT GTC      1557
   Arg Thr Leu Ser Ala Asn Asp Asp Gly Val Tyr Met Pro Leu Gly Val
   1255                1260                1265
   ATC AGT GAA ACA TTT TTG ACT CCG ATT AAT GGG TTT GGC CTC CAA GCT      1605
   Ile Ser Glu Thr Phe Leu Thr Pro Ile Asn Gly Phe Gly Leu Gln Ala
   1270                1275                1280                1285
   GAT GAA AAT TCA AGA TTA ATT ACT TTA ACA TGT AAA TCA TAT TTA AGA      1653
   Asp Glu Asn Ser Arg Leu Ile Thr Leu Thr Cys Lys Ser Tyr Leu Arg
               1290                1295                1300
   GAA CTA CTG CTA GCA ACA GAC TTA AGC AAT AAA GAA ACT AAA TTG ATC      1701
   Glu Leu Leu Leu Ala Thr Asp Leu Ser Asn Lys Glu Thr Lys Leu Ile
           1305                1310                1315
   GTC CCG CCA AGT GGT TTT ATT AGC AAT ATT GTA GAG AAC GGG TCC ATA      1749
   Val Pro Pro Ser Gly Phe Ile Ser Asn Ile Val Glu Asn Gly Ser Ile
       1320                1325                1330
   GAA GAG GAC AAT TTA GAG CCG TGG AAA GCA AAT AAT AAG AAT GCG TAT      1797
   Glu Glu Asp Asn Leu Glu Pro Trp Lys Ala Asn Asn Lys Asn Ala Tyr
       1335                1340                1345
   GTA GAT CAT ACA GGC GGA GTG AAT GGA ACT AAA GCT TTA TAT GTT CAT      1845
   Val Asp His Thr Gly Gly Val Asn Gly Thr Lys Ala Leu Tyr Val His
   1350                1355                1360                1365
   AAG GAC GGA GGA ATT TCA CAA TTT ATT GGA GAT AAG TTA AAA CCG AAA      1893
   Lys Asp Gly Gly Ile Ser Gln Phe Ile Gly Asp Lys Leu Lys Pro Lys
                   1370                1375                1380
   ACT GAG TAT GTA ATC CAA TAT ACT GTT AAA GGA AAA CCT TCT ATT CAT      1941
   Thr Glu Tyr Val Ile Gln Tyr Thr Val Lys Gly Lys Pro Ser Ile His
               1385                1390                1395
   TTA AAA GAT GAA AAT ACT GGA TAT ATT CAT TAT GAA GAT ACA AAT AAT      1989
   Leu Lys Asp Glu Asn Thr Gly Tyr Ile His Tyr Glu Asp Thr Asn Asn
           1400                1405                1410
   AAT TTA GAA GAT TAT CAA ACT ATT AAT AAA CGT TTT ACT ACA GGA ACT      2037
   Asn Leu Glu Asp Tyr Gln Thr Ile Asn Lys Arg Phe Thr Thr Gly Thr
       1415                1420                1425
   GAT TTA AAG GGA GTG TAT TTA ATT TTA AAA AGT CAA AAT GGA GAT GAA      2085
   Asp Leu Lys Gly Val Tyr Leu Ile Leu Lys Ser Gln Asn Gly Asp Glu
   1430                1435                1440                1445
   GCT TGG GGA GAT AAC TTT ATT ATT TTG GAA ATT AGT CCT TCT GAA AAG      2133
   Ala Trp Gly Asp Asn Phe Ile Ile Leu Glu Ile Ser Pro Ser Glu Lys
                   1450                1455                1460
   TTA TTA AGT CCA GAA TTA ATT AAT ACA AAT AAT TGG ACG AGT ACG GGA      2181
   Leu Leu Ser Pro Glu Leu Ile Asn Thr Asn Asn Trp Thr Ser Thr Gly
               1465                1470                1475
   TCA ACT AAT ATT AGC GGT AAT ACA CTC ACT CTT TAT CAG GGA GGA CGA      2229
   Ser Thr Asn Ile Ser Gly Asn Thr Leu Thr Leu Tyr Gln Gly Gly Arg
           1480                1485                1490
   GGG ATT CTA AAA CAA AAC CTT CAA TTA GAT AGT TTT TCA ACT TAT AGA      2277
   Gly Ile Leu Lys Gln Asn Leu Gln Leu Asp Ser Phe Ser Thr Tyr Arg
       1495                1500                1505
   GTG TAT TTC TCT GTG TCC GGA GAT GCT AAT GTA AGG ATT AGA AAT TCT      2325
   Val Tyr Phe Ser Val Ser Gly Asp Ala Asn Val Arg Ile Arg Asn Ser
   1510                1515                1520                1525
   AGG GAA GTG TTA TTT GAA AAA AGA TAT ATG AGC GGT GCT AAA GAT GTT      2373
   Arg Glu Val Leu Phe Glu Lys Arg Tyr Met Ser Gly Ala Lys Asp Val
                   1530                1535                1540
   TCT GAA ATG TTC ACT ACA AAA TTT GAG AAA GAT AAC TTC TAT ATA GAG      2421
   Ser Glu Met Phe Thr Thr Lys Phe Glu Lys Asp Asn Phe Tyr Ile Glu
               1545                1550                1555
   CTT TCT CAA GGG AAT AAT TTA TAT GGT GGT CCT ATT GTA CAT TTT TAC      2469
   Leu Ser Gln Gly Asn Asn Leu Tyr Gly Gly Pro Ile Val His Phe Tyr
           1560                1565                1570
   GAT GTC TCT ATT AAG TAAGATCGGG ATCTAATATT AACAGTTTTT AGAAGCTAAT      2524
   Asp Val Ser Ile Lys
       1575
   TCTTGTATAA TGTCCTTGAT TATGGAAAAA CACAATTTTG TTTGCTAAGA TGTATATATA    2584
   GCTCACTCAT TAAAAGGCAA TCAAGCTT                                       2612
(2) INFORMATION FOR SEQ ID NO:5:
   (i) SEQUENCE CHARACTERISTICS:
      (A) LENGTH: 789 amino acids
      (B) TYPE: amino acid
      (D) TOPOLOGY: linear
   (ii) MOLECULE TYPE: protein
   (xi) SEQUENCE DESCRIPTION: SEQ ID NO:5:
   Met Asn Lys Asn Asn Thr Lys Leu Ser Thr Arg Ala Leu Pro Ser Phe
   1               5                   10                  15
   Ile Asp Tyr Phe Asn Gly Ile Tyr Gly Phe Ala Thr Gly Ile Lys Asp
               20                  25                  30
   Ile Met Asn Met Ile Phe Lys Thr Asp Thr Gly Gly Asp Leu Thr Leu
           35                  40                  45
   Asp Glu Ile Leu Lys Asn Gln Gln Leu Leu Asn Asp Ile Ser Gly Lys
       50                  55                  60
   Leu Asp Gly Val Asn Gly Ser Leu Asn Asp Leu Ile Ala Gln Gly Asn
   65                  70                  75                  80
   Leu Asn Thr Glu Leu Ser Lys Glu Ile Leu Lys Ile Ala Asn Glu Gln
                   85                  90                  95
   Asn Gln Val Leu Asn Asp Val Asn Asn Lys Leu Asp Ala Ile Asn Thr
               100                 105                 110
   Met Leu Arg Val Tyr Leu Pro Lys Ile Thr Ser Met Leu Ser Asp Val
           115                 120                 125
   Met Lys Gln Asn Tyr Ala Leu Ser Leu Gln Ile Glu Tyr Leu Ser Lys
       130                 135                 140
   Gln Leu Gln Glu Ile Ser Asp Lys Leu Asp Ile Ile Asn Val Asn Val
   145                 150                 155                 160
   Leu Ile Asn Ser Thr Leu Thr Glu Ile Thr Pro Ala Tyr Gln Arg Ile
   165 170 175
   Lys Tyr Val Asn Glu Lys Phe Glu Glu Leu Thr Phe Ala Thr Glu Thr
   180 185 190
   Ser Ser Lys Val Lys Lys Asp Gly Ser Pro Ala Asp Ile Arg Asp Glu
   195 200 205
   Leu Thr Glu Leu Thr Glu Leu Ala Lys Ser Val Thr Lys Asn Asp Val
   210 215 220
   Asp Gly Phe Glu Phe Tyr Leu Asn Thr Phe His Asp Val Met Val Gly
   225 230 235 240
   Asn Asn Leu Phe Gly Arg Ser Ala Leu Lys Thr Ala Ser Glu Leu Ile
   245 250 255
   Thr Lys Glu Asn Val Lys Thr Ser Gly Ser Glu Val Gly Asn Val Tyr
   260 265 270
   Asn Phe Leu Ile Val Leu Thr Ala Leu Gln Ala Lys Ala Phe Leu Thr
   275 280 285
   Leu Thr Pro Cys Arg Lys Leu Leu Gly Leu Ala Asp Ile Asp Tyr Thr
   290 295 300
   Ser Ile Met Asn Glu His Leu Asn Lys Glu Lys Glu Glu Phe Arg Val
   305 310 315 320
   Asn Ile Leu Pro Thr Leu Ser Asn Thr Phe Ser Asn Pro Asn Tyr Ala
   325 330 335
   Lys Val Lys Gly Ser Asp Glu Asp Ala Lys Met Ile Val Glu Ala Lys
   340 345 350
   Pro Gly His Ala Leu Ile Gly Phe Glu Ile Ser Asn Asp Ser Ile Thr
   355 360 365
   Val Leu Lys Val Tyr Glu Ala Lys Leu Lys Gln Asn Tyr Gln Val Asp
   370 375 380
   Lys Asp Ser Leu Ser Glu Val Ile Tyr Gly Asp Met Asp Lys Leu Leu
   385 390 395 400
   Cys Pro Asp Gln Ser Gly Gln Ile Tyr Tyr Thr Asn Asn Ile Val Phe
   405 410 415
   Pro Asn Glu Tyr Val Ile Thr Lys Ile Asp Phe Thr Lys Lys Met Lys
   420 425 430
   Thr Leu Arg Tyr Glu Val Thr Ala Asn Phe Tyr Asp Ser Ser Thr Gly
   435 440 445
   Glu Ile Asp Leu Asn Lys Lys Lys Val Glu Ser Ser Glu Ala Glu Tyr
       450                 455                 460
   Arg Thr Leu Ser Ala Asn Asp Asp Gly Val Tyr Met Pro Leu Gly Val
   465                 470                 475                 480
   Ile Ser Glu Thr Phe Leu Thr Pro Ile Asn Gly Phe Gly Leu Gln Ala
                   485                 490                 495
   Asp Glu Asn Ser Arg Leu Ile Thr Leu Thr Cys Lys Ser Tyr Leu Arg
               500                 505                 510
   Glu Leu Leu Leu Ala Thr Asp Leu Ser Asn Lys Glu Thr Lys Leu Ile
           515                 520                 525
   Val Pro Pro Ser Gly Phe Ile Ser Asn Ile Val Glu Asn Gly Ser Ile
       530                 535                 540
   Glu Glu Asp Asn Leu Glu Pro Trp Lys Ala Asn Asn Lys Asn Ala Tyr
   545                 550                 555                 560
   Val Asp His Thr Gly Gly Val Asn Gly Thr Lys Ala Leu Tyr Val His
                   565                 570                 575
   Lys Asp Gly Gly Ile Ser Gln Phe Ile Gly Asp Lys Leu Lys Pro Lys
               580                 585                 590
   Thr Glu Tyr Val Ile Gln Tyr Thr Val Lys Gly Lys Pro Ser Ile His
           595                 600                 605
   Leu Lys Asp Glu Asn Thr Gly Tyr Ile His Tyr Glu Asp Thr Asn Asn
       610                 615                 620
   Asn Leu Glu Asp Tyr Gln Thr Ile Asn Lys Arg Phe Thr Thr Gly Thr
   625                 630                 635                 640
   Asp Leu Lys Gly Val Tyr Leu Ile Leu Lys Ser Gln Asn Gly Asp Glu
                   645                 650                 655
   Ala Trp Gly Asp Asn Phe Ile Ile Leu Glu Ile Ser Pro Ser Glu Lys
               660                 665                 670
   Leu Leu Ser Pro Glu Leu Ile Asn Thr Asn Asn Trp Thr Ser Thr Gly
           675                 680                 685
   Ser Thr Asn Ile Ser Gly Asn Thr Leu Thr Leu Tyr Gln Gly Gly Arg
       690                 695                 700
   Gly Ile Leu Lys Gln Asn Leu Gln Leu Asp Ser Phe Ser Thr Tyr Arg
   705                 710                 715                 720
   Val Tyr Phe Ser Val Ser Gly Asp Ala Asn Val Arg Ile Arg Asn Ser
                   725                 730                 735
   Arg Glu Val Leu Phe Glu Lys Arg Tyr Met Ser Gly Ala Lys Asp Val
               740                 745                 750
   Ser Glu Met Phe Thr Thr Lys Phe Glu Lys Asp Asn Phe Tyr Ile Glu
   755 760 765
   Leu Ser Gln Gly Asn Asn Leu Tyr Gly Gly Pro Ile Val His Phe Tyr
   770 775 780
   Asp Val Ser Ile Lys
   785
(2) INFORMATION FOR SEQ ID NO:6:
   (i) SEQUENCE CHARACTERISTICS:
      (A) LENGTH: 2444 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (ix) FEATURE:
      (A) NAME/KEY: CDS
      (B) LOCATION: 17..2444
      (D) OTHER INFORMATION: /product= "3A(a) synthetic:native fusion"
   (xi) SEQUENCE DESCRIPTION: SEQ ID NO:6:
   GGATCCACCA ATGAAC ATG AAC AAG AAC AAC ACC AAG CTG AGC ACC CGC        49
                     Met Asn Lys Asn Asn Thr Lys Leu Ser Thr Arg
                      1               5                   10
   GCC CTG CCG AGC TTC ATC GAC TAC TTC AAC GGC ATC TAC GGC TTC GCC      97
   Ala Leu Pro Ser Phe Ile Asp Tyr Phe Asn Gly Ile Tyr Gly Phe Ala
               15                  20                  25
   ACC GGC ATC AAG GAC ATC ATG AAC ATG ATC TTC AAG ACC GAC ACC GGC     145
   Thr Gly Ile Lys Asp Ile Met Asn Met Ile Phe Lys Thr Asp Thr Gly
           30                  35                  40
   GGC GAC CTG ACC CTG GAC GAG ATC CTG AAG AAC CAG CAG CTG CTG AAC     193
   Gly Asp Leu Thr Leu Asp Glu Ile Leu Lys Asn Gln Gln Leu Leu Asn
       45                  50                  55
   GAC ATC AGC GGC AAG CTG GAC GGC GTG AAC GGC AGC CTG AAC GAC CTG     241
   Asp Ile Ser Gly Lys Leu Asp Gly Val Asn Gly Ser Leu Asn Asp Leu
    60                 65                  70                  75
   ATC GCC CAG GGC AAC CTG AAC ACC GAG CTG AGC AAG GAG ATC CTT AAG     289
   Ile Ala Gln Gly Asn Leu Asn Thr Glu Leu Ser Lys Glu Ile Leu Lys
                   80                  85                  90
   ATC GCC AAC GAG CAG AAC CAG GTG CTG AAC GAC GTG AAC AAC AAG CTG     337
   Ile Ala Asn Glu Gln Asn Gln Val Leu Asn Asp Val Asn Asn Lys Leu
               95                  100                 105
   GAC GCC ATC AAC ACC ATG CTG CGC GTG TAC CTG CCG AAG ATC ACC AGC     385
   Asp Ala Ile Asn Thr Met Leu Arg Val Tyr Leu Pro Lys Ile Thr Ser
           110                 115                 120
   ATG CTG AGC GAC GTG ATG AAG CAG AAC TAC GCC CTG AGC CTG CAG ATC     433
   Met Leu Ser Asp Val Met Lys Gln Asn Tyr Ala Leu Ser Leu Gln Ile
       125                 130                 135
   GAG TAC CTG AGC AAG CAG CTG CAG GAG ATC AGC GAC AAG CTG GAC ATC     481
   Glu Tyr Leu Ser Lys Gln Leu Gln Glu Ile Ser Asp Lys Leu Asp Ile
   140                 145                 150                 155
   ATC AAC GTG AAC GTC CTG ATC AAC AGC ACC CTG ACC GAG ATC ACC CCG     529
   Ile Asn Val Asn Val Leu Ile Asn Ser Thr Leu Thr Glu Ile Thr Pro
                   160                 165                 170
   GCC TAC CAG CGC ATC AAG TAC GTG AAC GAG AAG TTC GAA GAG CTG ACC     577
   Ala Tyr Gln Arg Ile Lys Tyr Val Asn Glu Lys Phe Glu Glu Leu Thr
               175                 180                 185
   TTC GCC ACC GAG ACC AGC AGC AAG GTG AAG AAG GAC GGC AGC CCG GCC     625
   Phe Ala Thr Glu Thr Ser Ser Lys Val Lys Lys Asp Gly Ser Pro Ala
           190                 195                 200
   GAC ATC CTG GAC GAG CTG ACC GAG CTG ACC GAG CTG GCC AAG AGC GTG     673
   Asp Ile Leu Asp Glu Leu Thr Glu Leu Thr Glu Leu Ala Lys Ser Val
       205                 210                 215
   ACC AAG AAC GAC GTG GAC GGC TTC GAG TTC TAC CTG AAC ACC TTC CAC      721
   Thr Lys Asn Asp Val Asp Gly Phe Glu Phe Tyr Leu Asn Thr Phe His
   220                 225                 230                 235
   GAC GTG ATG GTG GGC AAC AAC CTG TTC GGC CGC AGC GCC CTG AAG ACC      769
   Asp Val Met Val Gly Asn Asn Leu Phe Gly Arg Ser Ala Leu Lys Thr
                   240                 245                 250
   GCC AGC GAG CTG ATC ACC AAG GAG AAC GTG AAG ACC AGC GGC AGC GAG      817
   Ala Ser Glu Leu Ile Thr Lys Glu Asn Val Lys Thr Ser Gly Ser Glu
               255                 260                 265
   GTG GGC AAC GTG TAC AAC TTC CTG ATC GTG CTG ACC GCC CTG CAG GCC      865
   Val Gly Asn Val Tyr Asn Phe Leu Ile Val Leu Thr Ala Leu Gln Ala
           270                 275                 280
   CAG GCC TTC CTG ACC CTG ACC ACC TGT CGC AAG CTG CTG GGC CTG GCC      913
   Gln Ala Phe Leu Thr Leu Thr Thr Cys Arg Lys Leu Leu Gly Leu Ala
       285                 290                 295
   GAC ATC GAC TAC ACC AGC ATC ATG AAC GAG CAC TTG AAC AAG GAG AAG      961
   Asp Ile Asp Tyr Thr Ser Ile Met Asn Glu His Leu Asn Lys Glu Lys
   300                 305                 310                 315
   GAG GAG TTC CGC GTG AAC ATC CTG CCG ACC CTG AGC AAC ACC TTC AGC     1009
   Glu Glu Phe Arg Val Asn Ile Leu Pro Thr Leu Ser Asn Thr Phe Ser
                   320                 325                 330
   AAC CCG AAC TAC GCC AAG GTG AAG GGC AGC GAC GAG GAC GCC AAG ATG     1057
   Asn Pro Asn Tyr Ala Lys Val Lys Gly Ser Asp Glu Asp Ala Lys Met
               335                 340                 345
   ATC GTG GAG GCT AAG CCG GGC CAC GCG TTG ATC GGC TTC GAG ATC AGC     1105
   Ile Val Glu Ala Lys Pro Gly His Ala Leu Ile Gly Phe Glu Ile Ser
           350                 355                 360
   AAC GAC AGC ATC ACC GTG CTG AAG GTG TAC GAG GCC AAG CTG AAG CAG     1153
   Asn Asp Ser Ile Thr Val Leu Lys Val Tyr Glu Ala Lys Leu Lys Gln
       365                 370                 375
   AAC TAC CAG GTG GAC AAG GAC AGC TTG AGC GAG GTG ATC TAC GGC GAC     1201
   Asn Tyr Gln Val Asp Lys Asp Ser Leu Ser Glu Val Ile Tyr Gly Asp
   380                 385                 390                 395
   ATG GAC AAG CTG CTG TGT CCG GAC CAG AGC GAG CAA ATC TAC TAC ACC     1249
   Met Asp Lys Leu Leu Cys Pro Asp Gln Ser Glu Gln Ile Tyr Tyr Thr
                   400                 405                 410
   AAC AAC ATC GTG TTC CCG AAC GAG TAC GTG ATC ACC AAG ATC GAC TTC     1297
   Asn Asn Ile Val Phe Pro Asn Glu Tyr Val Ile Thr Lys Ile Asp Phe
               415                 420                 425
   ACC AAG AAG ATG AAG ACC CTG CGC TAC GAG GTG ACC GCC AAC TTC TAC     1345
   Thr Lys Lys Met Lys Thr Leu Arg Tyr Glu Val Thr Ala Asn Phe Tyr
           430                 435                 440
   GAC AGC AGC ACC GGC GAG ATC GAC CTG AAC AAG AAG AAG GTG GAG AGC     1393
   Asp Ser Ser Thr Gly Glu Ile Asp Leu Asn Lys Lys Lys Val Glu Ser
       445                 450                 455
   AGC GAG GCC GAG TAC CGC ACC CTG AGC GCG AAC GAC GAC GGC GTC TAC     1441
   Ser Glu Ala Glu Tyr Arg Thr Leu Ser Ala Asn Asp Asp Gly Val Tyr
   460                 465                 470                 475
   ATG CCA CTG GGC GTG ATC AGC GAG ACC TTC CTG ACC CCG ATC AAC GGC     1489
   Met Pro Leu Gly Val Ile Ser Glu Thr Phe Leu Thr Pro Ile Asn Gly
                   480                 485                 490
   TTT GGC CTG CAG GCC GAC GAG AAC AGC CGC CTG ATC ACC CTG ACC TGT     1537
   Phe Gly Leu Gln Ala Asp Glu Asn Ser Arg Leu Ile Thr Leu Thr Cys
               495                 500                 505
   AAG AGC TAC CTG CGC GAG CTG CTG CTA GCC ACC GAC CTG AGC AAC AAG     1585
   Lys Ser Tyr Leu Arg Glu Leu Leu Leu Ala Thr Asp Leu Ser Asn Lys
           510                 515                 520
   GAG ACC AAG CTG ATC GTG CCA CCG AGC GGC TTC ATC AGC AAC ATC GTG     1633
   Glu Thr Lys Leu Ile Val Pro Pro Ser Gly Phe Ile Ser Asn Ile Val
       525                 530                 535
   GAG AAC GGC AGC ATC GAG GAG GAC AAC CTG GAG CCG TGG AAG GCC AAC     1681
   Glu Asn Gly Ser Ile Glu Glu Asp Asn Leu Glu Pro Trp Lys Ala Asn
   540                 545                 550                 555
   AAC AAG AAC GCC TAC GTG GAC CAC ACC GGC GGC GTG AAC GGC ACC AAG     1729
   Asn Lys Asn Ala Tyr Val Asp His Thr Gly Gly Val Asn Gly Thr Lys
                   560                 565                 570
   GCC CTG TAC GTG CAC AAG GAC GGC GGC ATC AGC CAG TTC ATC GGC GAC     1777
   Ala Leu Tyr Val His Lys Asp Gly Gly Ile Ser Gln Phe Ile Gly Asp
               575                 580                 585
   AAG CTG AAG CCG AAG ACC GAG TAC GTG ATC CAG TAC ACC GTG AAG GGC     1825
   Lys Leu Lys Pro Lys Thr Glu Tyr Val Ile Gln Tyr Thr Val Lys Gly
           590                 595                 600
   AAG CCA TCG ATT CAC CTG AAG GAC GAG AAC ACC GGC TAC ATC CAC TAC     1873
   Lys Pro Ser Ile His Leu Lys Asp Glu Asn Thr Gly Tyr Ile His Tyr
       605                 610                 615
   GAG GAC ACC AAC AAC AAC CTG GAG GAC TAC CAG ACC ATC AAC AAG CGC     1921
   Glu Asp Thr Asn Asn Asn Leu Glu Asp Tyr Gln Thr Ile Asn Lys Arg
   620                 625                 630                 635
   TTC ACC ACC GGC ACC GAC CTG AAG GGC GTG TAC CTG ATC CTG AAG AGC     1969
   Phe Thr Thr Gly Thr Asp Leu Lys Gly Val Tyr Leu Ile Leu Lys Ser
                   640                 645                 650
   CAG AAC GGC GAC GAG GCC TGG GGC GAC AAC TTC ATC ATC CTG GAG ATC      2017
   Gln Asn Gly Asp Glu Ala Trp Gly Asp Asn Phe Ile Ile Leu Glu Ile
               655                 660                 665
   AGC CCG AGC GAG AAG CTG CTG AGC CCG GAG CTG ATC AAC ACC AAC AAC      2065
   Ser Pro Ser Glu Lys Leu Leu Ser Pro Glu Leu Ile Asn Thr Asn Asn
           670                 675                 680
   TGG ACC AGC ACC GGC AGC ACC AAC ATC AGC GGC AAC ACC CTG ACC CTG      2113
   Trp Thr Ser Thr Gly Ser Thr Asn Ile Ser Gly Asn Thr Leu Thr Leu
       685                 690                 695
   TAC CAG GGC GGC CGG GGG ATT CTA AAA CAA AAC CTT CAA TTA GAT AGT      2161
   Tyr Gln Gly Gly Arg Gly Ile Leu Lys Gln Asn Leu Gln Leu Asp Ser
   700                 705                 710                 715
   TTT TCA ACT TAT AGA GTG TAT TTT TCT GTG TCC GGA GAT GCT AAT GTA      2209
   Phe Ser Thr Tyr Arg Val Tyr Phe Ser Val Ser Gly Asp Ala Asn Val
                   720                 725                 730
   AGG ATT AGA AAT TCT AGG GAA GTG TTA TTT GAA AAA AGA TAT ATG AGC      2257
   Arg Ile Arg Asn Ser Arg Glu Val Leu Phe Glu Lys Arg Tyr Met Ser
               735                 740                 745
   GGT GCT AAA GAT GTT TCT GAA ATG TTC ACT ACA AAA TTT GAG AAA GAT      2305
   Gly Ala Lys Asp Val Ser Glu Met Phe Thr Thr Lys Phe Glu Lys Asp
           750                 755                 760
   AAC TTT TAT ATA GAG CTT TCT CAA GGG AAT AAT TTA TAT GGT GGT CCT      2353
   Asn Phe Tyr Ile Glu Leu Ser Gln Gly Asn Asn Leu Tyr Gly Gly Pro
       765                 770                 775
   ATT GTA CAT TTT TAC GAT GTC TCT ATT AAG NAA GAT CGG GAT CTA ATA      2401
   Ile Val His Phe Tyr Asp Val Ser Ile Lys Xaa Asp Arg Asp Leu Ile
   780                 785                 790                 795
   TTA ACA GTT TTT AAA AGC NAA TTC TTG TAT AAT GTC CTT GAT  T           2444
   Leu Thr Val Phe Lys Ser Xaa Phe Leu Tyr Asn Val Leu Asp
                   800                 805
(2) INFORMATION FOR SEQ ID NO:7:
   (i) SEQUENCE CHARACTERISTICS:
      (A) LENGTH: 809 amino acids
      (B) TYPE: amino acid
      (D) TOPOLOGY: linear
   (ii) MOLECULE TYPE: protein
   (xi) SEQUENCE DESCRIPTION: SEQ ID NO:7:
   Met Asn Lys Asn Asn Thr Lys Leu Ser Thr Arg Ala Leu Pro Ser Phe
    1               5                  10                  15
   Ile Asp Tyr Phe Asn Gly Ile Tyr Gly Phe Ala Thr Gly Ile Lys Asp
   20 25 30
   Ile Met Asn Met Ile Phe Lys Thr Asp Thr Gly Gly Asp Leu Thr Leu
   35 40 45
   Asp Glu Ile Leu Lys Asn Gln Gln Leu Leu Asn Asp Ile Ser Gly Lys
   50 55 60
   Leu Asp Gly Val Asn Gly Ser Leu Asn Asp Leu Ile Ala Gln Gly Asn
   65 70 75 80
   Leu Asn Thr Glu Leu Ser Lys Glu Ile Leu Lys Ile Ala Asn Glu Gln
   85 90 95
   Asn Gln Val Leu Asn Asp Val Asn Asn Lys Leu Asp Ala Ile Asn Thr
   100 105 110
   Met Leu Arg Val Tyr Leu Pro Lys Ile Thr Ser Met Leu Ser Asp Val
   115 120 125
   Met Lys Gln Asn Tyr Ala Leu Ser Leu Gln Ile Glu Tyr Leu Ser Lys
   130 135 140
   Gln Leu Gln Glu Ile Ser Asp Lys Leu Asp Ile Ile Asn Val Asn Val
   145 150 155 160
   Leu Ile Asn Ser Thr Leu Thr Glu Ile Thr Pro Ala Tyr Gln Arg Ile
   165 170 175
   Lys Tyr Val Asn Glu Lys Phe Glu Glu Leu Thr Phe Ala Thr Glu Thr
   180 185 190
   Ser Ser Lys Val Lys Lys Asp Gly Ser Pro Ala Asp Ile Leu Asp Glu
   195 200 205
   Leu Thr Glu Leu Thr Glu Leu Ala Lys Ser Val Thr Lys Asn Asp Val
   210 215 220
   Asp Gly Phe Glu Phe Tyr Leu Asn Thr Phe His Asp Val Met Val Gly
   225 230 235 240
   Asn Asn Leu Phe Gly Arg Ser Ala Leu Lys Thr Ala Ser Glu Leu Ile
   245 250 255
   Thr Lys Glu Asn Val Lys Thr Ser Gly Ser Glu Val Gly Asn Val Tyr
   260 265 270
   Asn Phe Leu Ile Val Leu Thr Ala Leu Gln Ala Gln Ala Phe Leu Thr
   275 280 285
   Leu Thr Thr Cys Arg Lys Leu Leu Gly Leu Ala Asp Ile Asp Tyr Thr
   290 295 300
   Ser Ile Met Asn Glu His Leu Asn Lys Glu Lys Glu Glu Phe Arg Val
   305 310 315 320
   Asn Ile Leu Pro Thr Leu Ser Asn Thr Phe Ser Asn Pro Asn Tyr Ala
   325 330 335
   Lys Val Lys Gly Ser Asp Glu Asp Ala Lys Met Ile Val Glu Ala Lys
   340 345 350
   Pro Gly His Ala Leu Ile Gly Phe Glu Ile Ser Asn Asp Ser Ile Thr
   355 360 365
   Val Leu Lys Val Tyr Glu Ala Lys Leu Lys Gln Asn Tyr Gln Val Asp
   370 375 380
   Lys Asp Ser Leu Ser Glu Val Ile Tyr Gly Asp Met Asp Lys Leu Leu
   385 390 395 400
   Cys Pro Asp Gln Ser Glu Gln Ile Tyr Tyr Thr Asn Asn Ile Val Phe
   405 410 415
   Pro Asn Glu Tyr Val Ile Thr Lys Ile Asp Phe Thr Lys Lys Met Lys
   420 425 430
   Thr Leu Arg Tyr Glu Val Thr Ala Asn Phe Tyr Asp Ser Ser Thr Gly
   435 440 445
   Glu Ile Asp Leu Asn Lys Lys Lys Val Glu Ser Ser Glu Ala Glu Tyr
   450 455 460
   Arg Thr Leu Ser Ala Asn Asp Asp Gly Val Tyr Met Pro Leu Gly Val
   465 470 475 480
   Ile Ser Glu Thr Phe Leu Thr Pro Ile Asn Gly Phe Gly Leu Gln Ala
   485 490 495
   Asp Glu Asn Ser Arg Leu Ile Thr Leu Thr Cys Lys Ser Tyr Leu Arg
   500 505 510
   Glu Leu Leu Leu Ala Thr Asp Leu Ser Asn Lys Glu Thr Lys Leu Ile
   515 520 525
   Val Pro Pro Ser Gly Phe Ile Ser Asn Ile Val Glu Asn Gly Ser Ile
   530 535 540
   Glu Glu Asp Asn Leu Glu Pro Trp Lys Ala Asn Asn Lys Asn Ala Tyr
   545 550 555 560
   Val Asp His Thr Gly Gly Val Asn Gly Thr Lys Ala Leu Tyr Val His
   565 570 575
   Lys Asp Gly Gly Ile Ser Gln Phe Ile Gly Asp Lys Leu Lys Pro Lys
   580 585 590
   Thr Glu Tyr Val Ile Gln Tyr Thr Val Lys Gly Lys Pro Ser Ile His
   595 600 605
   Leu Lys Asp Glu Asn Thr Gly Tyr Ile His Tyr Glu Asp Thr Asn Asn
       610                 615                 620
   Asn Leu Glu Asp Tyr Gln Thr Ile Asn Lys Arg Phe Thr Thr Gly Thr
   625                 630                 635                 640
   Asp Leu Lys Gly Val Tyr Leu Ile Leu Lys Ser Gln Asn Gly Asp Glu
                   645                 650                 655
   Ala Trp Gly Asp Asn Phe Ile Ile Leu Glu Ile Ser Pro Ser Glu Lys
               660                 665                 670
   Leu Leu Ser Pro Glu Leu Ile Asn Thr Asn Asn Trp Thr Ser Thr Gly
           675                 680                 685
   Ser Thr Asn Ile Ser Gly Asn Thr Leu Thr Leu Tyr Gln Gly Gly Arg
       690                 695                 700
   Gly Ile Leu Lys Gln Asn Leu Gln Leu Asp Ser Phe Ser Thr Tyr Arg
   705                 710                 715                 720
   Val Tyr Phe Ser Val Ser Gly Asp Ala Asn Val Arg Ile Arg Asn Ser
                   725                 730                 735
   Arg Glu Val Leu Phe Glu Lys Arg Tyr Met Ser Gly Ala Lys Asp Val
               740                 745                 750
   Ser Glu Met Phe Thr Thr Lys Phe Glu Lys Asp Asn Phe Tyr Ile Glu
           755                 760                 765
   Leu Ser Gln Gly Asn Asn Leu Tyr Gly Gly Pro Ile Val His Phe Tyr
       770                 775                 780
   Asp Val Ser Ile Lys Xaa Asp Arg Asp Leu Ile Leu Thr Val Phe Lys
   785                 790                 795                 800
   Ser Xaa Phe Leu Tyr Asn Val Leu Asp
                   805

## Description

The present invention is drawn to methods and compositions for controlling plant and non-plant pests. Particularly, new pesticidal proteins are disclosed which are isolatable from the vegetative growth stage of *Bacillus. Bacillus* strains, proteins, and genes encoding the proteins are provided. The methods and compositions of the invention may be used in a variety of systems for controlling plant and non-plant pests.

Insect pests are a major factor in the loss of the world's commercially important agricultural crops. Broad spectrum chemical pesticides have been used extensively to control or eradicate pests of agricultural importance. There is, however, substantial interest in developing effective alternative pesticides.

Microbial pesticides have played an important role as alternatives to chemical pest control. The most extensively used microbial product is based on the bacterium *Bacillus thuringiensis* (Bt). Bt is a gram-positive spore forming *Bacillus* which produces an insecticidal crystal protein (ICP) during sporulation.

Numerous varieties of Bt are known that produce more than 25 different but related ICP's. The majority of ICP's made by Bt are toxic to larvae of certain insects in the orders *Lepidoptera, Diptera* and *Coleoptera.* In general, when an ICP is ingested by a susceptible insect the crystal is solubilized and transformed into a toxic moiety by the insect gut proteases. None of the ICP's active against coleopteran larvae such as Colorado potato beetle (*Leptinotarsa decemlineata*) or Yellow mealworm (*Tenebrio molitor)* have demonstrated significant effects on members of the genus *Diabrotica* particularly *Diabrotica virgifera virgifera*, the western corn rootworm (WCRW) or *Diabrotica longicornis barberi*, the northern corn rootworm.

*Bacillus cereus* (Bc) is closely related to Bt. A major distinguishing characteristic is the absence of a parasporal crystal in Bc. Bc is a widely distributed bacterium that is commonly found in soil and has been isolated from a variety of foods and drugs. The organism has been implicated in the spoilage of food.

Although Bt has been very useful in controlling insect pests, there is a need to expand the number of potential biological control agents.

Within the present invention compositions and methods for controlling plant pests are provided. In particular, novel pesticidal proteins are provided which are produced during vegetative growth of Bacillus strains. The proteins are useful as pesticidal agents.

More specifically, the present invention relates to a substantially purified *Bacillus* strain which produces a pesticidal protein during vegetative growth wherein said *Bacillus* is not *B. sphaericus* SSII-1. Preferred are a *Bacillus thuringiensis* strains having Accession Numbers, NRRL B-21225, and NRRL B-21439.

The invention further relates to an insect-specific protein isolatable during the vegetative growth phase of *Bacillus* spp, but preferably of a *Bacillus thuringiensis* and *B. cereus* strain, and components thereof, wherein said protein is not the mosquitocidal toxin from *B. sphaericus* SSII-1. The insect-specific protein of the invention is preferably toxic to Coleoptera or Lepidoptera insects and has a molecular weight of about 30 kDa or greater, preferably of about 60 to about 100 kDa, and more preferably of about 80 kDa.

More particularly, the insect-specific protein of the invention has a spectrum of insecticidal activity that includes an activity against *Agrotis* and/or *Spodoptera* species, but preferably a black cutworm [*Agrotis ipsilon* ; BCW] and/or fall armyworm [*Spodoptera frugiperda*] and/or beet armyworm [*Spodoptera exigua* ] and/or tobacco budworm and/or corn earworm [*Helicoverpa zea*] activity.

The insect-specific protein of the invention can preferably be isolated from a *Bacillus spp* strain selected from Accession Numbers NRRL B-21225, and NRRL B-21439.

Preferred is an insect-specific protein, wherein said protein has the sequence selected from the group consisting of SEQ ID NO:2 and SEQ ID NO:5, including any proteins that are structurally and/or functionally homologous thereto.

A further preferred embodiment of the invention comprises an insect-specific protein of the invention, wherein the sequences representing the secretion signal have been removed or inactivated.

The present invention further relates to multimeric pesticidal proteins, which comprise more than one polypeptide chain and wherein at least one of the said polypeptide chains represents an insect-specific protein of the invention and at least one of the said polypeptide chains represents an auxiliary protein of the invention, which activates or enhances the pesticidal activity of the said insect-specific protein.

The multimeric pesticidal proteins according to the invention preferably have a molecular weight of about 50 kDa to about 200 kDa.

The present invention further relates to fusion proteins comprising several protein domains including at least an insect-specific protein of the invention which, when translated by ribosomes, produce a fusion protein with at least the combined attributes of the insect-specific protein of the invention and, optionally, of the other components used in the fusion.

As used in the present application, substantial sequence homology means close structural relationship between sequences of amino acids. For example, substantially homologous proteins may be 40% homologous, preferably 50% and most preferably 60% or 80% homologous, or more. Homology also includes a relationship wherein one or several subsequences of amino acids are missing, or subsequences with additional amino acids are interdispersed.

A further aspect of the invention relates to a DNA molecule comprising a nucleotide sequence which encodes an insect-specific protein isolatable during the vegetative growth phase of *Bacillus* spp. and components thereof, wherein said protein is not the mosquitocidal toxin from *B. sphaericus* SSII-1. In particular, the present invention relates to a DNA molecule comprising a nucleotide sequence which encodes an insect-specific protein wherein the spectrum of insecticidal activity includes an activity against *Agrotis* and/or *Spodoptera* species, but preferably a black cutworm [*Agrotis ipsilon* ; BCW] and/or fall armyworm [*Spodoptera frugiperda*] and/or beet armyworm [*Spodoptera exigua* ] and/or tobacco budworm and/or corn earworm [*Helicoverpa zea*] activity.

Preferred is a DNA molecule, wherein the said molecule comprises a nucleotide sequence as given SEQ ID NO:1 or SEQ ID NO:4, including any DNA molecules that are structurally and/or functionally homologous thereto.

A further embodiment of the invention relates to a DNA molecule comprising a nucleotide sequence which encodes an insect-specific protein isolatable during the vegetative growth phase of *Bacillus* spp. and components thereof, wherein said protein is not the mosquitocidal toxin from *B. sphaericus* SSII-1, which nucleotide sequence has been optimized for expression in a microorganism or a plant.

Preferred is a DNA molecule, wherein the said molecule comprises a nucleotide sequence as given in SEQ ID NO:30, including any DNA molecules that are structurally and/or functionally homologous thereto.

The invention further relates to a DNA molecule which comprises a nucleotide sequence encoding a multimeric pesticidal protein, which comprises more than one polypeptide chains and wherein at least one of the said polypeptide chains represents an insect-specific protein of the invention and at least one of the said polypeptide chains represents an auxiliary protein according to the invention, which activates or enhances the pesticidal activity of the said insect-specific protein.

A further embodiment of the invention relates to a DNA molecule which comprises a nucleotide sequence encoding a fusion protein comprising several protein domains including at least an insect-specific protein of the invention produced by in frame genetic fusions, which, when translated by ribosomes, produce a fusion protein with at least the combined attributes of the insect-specific protein of the invention and, optionally, of the other components used in the fusion.

The invention further relates to a DNA molecule which comprises a nucleotide sequence encoding a fusion protein comprising an insect-specific protein of the invention fused to a signal sequence, preferably a secretion signal sequence or a targeting sequence that directs the transgene product to a specific organelle or cell compartment, which signal sequence is of herterologous origin with respect to the recipient DNA.

The present invention further encompasses a DNA molecule comprising a nucleotide sequence encoding a fusion protein according to the invention that has been optimized for expression in a microorganism or plant.

The invention further relates to an optimized DNA molecule, wherein the sequences encoding the secretion signal have been removed from its 5' end. As used in the present application, substantial sequence homology means close structural relationship between sequences of nucleotides. For example, substantially homologous DNA molecules may be 60% homologous, preferably 80% and most preferably 90% or 95% homologous, or more. Homology also includes a relationship wherein one or several subsequences of nucleotides or amino acids are missing, or subsequences with additional nucleotides or amino acids are interdispersed.

Also comprised by the present invention are DNA molecules which hybridizes to a DNA molecule according to the invention as defined hereinbefore, but preferably to an oligonucleotide probe obtainable from said DNA molecule comprising a contiguous portion of the coding sequence for the said insect-specific protein at least 10 nucleotides in length, under moderately stringent conditions and which molecules have insect-specific activity and also the insect-specific proteins being encoded by the said DNA molecules.

Preferred are DNA molecules, wherein hybridization occurs at 65°C in a buffer comprising 7% SDS and 0.5 M sodium phosphate.

Especially preferred is a DNA molecule comprising a nucleotide sequence which encodes an insect-specific protein according to the invention obtainable by a process comprising
(a) obtaining a DNA molecule comprising a nucleotide sequence encoding an insect-specific protein; and
(b) hybridizing said DNA molecule with an oligonucleotide probe acording to claim 107 obtained from a DNA molecule comprising a nucleotide sequence as given in SEQ ID NO:1, SEQ ID NO:3, or SEQ ID NO:4; and
(c) isolating said hybridized DNA.

The invention further relates to an insect-specific protein, wherein the said protein is encoded by a DNA molecule according to the invention.

Also encompassed by the invention is an expression cassette comprising a DNA molecule according to the invention operably linked to expression sequences including the transcriptional and translational regulatory signals necessary for expression of the associated DNA constructs in a host organism, preferably a microorganism or a plant, and optionally further regulatory sequences.

The invention further relates to a vector molecule comprising an expression cassette according to the invention.

The expression cassette and/or the vector molecule according to the invention are preferably part of the plant genome.

A further embodiment of the invention relates to a host organism, preferably a host organism selected from the group consisting of plant and insect cells, bacteria, yeast, baculoviruses, protozoa, nematodes and algae, comprising a DNA molecule according to the invention, an expression cassette comprising the said DNA molecule or a vector molecule comprising the said expression cassette, preferably stably incorporated into the genome of the host organism.

The invention further relates to a transgenic plant, but preferably a maize plant, including parts as well as progeny and seed thereof comprising a DNA molecule according to the invention, an expression cassette comprising the said DNA molecule or a vector molecule comprising the said expression cassette, preferably stably incorporated into the plant genome.

Preferred is a transgenic plant including parts as well as progeny and seed thereof which has been stably transformed with a DNA molecule according to the invention, an expression cassette comprising the said DNA molecule or a vector molecule comprising the said expression cassette.

Also preferred is a transgenic plant including parts as well as progeny and seed thereof which expresses an insect-specific protein according to the invention.

The invention further relates to a transgenic plant, preferably a maize plant, according to the invention as defined hereinbefore, which further expresses a second distinct insect control principle, but preferably a *Bt* δ-endotoxin. The said plant is preferably a hybrid plant.

Parts of transgenic plants are to be understood within the scope of the invention to comprise, for example, plant cells, protoplasts, tissues, callus, embryos as well as flowers, stems, fruits, leaves, roots originating in transgenic plants or their progeny previously transformed with a DNA molecule according to the invention and therefore consisting at least in part of transgenic cells, are also an object of the present invention.

The invention further relates to plant propagating material of a plant according to the invention, which is treated with a seed protectant coating.

The invention further encompasses a microorganism transformed with a DNA molecule according to the invention, an expression cassette comprising the said DNA molecule or a vector molecule comprising the said expression cassette, wherein the said microorganism is preferably a microorganism that multiply on plants and more preferably a root colonizing bacterium.

A further embodiment of the invention relates to an encapsulated insect-specific protein which comprises a microorganism comprising an insect specific protein according to the invention.

The invention also relates to an entomocidal composition comprising a host organism of the invention, but preferably a purified Bacillus strain, in an insecticidally-effective amount together with a suitable carrier.

Further comprised by the invention is an entomocidal composition comprising an isolated protein molecule according to the invention, alone or in combination with a host organism of the invention and/or an encapsulated insect-specific protein according to the invention, in an insecticidally-effective amount, together with a suitable carrier.

A further embodiment of the invention relates to a method of obtaining a purified insect-specific protein according to the invention, said method comprising applying a solution comprising said insect-specific protein to a NAD column and eluting bound protein.

Also comprised is a method for identifying insect activity of an insect-specific protein according to the invention, said method comprising:
growing a *Bacillus* strain in a culture;
obtaining supernatant from said culture;
allowing insect larvae to feed on diet with said supernatant; and,
determining mortality.

Another aspect of the invention relates to a method for isolating an insect-specific protein according to the invention, said method comprising:
growing a *Bacillus* strain in a culture;
obtaining supernatant from said culture; and,
isolating said insect-specific protein from said supernatant.

The invention also encompasses a method for isolating a DNA molecule comprising a nucleotide sequence encoding an insect-specific protein exhibiting the insecticidal activity of the proteins according to the invention, said method comprising:
obtaining a DNA molecule comprising a nucleotide sequence encoding an insect-specific protein; and
hybridizing said DNA molecule with DNA obtained from a Bacillus species; and
isolating said hybridized DNA.

The invention further relates to a method of increasing insect target range by using an insect specific protein according to the invention in combination with at least one second insecticidal protein that is different from the insect specific protein according to the invention, but preferably with an insecticidal protein selected from the group consisting of *Bt* δ-endotoxins, protease inhibitors, lectins, α-amylases and peroxidases.

Preferred is a method for increasing insect target range within a plant by expressing within the said plant a insect specific protein according to the invention in combination with at least one second insecticidal protein that is different from the insect specific protein according to the invention, but preferably with an insecticidal protein selected from the group consisting of *Bt* δ-endotoxins, protease inhibitors, lectins, α-amylases and peroxidases.

Also comprised is a method of protecting plants against damage caused by an insect pest, but preferably by *Spodoptera* and/or *Agrotis* species, and more preferably by an insect pest selected from the group consisting of black cutworm [*Agrotis ipsilon* ; BCW], fall armyworm [*Spodoptera frugiperda*], beet armyworm [*Spodoptera exigua* ], tobacco budworm and corn earworm [*Helicoverpa zea*] comprising applying to the plant or the growing area of the said plant an entomocidal composition or a toxin protein according to the invention.

The invention further relates to method of protecting plants against damage caused by an insect pest, but preferably by *Spodoptera* and/or *Agrotis* species, and more preferably by an insect pest selected from the group consisting of black cutworm [*Agrotis ipsilon* ; BCW], fall armyworm [*Spodoptera frugiperda*], beet armyworm [*Spodoptera exigua*], tobacco budworm and corn earworm [*Helicoverpa zea*] comprising planting a transgenic plant expressing a insect-specific protein according to the invention within an area where the said insect pest may occur.

The invention also encompasses a method of producing a host organism which comprises stably integrated into its genome a DNA molecule according to the invention and preferably expresses an insect-specific protein according to the invention comprising transforming the said host organism with a DNA molecule according to the invention, an expression cassette comprising the said DNA molecule or a vector molecule comprising the said expression cassette.

A further embodiment of the invention relates to a method of producing a transgenic plant or plant cell which comprises stably integrated into the plant genome a DNA molecule according to the invention and preferably expresses an insect-specific protein according to the invention comprising transforming the said plant and plant cell, respectively, with a DNA molecule according to the invention, an expression cassette comprising the said DNA molecule or a vector molecule comprising the said expression cassette.

The invention also relates to a method of producing an entomocidal composition comprising mixing an isolated *Bacillus* strain and/or a host organism and/or an isolated protein molecule, and/or an encapsulated protein according to the invention in an insecticidally-effective amount with a suitable carrier.

The invention also encompasses a method of producing transgenic progeny of a transgenic parent plant comprising stably incorporated into the plant genome a DNA molecule comprising a nucleotide sequence encoding an insect-specific protein according to the invention comprising transforming the said parent plant with a DNA molecule according to the invention, an expression cassette comprising the said DNA molecule or a vector molecule comprising the said expression cassette and transferring the pesticidal trait to the progeny of the said transgenic parent plant involving known plant breeding techniques.

Also encompassed by the invention is oligonucleotide probe capable of specifically hybridizing to a nucleotide sequence encoding a insect-specific protein isolatable during the vegetative growth phase of *Bacillus* spp. and components thereof, wherein said protein is not the mosquitocidal toxin from *B. sphaericus* SSII-1, wherein said probe comprises a contiguous portion of the coding sequence for the said insect-specific protein at least 10 nucleotides in length and the use of the said oligonucleotide probe for screening of any *Bacillus* strain or other organisms to determine whether the insect-specific protein is naturally present or whether a particular transformed organism includes the said gene.

The present invention recognizes that pesticidal proteins are produced during vegetative growth of *Bacillus* strains, hereinafter referred to as VIPs.

The present VIPs are not abundant after sporulation and are particularly expressed during log phase growth before stationary phase. For the purpose of the present invention vegetative growth is defined as that period of time before the onset of sporulation. Genes encoding such VIPs can be isolated, cloned and transformed into various delivery vehicles for use in pest management programs.

For purposes of the present invention, pests include but are not limited to insects, fungi, bacteria, nematodes, mites, ticks, protozoan pathogens, animal-parasitic liver flukes, and the like. Insect pests include insects selected from the orders Coleoptera, Diptera, Hymenoptera, Lepidoptera, Mallophaga, Homoptera, Hemiptera, Orthroptera, Thysanoptera, Dermaptera, Isoptera, Anoplura, Siphonaptera, Trichoptera, etc., particularly Coleoptera and Lepidoptera.

Tables 1 - 10 gives a list of pests associated with major crop plants and pests of human and veterinary importance. Such pests are included within the scope of the present invention.

**TABLE 1**

| Lepidoptera (Butterflies and Moth) |
|---|
| Maize |
| *Ostrinia nubilalis*, European corn borer |
| *Agrotis ipsilon*, black cutworm |
| *Helicoverpa zea*, corn earworm |
| *Spodoptera frugiperda*, fall armyworm |
| *Diatraea grandiosella*, southwestern corn borer |
| *Elasmopalpus lignosellus,* lesser cornstalk borer |
| *Diatraea saccharalis*, sugarcane borer |
| |

| Sorghum |
|---|
| *Chilo partellus*, sorghum borer |
| *Spodoptera frugiperda*, fall armyworm |
| *Helicoverpa zea*, corn earworm |
| *Elasmopalpus lignosellus*, lesser cornstalk borer |
| *Feltia subterranea*, granulate cutworm |
| |

| Wheat |
|---|
| *Pseudaletia unipunctata*, army worm |
| *Spodoptera frugiperda*, fall armyworm |
| *Elasmopalpus lignosellus,* lesser cornstalk borer |
| *Agrotis orthogonia*, pale western cutworm |
| *Elasmopalpus lignosellus*, lesser cornstalk borer |
| |

| Sunflower |
|---|
| *Suleima helianthana*, sunflower bud moth |
| *Homoeosoma electellum*, sunflower moth |
| |

| Cotton |
|---|
| *Heliothis virescens,* cotton boll worm |
| *Helicoverpa zea*, cotton bollworm |
| *Spodoptera exigua,* beet armyworm |
| *Pectinophora gossypiella*, pink bollworm |
| |

| Rice |
|---|
| *Diatraea saccharalis*, sugarcane borer |
| *Spodoptera frugiperda*, fall armyworm |
| *Helicoverpa zea,* corn earworm |

| Soybean |
|---|
| *Pseudoplusia includens*, soybean looper |
| *Anticarsia gemmatalis*, velvetbean caterpillar |
| *Plathypena scabra,* green cloverworm |
| *Ostrinia nubilalis*, European corn borer |
| *Agrotis ipsilon*, black cutworm |
| *Spodoptera exigua*, beet armyworm |
| *Heliothis virescens,* cotton boll worm |
| *Helicoverpa zea*, cotton bollworm |
| |

| Barley |
|---|
| *Ostrinia nubilalis*, European corn borer |
| *Agrotis ipsilon*, black cutworm |

**TABLE 2**

| Coleoptera (Beetles) |
|---|
| Maize |
| *Diabrotica virgifera virgifera*, western corn rootworm |
| *Diabrotica longicornis barberi*, northern corn rootworm |
| *Diabrotica undecimpunctata howardi*, southern corn rootworm |
| *Melanotus spp*., wireworms |
| *Cyclocephala borealis*, northern masked chafer (white grub) |
| *Cyclocephala immaculata,* southern masked chafer (white grub) |
| *Popillia japonica*, Japanese beetle |
| *Chaetocnema pulicaria,* corn flea beetle |
| *Sphenophorus maidis*, maize billbug |
| |

| Sorghum |
|---|
| *Phyllophaga crinita*, white grub |
| *Eleodes, Conoderus,* and *Aeolus spp*., wireworms |
| *Oulema melanopus,* cereal leaf beetle |
| *Chaetocnema pulicaria*, corn flea beetle |
| *Sphenophorus maidis*, maize billbug |
| |

| Wheat |
|---|
| *Oulema melanopus*, cereal leaf beetle |
| *Hypera punctata,* clover leaf weevil |
| *Diabrotica undecimpunctata howardi*, southern corn rootworm |
| |

| Sunflower |
|---|
| *Zygogramma exclamationis*, sunflower beetle |
| *Bothyrus gibbosus,* carrot beetle |
| |

| Cotton |
|---|
| *Anthonomus grandis*, boll weevil |
| |

| Rice |
|---|
| *Colaspis brunnea,* grape colaspis |
| *Lissorhoptrus oryzophilus*, rice water weevil |
| *Sitophilus oryzae*, rice weevil |
| |

| Soybean |
|---|
| *Epilachna varivestis,* Mexican bean beetle |

**TABLE 3**

| Homoptera (Whiteflies, Aphids etc..) |
|---|
| Maize |
| *Rhopalosiphum maidis*, corn leaf aphid |
| *Anuraphis maidiradicis*, corn root aphid |
| |

| Sorghum |
|---|
| *Rhopalosiphum maidis*, corn leaf aphid |
| *Sipha flava*, yellow sugarcane aphid |
| |

| Wheat |
|---|
| Russian wheat aphid |
| *Schizaphis graminum,* greenbug |
| *Macrosiphum avenae*, English grain aphid |
| |

| Cotton |
|---|
| *Aphis gossypii*, cotton aphid |
| *Pseudatomoscelis seriatus,* cotton fleahopper |
| *Trialeurodes abutilonea*, bandedwinged whitefly |
| |

| Rice |
|---|
| *Nephotettix nigropictus,* rice leafhopper |

| Soybean |
|---|
| *Myzus persicae*, green peach aphid |
| *Empoasca fabae*, potato leafhopper |
| |

| Barley |
|---|
| *Schizaphis graminum,* greenbug |
| |

| Oil Seed Rape |
|---|
| *Brevicoryne brassicae*, cabbage aphid |

**TABLE 4**

| Hemiptera (Bugs) |
|---|
| Maize |
| *Blissus leucopterus leucopterus,* chinch bug |
| |

| Sorghum |
|---|
| *Blissus leucopterus leucopterus,* chinch bug |
| |

| Cotton |
|---|
| *Lygus lineolaris*, tarnished plant bug |
| |

| Rice |
|---|
| *Blissus leucopterus leucopterus,* chinch bug |
| *Acrosternum hilare*, green stink bug |
| |

| Soybean |
|---|
| *Acrosternum hilare*, green stink bug |
| |

| Barley |
|---|
| *Blissus leucopterus leucopterus,* chinch bug |
| *Acrosternum hilare,* green stink bug |
| *Euschistus servus,* brown stink bug |

**TABLE 5**

| Orthoptera (Grasshoppers, Crickets, and Cockroaches) |
|---|
| Maize |
| *Melanoplus femurrubrum,* redlegged grasshopper |
| *Melanoplus sanguinipes*, migratory grasshopper |
| |

| Wheat |
|---|
| *Melanoplus femurrubrum,* redlegged grasshopper |
| *Melanoplus differentialis*, differential grasshopper |
| *Melanoplus sanguinipes,* migratory grasshopper |
| |

| Cotton |
|---|
| *Melanoplus femurrubrum,* redlegged grasshopper |
| *Melanoplus differentialis*, differential grasshopper |
| |

| Soybean |
|---|
| *Melanoplus femurrubrum,* redlegged grasshopper |
| *Melanoplus differentialis*, differential grasshopper |
| |

| Structural/Household |
|---|
| *Periplaneta americana,* American cockroach |
| *Blattella germanica*, German cockroach |
| *Blatta orientalis*, oriental cockroach |

**TABLE 6**

| Diptera (Flies and Mosguitoes) |
|---|
| Maize |
| *Hylemya platura*, seedcorn maggot |
| *Agromyza parvicornis*, corn blotch leafminer |
| |

| Sorghum |
|---|
| *Contarinia sorghicola*, sorghum midge |

| Wheat |
|---|
| *Mayetiola destructor,* Hessian fly |
| *Sitodiplosis mosellana*, wheat midge |
| *Meromyza americana,* wheat stem maggot |
| *Hylemya coarctata*, wheat bulb fly |
| |

| Sunflower |
|---|
| *Neolasioptera murtfeldtiana*, sunflower seed midge |
| |

| Soybean |
|---|
| *Hylemya platura,* seedcorn maggot |
| |

| Barley |
|---|
| *Hylemya platura*, seedcorn maggot |
| *Mayetiola destructor,* Hessian fly |
| |

| Insects attacking humans and animals and disease carriers |
|---|
| *Aedes aegypti*, yellowfever mosquito |
| *Aedes albopictus*, forest day mosquito |
| *Phlebotomus papatasii*, sand fly |
| *Musca domestica,* house fly |
| *Tabanus atratus,* black horse fly |
| *Cochliomyia hominivorax*, screwworm fly |

**TABLE 7**

| Thysanoptera (Thrips) |
|---|
| Maize |
| *Anaphothrips obscurus,* grass thrips |
| |

| Wheat |
|---|
| *Frankliniella fusca,* tobacco thrips |
| |

| Cotton |
|---|
| *Thrips tabaci,* onion thrips |
| *Frankliniella fusca*, tobacco thrips |

| Soybean |
|---|
| *Sericothrips variabilis*, soybean thrips |
| *Thrips tabaci,* onion thrips |

**TABLE 8**

| Hymenoptera (Sawflies, Ants, Wasps, etc.) |
|---|
| Maize |
| *Solenopsis milesta,* thief ant |
| |

| Wheat |
|---|
| *Cephus cinctus,* wheat stem sawfly |

**TABLE 9**

| Other Orders and Representative Species |
|---|
| *Dermaptera* (Earwigs) |
| *Forficula auricularia,* European earwig |
| |

| *Isoptera* (Termites) |
|---|
| *Reticulitermes flavipes*, eastern subterranean termite |
| |

| *Mallophaga* (Chewing Lice) |
|---|
| *Cuclotogaster heterographa*, chicken head louse |
| *Bovicola bovis,* cattle biting louse |
| |

| *Anoplura* (Sucking Lice) |
|---|
| Pediculus humanus, head and body louse |
| |

| *Siphonaptera* (Fleas) |
|---|
| *Ctenocephalides felis,* cat flea |

**TABLE 10**

| Acari (Mites and Ticks) |
|---|
| Maize |
| *Tetranychus urticae,* twospotted spider mite |
| |

| Sorghum |
|---|
| *Tetranychus cinnabarinus,* carmine spider mite |
| *Tetranychus urticae*, twospotted spider mite |
| |

| Wheat |
|---|
| *Aceria tulipae,* wheat curl mite |
| |

| Cotton |
|---|
| *Tetranychus cinnabarinus*, carmine spider mite |
| *Tetranychus urticae*, twospotted spider mite |
| |

| Soybean |
|---|
| *Tetranychus turkestani,* strawberry spider mite |
| *Tetranychus urticae*, twospotted spider mite |
| |

| Barley |
|---|
| *Petrobia latens,* brown wheat mite |
| |

| Important human and animal *Acari* |
|---|
| *Demacentor variabilis*, American dog tick |
| *Argas persicus,* fowl tick |
| *Dermatophagoides farinae*, American house dust mite |
| *Dermatophagoides pteronyssinus*, European house dust mite |

Now that it has been recognized that pesticidal proteins can be isolated from the vegetative growth phase of *Bacillus,* other strains can be isolated by standard techniques and tested for activity against particular plant and non-plant pests. Generally *Bacillus* strains can be isolated from any environmental sample, including soil, plant, insect, grain elevator dust, and other sample material, etc., by methods known in the art. See, for example, Travers *et al*. (1987) Appl. Environ. Microbiol. 53:1263-1266; Saleh *et al*. (1969) Can J. Microbiol. 15:1101-1104; DeLucca *et al*. (1981) Can. J. Microbiol. 27:865-870; and Norris, *et al*. (1981) "The genera *Bacillus* and *Sporolactobacillus,*" In Starr *et al.* (eds.), The Prokaryotes: A Handbook on Habitats, Isolation, and Identification of Bacteria, Vol. II, Springer-Verlog Berlin Heidelberg. After isolation, strains can be tested for pesticidal activity during vegetative growth. In this manner, new pesticidal proteins and strains can be identified.

Such *Bacillus* microorganisms which find use in the invention include *Bacillus* cereus and *Bacillus thuringiensis,* as well as those *Bacillus* species listed in Table 11.

**TABLE 11**

| List of *Bacillus* species |
|---|
| Morphological Group 1 |
| *B. megaterium* |
| *B. cereus** |
| *B. cereus var. mycoides* |
| *B. thuringiensis** |
| *B. licheniformis* |
| *B. subtilis** |
| *B. pumilus* |
| B. *firmus** |
| *B. coagulans* |
| |

| Morphological Group 2 |
|---|
| *B. polymyxa* |
| *B. macerans* |
| *B. circulans* |
| *B. stearothermophilus* |
| *B. alvei** |
| *B. laterosporus** |
| *B. brevis* |
| *B. pulvifaciens* |
| *B. popilliae** |
| B. *lentimorbus** |
| *B. larvae** |

| Morphological Group 3 |
|---|
| *B. sphaericus** |
| *B. pasteurii* |
| |

| Unassigned Strains |
|---|
| Subgroup A |
| *B. apiarus** |
| *B. filicolonicus* |
| *B. thiaminolyticus* |
| *B. alcalophilus* |
| |

| Subgroup B |
|---|
| *B. cirroflagellosus* |
| *B. chitinosporus* |
| *B. lentus* |
| |

| Subgroup C |
|---|
| *B. badius* |
| *B. aneurinolyticus* |
| *B. macroides* |
| *B. freundenreichii* |
| |

| Subgroup D |
|---|
| *B. pantothenticus* |
| *B. epiphytus* |
| |

| Subgroup E1 |
|---|
| *B. aminovorans* |
| *B. globisporus* |
| *B. insolitus* |
| *B. psychrophilus* |
| |

| Subgroup E2 |
|---|
| *B. psychrosaccharolyticus* |
| *B. macquariensis* |

| |
|---|
| *=Those *Bacillus* strains that have been previously found associated with insects Grouping according to Parry, J.M. *et al*. (1983) Color Atlas of *Bacillus* species, Wolfe Medical Publications, London. |

In accordance with the present invention, the pesticidal proteins produced during vegetative growth can be isolated from *Bacillus.* In one embodiment, insecticidal proteins produced during vegetative growth, can be isolated. Methods for protein isolation are known in the art. Generally, proteins can be purified by conventional chromatography, including gel-filtration, ion-exchange, and immunoaffinity chromatography, by high-performance liquid chromatography, such as reversed-phase high-performance liquid chromatography, ion-exchange high-performance liquid chromatography, size-exclusion high-performance liquid chromatography, high-performance chromatofocusing and hydrophobic interaction chromatography, etc., by electrophoretic separation, such as one-dimensional gel electrophoresis, two-dimensional gel electrophoresis, etc. Such methods are known in the art. See for example Current Protocols in Molecular Biology, Vols. 1 and 2, Ausubel *et al*. (eds.), John Wiley & Sons, NY (1988). Additionally, antibodies can be prepared against substantially pure preparations of the protein. See, for example, Radka *et al*. (1983) J. Immunol. 128:2804; and Radka *et al.* (1984) Immunogenetios 19:63. Any combination of methods may be utilized to purify protein having pesticidal properties. As the protocol is being formulated, pesticidal activity is determined after each purification step.

Such purification steps will result in a substantially purified protein fraction. By "substantially purified" or "substantially pure" is intended protein which is substantially free of any compound normally associated with the protein in its natural state. "Substantially pure" preparations of protein can be assessed by the absence of other detectable protein bands following SDS-PAGE as determined visually or by densitometry scanning. Alternatively, the absence of other amino-terminal sequences or N-terminal residues in a purified preparation can indicate the level of purity. Purity can be verified by rechromatography of "pure" preparations showing the absence of other peaks by ion exchange, reverse phase or capillary electrophoresis. The terms "substantially pure" or "substantially purified" are not meant to exclude artificial or synthetic mixtures of the proteins with other compounds. The terms are also not meant to exclude the presence of minor impurities which do not interfere with the biological activity of the protein, and which may be present, for example, due to incomplete purification.

Once purified protein is isolated, the protein, or the polypeptides of which it is comprised, can be characterized and sequenced by standard methods known in the art. For example, the purified protein, or the polypeptides of which it is comprised, may be fragmented as with cyanogen bromide, or with proteases such as papain, chymotrypsin, trypsin, lysyl-C endopeptidase, etc. (Oike *et al*. (1982) J. Biol. Chem. 257:9751-9758; Liu *et al*. (1983) Int. J. Pept. Protein Res. 21:209-215). The resulting peptides are separated, preferably by HPLC, or by resolution of gels and electroblotting onto PVDF membranes, and subjected to amino acid sequencing. To accomplish this task, the peptides are preferably analyzed by automated sequenators. It is recognized that N-terminal, C-terminal, or internal amino acid sequences can be determined. From the amino acid sequence of the purified protein, a nucleotide sequence can be synthesized which can be used as a probe to aid in the isolation of the gene encoding the pesticidal protein.

It is recognized that the pesticidal proteins may be oligomeric and will vary in molecular weight, number of protomers, component peptides, activity against particular pests, and in other characteristics. However, by the methods set forth herein, proteins active against a variety of pests may be isolated and characterized.

Once the purified protein has been isolated and characterized it is recognized that it may be altered in various ways including amino acid substitutions, deletions, truncations, and insertions. Methods for such manipulations are generally known in the art. For example, amino acid sequence variants of the pesticidal proteins can be prepared by mutations in the DNA. Such variants will possess the desired pesticidal activity. Obviously, the mutations that will be made in the DNA encoding the variant must not place the sequence out of reading frame and preferably will not create complementary regions that could produce secondary mRNA structure. See, EP Patent Application Publication No. 75,444.

In this manner, the present invention encompasses the pesticidal proteins as well as components and fragments thereof. That is, it is recognized that component protomers, polypeptides or fragments of the proteins may be produced which retain pesticidal activity. These fragments include truncated sequences, as well as N-terminal, C-terminal, internal and internally deleted amino acid sequences of the proteins.

Most deletions, insertions, and substitutions of the protein sequence are not expected to produce radical changes in the characteristics of the pesticidal protein. However, when it is difficult to predict the exact effect of the substitution, deletion, or insertion in advance of doing so, one skilled in the art will appreciate that the effect will be evaluated by routine screening assays.

The proteins or other component polypeptides described herein may be used alone or in combination. That is, several proteins may be used to control different insect pests.

Some proteins are single polypeptide chains while many proteins consist of more than one polypeptide chain, i.e., they are oligomeric. Additionally, some VIPs are pesticidally active as oligomers. In these instances, additional protomers are utilized to enhance the pesticidal activity or to activate pesticidal proteins. Those protomers which enhance or activate are referred to as auxiliary proteins. Auxiliary proteins activate or enhance a pesticidal protein by interacting with the pesticidal protein to form an oligomeric protein having increased pesticidal activity compared to that observed in the absence of the auxiliary protein.

Amongst the pesticidal proteins of the invention a new class of insect-specific proteins could be surprisingly identified within the scope of the present invention. The said proteins, which are designated throughout this application as VIP3, can be obtained from *Bacillus spp* strains, but preferably from *Bacillus thuringiensis* strains and most preferably from *Bacillus thuringiensis* strains AB88 and AB424. The said VIPs are present mostly in the supernatants of *Bacillus* cultures amounting to at least 75% of the total in strain AB88. The VIP3 proteins are further characterized by their unique spectrum of insectical acitivity, which includes an activity against *Agrotis* and/or *Spodoptera* species, but especially a black cutworm [BCW] and/or fall armyworm and/or beet armyworm and/or tobacco budworm and/or corn earworm activity.

Black cutworm is an agronomically important insect quite resistant to δ-endotoxins. Maclntosh et al (1990) J Invertebr Pathol 56, 258-266 report that the δ-endotoxins CrylA(b) and CrylA(c) possesses insecticidal properties against BCW with LC₅₀ of more than 80 µg and 18 µg/ml of diet respectively. The vip3A insecticidal proteins according to the invenition provide >50% mortality when added in an amount of protein at least 10 to 500, preferably 50 to 350, and more preferably 200 to 300 fold lower than the amount of CrylA proteins needed to achieve just 50% mortality. Especially preferred within the invention are vip3A insecticidal proteins which provide 100% mortality when added in an amount of protein at least 260 fold lower than the amount of CrylA proteins needed to achieve just 50% mortality.

The vip3 insecticidal proteins according to the invention are present mostly in the supernatants of the cultures and are therefore are to be classified as secreted proteins. They preferably contain in the N-terminal sequence a number of positively charged residues followed by a hydrophobic core region and are not N-terminally processed during export.

As the other pesticidal proteins reported hereto within the scope of the invention, the VIP3 proteins can be detected in growth stages prior to sporulation establishing a further clear distinction from other proteins that belong to the δ-endotoxin family. Preferably, expression of the insect-specific protein starts during mid-log phase and continues during sporulation. Owing to the specific expression pattern in combination with the high stability of the VIP3 proteins, large amounts of the VIP3 proteins can be found in supernatants of sporulating cultures. Especially preferred are the VIP3 proteins identified in SEQ ID NO:2 and SEQ ID NO:5 and the corresponding DNA molecules comprising nucleotide sequences encoding the said proteins, but especially those DNA molecules comprising the nucleotide sequences given in SEQ ID NO:1, SEQ ID NO:3 and SEQ ID NO:4.

The pesticidal proteins of the invention can be used in combination with Bt endotoxins or other insecticidal proteins to increase insect target range. Furthermore, the use of the VIPs of the present invention in combination with Bt δ-endotoxins or other insecticidal principles of a distinct nature has particular utility for the prevention and/or management of insect resistance. Other insecticidal principles include protease inhibitors (both serine and cysteine types), lectins, α-amylase and peroxidase. In one preferred embodiment, expression of VIPs in a transgenic plant is accompanied by the expression of one or more Bt δ-endotoxins. This co-expression of more than one insecticidal principle in the same transgenic plant can be achieved by genetically engineering a plant to contain and express all the genes necessary. Alternatively, a plant, Parent 1, can be genetically engineered for the expression of VIPs. A second plant, Parent 2, can be genetically engineered for the expression of Bt δ-endotoxin. By crossing Parent 1 with Parent 2, progeny plants are obtained which express all the genes introduced into Parents 1 and 2. Particularly preferred Bt δ-endotoxins are those disclosed in EP-A 0618976, herein incorporated by reference.

A substantial number of cytotoxic proteins, though not all, are binary in action. Binary toxins typically consist of two protein domains, one called the A domain and the other called the B domain (see Sourcebook of Bacterial Protein Toxins, J. E. Alouf and J. H. Freer eds.(1991) Academic Press). The A domain possesses a potent cytotoxic activity. The B domain binds an external cell surface receptor before being internalized. Typically, the cytotoxic A domain must be escorted to the cytoplasm by a translocation domain. Often the A and B domains are separate polypeptides or protomers, which are associated by a protein-protein interaction or a di-sulfide bond. However, the toxin can be a single polypeptide which is proteolytically processed within the cell into two domains as in the case for *Pseudomonas* exotoxin A. In summary binary toxins typically have three important domains, a cytotoxic A domain, a receptor binding B domain and a translocation domain. The A and B domain are often associated by protein-protein interacting domains.

The receptor binding domains of the present invention are useful for delivering any protein, toxin, enzyme, transcription factor, nucleic acid, chemical or any other factor into target insects having a receptor recognized by the receptor binding domain of the binary toxins described in this patent. Similarly, since binary toxins have translocation domains which penetrate phosopholipid bilayer membranes and escort cytotoxins across those membranes, such translocation domains may be useful in escorting any protein, toxin, enzyme, transcription factor, nucleic acid, chemical or any other factor across a phospholipid bilayer such as the plasma membrane or a vesicle membrane. The translocation domain may itself perforate membranes, thus having toxic or insecticidal properties. Further, all binary toxins have cytotoxic domains; such a cytotoxic domain may be useful as a lethal protein, either alone or when delivered into any target cell(s) by any means.

Finally, since binary toxins comprised of two polypeptides often form a complex, it is likely that there are protein-protein interacting regions within the components of the binary toxins of the invention. These protein-protein interacting domains may be useful in forming associations between any combination of toxins, enzymes, transcription factors, nucleic acids, antibodies, cell binding moieties, or any other chemicals, factors, proteins or protein domains.

Toxins, enzymes, transcription factors, antibodies, cell binding moieties or other protein domains can be fused to pesticidal or auxiliary proteins by producing in frame genetic fusions which, when translated by ribosomes, would produce a fusion protein with the combined attributes of the VIP and the other component used in the fusion. Furthermore, if the protein domain fused to the VIP has an affinity for another protein, nucleic acid, carbohydrate, lipid, or other chemical or factor, then a three-component complex can be formed. This complex will have the attributes of all of its components. A similar rationale can be used for producing four or more component complexes. These complexes are useful as insecticidal toxins, pharmaceuticals, laboratory reagents, and diagnostic reagents, etc. Examples where such complexes are currently used are fusion toxins for potential cancer therapies, reagents in ELISA assays and immunoblot analysis.

One strategy of altering pesticidal or auxiliary proteins is to fuse a 15-amino-acid "S-tag" to the protein without destroying the insect cell binding domain(s), translocation domains or protein-protein interacting domains of the proteins. The S-tag has a high affinity (K_{d} = 10⁻⁹ M) for a ribonuclease S-protein, which, when bound to the S-tag, forms an active ribonuclease (See F. M. Richards and H. W. Wyckoff (1971) in "The Enzymes", Vol. IV (Boyer, P.D. ed.). pp. 647-806. Academic Press, New York). The fusion can be made in such a way as to destroy or remove the cytotoxic activity of the pesticidal or auxiliary protein, thereby replacing the VIP cytotoxic activity with a new cytotoxic ribonuclease activity. The final toxin would be comprised of the S-protein, a pesticidal protein and an auxiliary protein, where either the pesticidal protein or the auxiliary protein is produced as translational fusions with the S-tag. Similar strategies can be used to fuse other potential cytotoxins to pesticidal or auxiliary proteins including (but not limited to) ribosome inactivating proteins, insect hormones, hormone receptors, transcription factors, proteases, phosphatases, *Pseudomonas* exotoxin A, or any other protein or chemical factor that is lethal when delivered into cells. Similarly, proteins can be delivered into cells which are not lethal, but might after cellular biochemistry or physiology.

The spectrum of toxicity toward different species can be altered by fusing domains to pesticidal or auxiliary proteins which recognize cell surface receptors from other species. Such domains might include (but are not limited to) antibodies, transferrin, hormones, or peptide sequences isolated from phage displayed affinity selectable libraries. Also, peptide sequences which are bound to nutrients, vitamins, hormones, or other chemicals that are transported into cells could be used to alter the spectrum of toxicity.

The pesticidal proteins of the present invention are those proteins which confer a specific pesticidal property. Such proteins may vary in molecular weight, having component polypeptides at least a molecular weight of 30 kDa or greater, preferably about 50 kDa or greater.

It is possible that the pesticidal protein may be a component of a multimeric, pesticidal protein. Such a pesticidal protein may vary in molecular weight, having at least a molecular weight of 50 kDa up to at least 200 kDa, preferably about 100 kDa to 150 kDa.

For purposes of the invention, the term "Vegetative Insecticidal Protein" (VIP) encompasses those proteins produced during vegetative growth which alone or in combination can be used for pesticidal activity. This includes pesticidal proteins, auxiliary proteins and those proteins which demonstrate activity only in the presence of the auxiliary protein or the polypeptide components of these proteins.

It is recognized that there are alternative methods available to obtain the nucleotide and amino acid sequences of the present proteins. For example, to obtain the nucleotide sequence encoding the pesticidal protein, cosmid clones, which express the pesticidal protein, can be isolated from a genomic library. From larger active cosmid clones, smaller subclones can be made and tested for activity. In this manner, clones which express an active pesticidal protein can be sequenced to determine the nucleotide sequence of the gene. Then, an amino acid sequence can be deduced for the protein. For general molecular methods, see, for example, Molecular Cloning, A Laboratory Manual, Second Edition, Vols. 1-3, Sambrook *et al*. (eds.) Cold Spring Harbor Laboratory Press, Cold Spring Harbor, NY (1989), and the references cited therein.

The present invention also encompasses nucleotide sequences from organisms other than *Bacillus,* where the nucleotide sequences are isolatable by hybridization with the *Bacillus* nucleotide sequences of the invention. Proteins encoded by such nucleotide sequences can be tested for pesticidal activity. The invention also encompasses the proteins encoded by the nucleotide sequences. Furthermore, the invention encompasses proteins obtained from organisms other than *Bacillus* wherein the protein cross-reacts with antibodies raised against the proteins of the invention. Again the isolated proteins can be assayed for pesticidal activity by the methods disclosed herein or others well-known in the art.

Once the nucleotide sequences encoding the pesticidal proteins of the invention have been isolated, they can be manipulated and used to express the protein in a variety of hosts including other organisms, including microorganisms and plants.

The pesticidal genes of the invention can be optimized for enhanced expression in plants. See, for example EP-A 0618976; EP-A 0359472; EP-A 0385962; WO 91/16432; Perlak et al. (1991) Proc. Natl. Acad. Sci. USA 88:3324-3328; and Murray et al. (1989) Nucleic Acids Research 17: 477-498. In this manner, the genes can be synthesized utilizing plant preferred codons. That is the preferred codon for a particular host is the single codon which most frequently encodes that amino acid in that host. The maize preferred codon, for example, for a particular amino acid may be derived from known gene sequences from maize. Maize codon usage for 28 genes from maize plants is found in Murray et al. (1989), Nucleic Acids Research 17:477-498, the disclosure of which is incorporated herein by reference. Synthetic genes can also be made based on the distribution of codons a particular host uses for a particular amino acid.

In this manner, the nucleotide sequences can be optimized for expression in any plant. It is recognized that all or any part of the gene sequence may be optimized or synthetic. That is, synthetic or partially optimized sequences may also be used.

In like manner, the nucleotide sequences can be optimized for expression in any microorganism. For *Bacillus* preferred codon usage, see, for example US Patent No. 5,024,837 and Johansen et al. (1988) Gene 65:293-304.

Methodologies for the construction of plant expression cassettes as well as the introduction of foreign DNA into plants are described in the art. Such expression cassettes may include promoters, terminators, enhancers, leader sequences, introns and other regulatory sequences operably linked to the pesticidal protein coding sequence. It is further recognized that promoters or terminators of the VIP genes can be used in expression cassettes.

Generally, for the introduction of foreign DNA into plants Ti plasmid vectors have been utilized for the delivery of foreign DNA as well as direct DNA uptake, liposomes, electroporation, micro-injection, and the use of microprojectiles. Such methods had been published in the art. See, for example, Guerche et al., (1987) Plant Science 52:111-116; Neuhause et al., (1987) Theor. Appl. Genet. 75:30-36; Klein et al., (1987) Nature 327: 70-73; Howell et al., (1980) Science 208:1265; Horsch et al., (1985) Science 227: 1229-1231; DeBlock et al., (1989) Plant Physiology 91:694-701; Methods for Plant Molecular Biology (Weissbach and Weissbach, eds.) Academic Press, Inc. (1988); and Methods in Plant Molecular Biology (Schuler and Zielinski, eds.) Academic Press, Inc. (1989). See also US patent application serial no. 08/008,374 herein incorporated by reference. See also, EP-A 0193259 and EP-A 0451878. It is understood that the method of transformation will depend upon the plant cell to be transformed.

It is further recognized that the components of the expression cassette may be modified to increase expression. For example, truncated sequences, nucleotide substitutions or other modifications may be employed. See, for example Perlak et al. (1991) Proc. Natl. Acad. Sci. USA 88:3324-3328; Murray et al., (1989) Nucleic Acids Research 17:477-498; and WO 91/16432.

The construct may also include any other necessary regulators such as terminators, (Guerineau et al., (1991), Mol. Gen. Genet., 226:141-144; Proudfoot, (1991), Cell, 64:671-674; Sanfacon et al., (1991), Genes Dev., 5:141-149; Mogen et al., (1990), Plant Cell, 2:1261-1272; Munroe et al., (1990), Gene, 91:151-158; Ballas et al et al., (1989), Nucleic Acids Res., 17:7891-7903; Joshi et al., (1987), Nucleic Acid Res., 15:9627-9639); plant translational consensus sequences (Joshi, C.P., (1987), Nucleic Acids Research, 15:6643-6653), introns (Luehrsen and Walbot, (1991), Mol. Gen. Genet., 225:81-93) and the like, operably linked to the nucleotide sequence. It may be beneficial to include 5' leader sequences in the expression cassette construct. Such leader sequences can act to enhance translation. Translational leaders are known in the art and include:
Picornavirus leaders, for example, EMCV leader (encephalomyocarditis 5' noncoding region) (Elroy-Stein, O., Fuerst, T.R., and Moss, B. (1989) PNAS USA 86:6126-6130);
Potyvirus leaders, for example, TEV leader (Tobacco Etch Virus) (Allison *et al*., (1986); MDMV leader (Maize Dwarf Mosaic Virus); Virology, 154:9-20), and
Human immunoglobulin heavy-chain binding protein (BiP), (Macejak, D.G., and Sarnow, P., (1991), Nature, 353:90-94;
Untranslated leader from the coat protein mRNA of alfalfa mosaic virus (AMV RNA 4), (Jobling, S.A., and Gehrke, L., (1987), Nature, 325:622-625;
Tobacco mosaic virus leader (TMV), (Gallie, D.R. et al., (1989), Molecular Biology of RNA, pages 237-256; and
Maize Chlorotic Mottle Virus leader (MCMV) (Lommel, S.A. et al., (1991), Virology, 81:382-385. See also, Della-Cioppa et al., (1987), Plant Physiology, 84:965-968.

A plant terminator may be utilized in the expression cassette. See, Rosenberg et al., (1987), Gene, 56:125; Guerineau et al., (1991), Mol. Gen. Genet., 226:141-144; Proudfoot, (1991), Cell, 64:671-674; Sanfacon et al., (1991), Genes Dev., 5:141-149; Mogen et al., (1990), Plant Cell, 2:1261-1272; Munroe et al., (1990), Gene, 91:151-158; Ballas et al., (1989), Nucleic Acids Res., 17:7891-7903; Joshi et al., (1987), Nucleic Acid Res., 15:9627-9639.

For tissue specific expression, the nucleotide sequences of the invention can be operably linked to tissue specific promoters. See, for example, EP-A 0618976, herein incorporated by reference.

Further comprised within the scope of the present invention are transgenic plants, in particular transgenic fertile plants transformed by means of the aforedescribed processes and their asexual and/or sexual progeny, which comprise and preferably also express the pesticidal protein according to the invention. Especially preferred are hybrid plants.

The transgenic plant according to the invention may be a dicotyledonous or a monocotyledonous plant. Preferred are monocotyledonous plants of the *Graminaceae* family involving *Lolium, Zea*, *Triticum, Triticale, Sorghum, Saccharum, Bromus, Oryzae, Avena, Hordeum, Secale* and *Setaria* plants.

Especially preferred are transgenic maize, wheat, barley, sorghum, rye, oats, turf grasses and rice.

Among the dicotyledonous plants soybean, cotton, tobacco, sugar beet, oilseed rape, and sunflower are especially preferred herein.

The expression 'progeny' is understood to embrace both, "asexually" and "sexually" generated progeny of transgenic plants. This definition is also meant to include all mutants and variants obtainable by means of known processes, such as for example cell fusion or mutant selection and which still exhibit the characteristic properties of the initially transformed parent plant, together with all crossing and fusion products of the transformed plant material.

Another object of the invention concerns the proliferation material of transgenic plants.

The proliferation material of transgenic plants is defined relative to the invention as any plant material that may be propagated sexually or asexually *in vivo* or *in vitro.* Particularly preferred within the scope of the present invention are protoplasts, cells, calli, tissues, organs, seeds, embryos, pollen, egg cells, zygotes, together with any other propagating material obtained from transgenic plants.

Parts of plants, such as for example flowers, stems, fruits, leaves, roots originating in transgenic plants or their progeny previously transformed by means of the process of the invention and therefore consisting at least in part of transgenic cells, are also an object of the present invention.

Before the plant propagation material [fruit, tuber, grains, seed], but expecially seed is sold as a commerical product, it is customarily treated with a protectant coating comprising herbicides, insecticides, fungicides, bactericides, nematicides, molluscicides or mixtures of several of these preparations, if desired together with further carriers, surfactants or application-promoting adjuvants customarily employed in the art of formulation to provide protection against damage caused by bacterial, fungal or animal pests.

In order to treat the seed, the protectant coating may be applied to the seeds either by impregnating the tubers or grains with a liquid formulation or by coating them with a combined wet or dry formulation. In addition, in special cases, other methods of application to plants are possible, eg treatment directed at the buds or the fruit.

The plant seed according to the invention comprising a DNA molecule comprising a nucleotide sequence encoding a pesticidal protein according to the invention may be treated with a seed protectant coating comprising a seed treatment compound, such as, for example, captan, carboxin, thiram (TMTD®), methalaxyl (Apron®) and pirimiphos-methyl (Actellic®) and others that are commonly used in seed treatment. Preferred within the scope of the invention are seed protectant coatings comprising an entomocidal composition according to the invention alone or in combination with one of the a seed protectant coating customarily used in seed treatment.

It is thus a further object of the present invention to provide plant propagation material for cultivated plants, but especially plant seed that is treated with a seed protectant coating as defined hereinbefore.

It is recognized that the genes encoding the pesticidal proteins can be used to transform insect pathogenic organisms. Such organisms include Baculoviruses, fungi, protozoa, bacteria and nematodes.

The *Bacillus* strains of the invention may be used for protecting agricultural crops and products from pests. Alternatively, a gene encoding the pesticide may be introduced via a suitable vector into a microbial host, and said host applied to the environment or plants or animals. Microorganism hosts may be selected which are known to occupy the "phytosphere" (phylloplane, phyllosphere, rhizosphere, and/or rhizoplana) of one or more crops of interest. These microorganisms are selected so as to be capable of successfully competing in the particular environment with the wild-type microorganisms, provide for stable maintenance and expression of the gene expressing the polypeptide pesticide, and, desirably, provide for improved protection of the pesticide from environmental degradation and inactivation.

Such microorganisms include bacteria, algae, and fungi. Of particular interest are microorganisms, such as bacteria, e.g., *Pseudomonas, Erwinia, Serratia, Klebsiella*, *Xanthomonas, Streptomyces, Rhizobium, Rhodopseudomonas, Methylius, Agrobacterium, Acetobacter, Lactobacillus, Arthrobacter, Azotobacter, Leuconostoc,* and *Alcaligenes;* fungi, particularly yeast, e.g., *Saccharomyces, Cryptococcus*, *Kluyveromyces, Sporobolomyces, Rhodotorula,* and *Aureobasidium.* Of particular interest are such phytosphere bacterial species as *Pseudomonas syringae*, *Pseudomonas fluorescens, Serratia marcescens, Acetobacter xylinum, Agrobacteria, Rhodopseudomonas spheroides, Xanthomonas campestris, Rhizobium melioti, Alcaligenes entrophus, Clavibacter xyli and Azotobacter vinlandir,* and phytosphere yeast species such as *Rhodotorula rubra, R. glutinis, R. marina, R. aurantiaca*, *Cryptococcus albidus, C. diffluens, C. laurentii, Saccharomyces rosei, S. pretoriensis, S. cerevisiae, Sporobolomyces rosues, S. odorus, Kluyveromyces veronae*, and *Aureobasidium pollulans*. Of particular interest are the pigmented microorganisms.

A number of ways are available for introducing a gene expressing the pesticidal protein into the microorganism host under conditions which allow for stable maintenance and expression of the gene. For example, expression cassettes can be constructed which include the DNA constructs of interest operably linked with the transcriptional and translational regulatory signals for expression of the DNA constructs, and a DNA sequence homologous with a sequence in the host organism, whereby integration will occur, and/or a replication system which is functional in the host, whereby integration or stable maintenance will occur.

Transcriptional and translational regulatory signals include but are not limited to promoter, transcriptional initiation start site, operators, activators, enhancers, other regulatory elements, ribosomal binding sites, an initiation codon, termination signals, and the like. See, for example, US Patent 5,039,523; US Patent No. 4,853,331; EPO 0480762A2; Sambrook *et al*. supra; Molecular Cloning, a Laboratory Manual, Maniatis *et al.* (eds) Cold Spring Harbor Laboratory, Cold Spring Harbor, NY (1982); Advanced Bacterial Genetics, Davis *et al*. (eds.) Cold Spring Harbor Laboratory, Cold Spring Harbor, NY (1980); and the references cited therein.

Suitable host cells, where the pesticide-containing cells will be treated to prolong the activity of the toxin in the cell when the then treated cell is applied to the environment of the target pest(s), may include either prokaryotes or eukaryotes, normally being limited to those cells which do not produce substances toxic to higher organisms, such as mammals. However, organisms which produce substances toxic to higher organisms could be used, where the toxin is unstable or the level of application sufficiently low as to avoid any possibility of toxicity to a mammalian host. As hosts, of particular interest will be the prokaryotes and the lower eukaryotes, such as fungi. Illustrative prokaryotes, both Gram-negative and -positive, include *Enterobacteriaceae,* such as *Escherichia, Erwinia, Shigella, Salmonella,* and *Proteus*; *Bacillaceae; Rhizobiceae, such as Rhizobium; Spirillaceae,* such as photobacterium, *Zymomonas, Serratia, Aeromonas, Vibrio, Desulfovibrio, Spirillum; Lactobacillaceae; Pseudomonadaceae,* such as *Pseudomonas* and *Acetobacter; Azotobacteraceae* and *Nitrobacteraceae.* Among eukaryotes are fungi, such as *Phycomycetes* and *Ascomycetes,* which includes yeast, such a *Saccharomyces* and *Schizosaccharromyces;* and *Basidiomycetes* yeast, such as *Rhodotorula, Aureobasidium, Sporobolomyces,* and the like.

Characteristics of particular interest in selecting a host cell for purposes of production include ease of introducing the protein gene into the host, availability of expression systems, efficiency of expression, stability of the protein in the host, and the presence of auxiliary genetic capabilities. Characteristics of interest for use as a pesticide microcapsule include protective qualities for the pesticide, such as thick cell walls, pigmentation, and intracellular packaging or formation of inclusion bodies; leaf affinity; lack of mammalian toxicity; attractiveness to pests for ingestion; ease of killing and fixing without damage to the toxin; and the like. Other considerations include ease of formulation and handling, economics, storage stability, and the like.

Host organisms of particular interest include yeast, such as *Rhodotorula sp., Aureobasidium sp., Saccharomyces sp., and Sporobolomyces sp*.; phylloplane organisms such as *Pseudomonas sp., Erwinia sp*. and *Flavobacterium sp*.; or such other organisms as *Escherichia, LactoBacillus sp., Bacillus sp.,* and the like. Specific organisms include *Pseudomonas aeurginosa, Pseudomonas fluorescens, Saccharomyces cerevisiae, Bacillus thuringiensis, Escherichia coli, Bacillus subtilis,* and the like.

VIP genes can be introduced into micro-organisms that multiply on plants (epiphytes) to deliver VIP proteins to potential target pests. Epiphytes can be gram-positive or gram-negative bacteria for example.

Root colonizing bacteria, for example, can be isolated from the plant of interest by methods known in the art. Specifically, a *Bacillus cereus* strain which colonizes roots could be isolated from roots of a plant ( for example see J. Handelsman, S. Raffel, E. Mester, L. Wunderlich and C. Grau, Appl. Environ. Microbiol. 56:713-718, (1990)). VIP1 and/or VIP2 and/or VIP3 could be introduced into a root colonizing *Bacillus* cereus by standard methods known in the art.

Also, VIP3 or other VIPs of the invention can be introduced into the root colonizing *Bacillus* by means of electro-transformation. Specifically, VIPs can be cloned into a shuttle vector, for example, pHT3101 (D. Lereclus et al., FEMS Microbiol. Letts., 60:211-218 (1989)) as described in Example 10. The shuttle vector pHT3101 containing the coding sequence for the particular VIP can then be transformed into the root colonizing *Bacillus* by means of electroporation (D. Lereclus et al. 1989, FEMS Microbiol. Letts. 60:211-218).

Expression systems can be designed so that VIP proteins are secreted outside the cytoplasm of gram negative bacteria, *E. coli*, for example. Advantages of having VIP proteins secreted are (1) it avoids potential toxic effects of VIP proteins expressed within the cytoplasm and (2) it can increase the level of VIP protein expressed and (3) can aid in efficient purification of VIP protein.

VIP proteins can be made to be secreted in *E. coli*, for example, by fusing an appropriate *E. coli* signal peptide to the amino-terminal end of the VIP signal peptide or replacing the VIP signal peptide with the *E. coli* signal peptide. Signal peptides recognized by *E. coli* can be found in proteins already known to be secreted in *E. coli*, for example the OmpA protein (J. Ghrayeb, H. Kimura, M. Takahara, Y. Masui and M. Inouye, EMBO J., 3:2437-2442 (1984)). OmpA is a major protein of the *E. coli* outer membrane and thus its signal peptide is thought to be efficient in the translocation process. Also, the OmpA signal peptide does not need to be modified before processing as may be the case for other signal peptides, for example lipoprotein signal peptide
( G. Duffaud, P. March and M. Inouye, Methods in Enzymology, 153:492 (1987)).

Specifically, unique BamHI restriction sites can be introduced at the amino-terminal and carboxy-terminal ends of the VIP coding sequences using standard methods known in the art. These BamHI fragments can be cloned, in frame, into the vector pIN-III-ompA1, A2 or A3 (J. Ghrayeb, H. Kimura, M. Takahara, H. Hsiung, Y. Masui and M. Inouye, EMBO J., 3:2437-2442 (1984)) thereby creating ompA:VIP fusion gene which is secreted into the periplasmic space. The other restriction sites in the polylinker of pIN-III-ompA can be eliminated by standard methods known in the art so that the VIP amino-terminal amino acid coding sequence is directly after the ompA signal peptide cleavage site. Thus, the secreted VIP sequence in *E. coli* would then be identical to the native VIP sequence.

When the VIP native signal peptide is not needed for proper folding of the mature protein, such signal sequences can be removed and replaced with the ompA signal sequence. Unique BamHI restriction sites can be introduced at the amino-termini of the proprotein coding sequences directly after the signal peptide coding sequences of VIP and at the carboxy-termini of VIP coding sequence. These BamHI fragments can then be cloned into the pIN-III-ompA vectors as described above.

General methods for employing the strains of the invention in pesticide control or in engineering other organisms as pesticidal agents are known in the art. See, for example US Patent No. 5,039,523 and EP 0480762A2.

VIPs can be fermented in a bacterial host and the resulting bacteria processed and used as a microbial spray in the same manner that *Bacillus thuringiensis* strains have been used as insecticidal sprays. In the case of a VIP(s) which is secreted from *Bacillus,* the secretion signal is removed or mutated using procedures known in the art. Such mutations and/or deletions prevent secretion of the VIP protein(s) into the growth medium during the fermentation process. The VIPs are retained within the cell and the cells are then processed to yield the encapsulated VIPs. Any suitable microorganism can be used for this purpose. *Psuedomonas* has been used to express *Bacillus thuringiensis* endotoxins as encapsulated proteins and the resulting cells processed and sprayed as an insecticide. (H. Gaertner *et al*. 1993, In Advanced Engineered Pesticides, L. Kim ed.)

Various strains of *Bacillus thuringiensis* are used in this manner. Such Bt strains produce endotoxin protein(s) as well as VIPs. Alternatively, such strains can produce only VIPs. A sporulation deficient strain of *Bacillus subtilis* has been shown to produce high levels of the CryIIIA endotoxin from *Bacillus thuringiensis* (Agaisse, H. and Lereclus, D., "Expression in Bacillus subtilis of the Bacillus thuringiensis CryIIIA toxin gene is not dependent on a sporulation-specific sigma factor and is increased in a spoOA mutant", J. Bacteriol., 176:4734-4741 (1994)). A similar *spoOA* mutant can be prepared in *Bacillus thuringiensis* and used to produce encapsulated VIPs which are not secreted into the medium but are retained within the cell.

To have VIPs maintained within the *Bacillus* cell the signal peptide can be disarmed so that it no longer functions as a secretion signal.

Alternatively, the signal peptides of VIPs of the invention can be eliminated from the sequence thereby making them unrecognizable as secretion proteins in *Bacillus*. Specifically, a methionine start site can be engineered in front of the proprotein sequence in using methods known in the art.

VIP genes can be introduced into micro-organisms that mutiply on plants (epiphytes) to deliver VIP proteins to potential target pests. Epiphytes can be gram-positive or gram-negative bacteria for example.

The *Bacillus* strains of the invention or the microorganisms which have been genetically altered to contain the pesticidal gene and protein may be used for protecting agricultural crops and products from pests. In one aspect of the invention, whole, i.e., unlysed, cells of a toxin (pesticide)-producing organism are treated with reagents that prolong the activity of the toxin produced in the cell when the cell is applied to the environment of target pest(s).

Alternatively, the pesticides are produced by introducing a heterologous gene into a cellular host. Expression of the heterologous gene results, directly or indirectly, in the intracellular production and maintenance of the pesticide. These cells are then treated under conditions that prolong the activity of the toxin produced in the cell when the cell is applied to the environment of target pest(s). The resulting product retains the toxicity of the toxin. These naturally encapsulated pesticides may then be formulated in accordance with conventional techniques for application to the environment hosting a target pest, e.g., soil, water, and foliage of plants. See, for example EPA 0192319, and the references cited therein.

The active ingredients of the present invention are normally applied in the form of compositions and can be applied to the crop area or plant to be treated, simultaneously or in succession, with other compounds. These compounds can be both fertilizers or micronutrient donors or other preparations that influence plant growth. They can also be selective herbicides, insecticides, fungicides, bactericides, nematicides, mollusicides or mixtures of several of these preparations, if desired, together with further agriculturally acceptable carriers, surfactants or application-promoting adjuvants customarily employed in the art of formulation. Suitable carriers and adjuvants can be solid or liquid and correspond to the substances ordinarily employed in formulation technology, e.g. natural or regenerated mineral substances, solvents, dispersants, wetting agents, tackifiers, binders or fertilizers.

Preferred methods of applying an active ingredient of the present invention or an agrochemical composition of the present invention which contains at least one of the insect-specific proteins produced by the bacterial strains of the present invention are leaf application, seed coating and soil application. The number of applications and the rate of application depend on the intensity of infestation by the corresponding pest.

The present invention thus further provides an entomocidal composition comprising as an active ingrdient at least one of the novel insect-specific proteins according to the invention and/or a recombinant microorganism containing at least one DNA molecule comprising a nucleotide sequence encoding the novel insect-specific proteins in recombinant form, but especially a recombinant *Bacillus spp* strain, such as *Bacillus cereus* or *Bacillus thuringiensis,* containing at least one one DNA molecule comprising a nucleotide sequence encoding the novel insect-specific proteins in recombinant form, or a derivative or mutant thereof, together with an agricultural adjuvant such as a carrier, diluent, surfactant or application-promoting adjuvant. The composition may also contain a further biologically active compound. The said compound can be both a fertilizer or micronutrient donor or other preparations that influence plant growth. It can also be a selective herbicide, insecticide, fungicide, bactericide, nematicide, molluscide or mixtures of several of these preparations, if desired, together with further agriculturally acceptable carriers, surfactants or application-promoting adjuvants customarily employed in the art of formulation. Suitable carriers and adjuvants can be solid or liquid and correspond to the substances ordinarily employed in formulation technology, e.g. natural or regenerated mineral substances, solvents, dispersants, wetting agents, tackifiers, binders or fertilizers

The composition may comprise from 0.1 to 99% by weight of the active ingredient, from 1 to 99.9% by weight of a solid or liquid adjuvant, and from 0 to 25% by weight of a surfactant. The acitve ingredient comprising at least one of the novel insect-specific proteins according to the invention or a recombinant microorganism containing at least one DNA molecule comprising a nucleotide sequence encoding the novel insect-specific proteins in recombinant form, but especially a recombinant *Bacillus spp strain*, *such as Bacillus cereus or Bacillus thuringiensis* strain containing at least one DNA molecule comprising a nucleotide sequence encoding the novel insect-specific proteins in recombinant form, or a derivative or mutant thereof, or the composition containing the said acitve ingredient, may be administered to the plants or crops to be protected together with certain other insecticides or chemicals (1993 Crop Protection Chemicals Reference, Chemical and Pharmaceutical Press, Canada) without loss of potency. It is compatible with most other commonly used agricultural spray materials but should not be used in extremely alkaline spray solutions. It may be administered as a dust, a suspension, a wettable powder or in any other material form suitable for agricultural application.

The invention further provides methods for for controlling or inhibiting of insect pests by applying an active ingredient comprising at least one of the novel insect-specific proteins according to the invention or a recombinant microorganism containing at least one DNA molecule comprising a nucleotide sequence encoding the novel insect-specific proteins in recombinant form or a composition comprising the said active ingredient to (a) an environment in which the insect pest may occur, (b) a plant or plant part in order to protect said plant or plant part from damage caused by an insect pest, or (c) seed in order to protect a plant which develops from said seed from damage caused by an insect pest.

A preferred method of application in the area of plant protection is application to the foliage of the plants (foliar application), with the number of applications and the rate of application depending on the plant to be protected and the risk of infestation by the pest in question. However, the active ingredient may also penetrate the plants through the roots (systemic action) if the locus of the plants is impregnated with a liquid formulation or if the active ingredient is incorporated in solid form into the locus of the plants, for example into the soil, e.g. in granular form (soil application). In paddy rice crops, such granules may be applied in metered amounts to the flooded rice field.

The compositions according to the invention are also suitable for protecting plant propagating material, e.g. seed, such as fruit, tubers or grains, or plant cuttings, from insect pests. The propagation material can be treated with the formulation before planting: seed, for example, can be dressed before being sown. The acitve ingredient of the invention can also be applied to grains (coating), either by impregnating the grains with a liquid formulation or by coating them with a solid formulation. The formulation can also be applied to the planting site when the propagating material is being planted, for example to the seed furrow during sowing. The invention relates also to those methods of treating plant propagation material and to the plant propagation material thus treated.

The compositions according to the invention comprising as an active ingredient a recombinant microorganism containing at least one of the novel toxin genes in recombinant form, but especially a recombinant *Bacillus spp strain, such as Bacillus cereus or Bacillus thuringiensis* strain containing at least one DNA molecule comprising a nucleotide sequence encoding the novel insect-specific proteins in recombinant form, or a derivative or mutant thereof may be applied in any method known for treatment of seed or soil with bacterial strains. For example, see US Patent No.4,863,866. The strains are effective for biocontrol even if the microorganism is not living. Preferred is, however, the application of the living microorganism.

Target crops to be protected within the scope of the present invention comprise, e.g., the following species of plants:
cereals (wheat, barley, rye, oats, rice, sorghum and related crops), beet (sugar beet and fodder beet), forage grasses (orchardgrass, fescue, and the like), drupes, pomes and soft fruit (apples, pears, plums, peaches, almonds, cherries, strawberries, raspberries and blackberries), leguminous plants (beans, lentils, peas, soybeans), oil plants (rape, mustard, poppy, olives, sunflowers, coconuts, castor oil plants, cocoa beans, groundnuts), cucumber plants (cucumber, marrows, melons) fiber plants (cotton, flax, hemp, jute), citrus fruit (oranges, lemons, grapefruit, mandarins), vegetables (spinach, lettuce, asparagus, cabbages and other Brassicae, onions, tomatoes, potatoes, paprika), lauraceae (avocados, carrots, cinnamon, camphor), deciduous trees and conifers (e.g. linden-trees, yew-trees, oak-trees, alders, poplars, birch-trees, firs, larches, pines), or plants such as maize, tobacco, nuts, coffee, sugar cane, tea, vines, hops, bananas and natural rubber plants, as well as ornamentals (including composites).

A recombinant *Bacillus spp strain, such as Bacillus cereus or Bacillus thuringiensis* strain, containing at least one DNA molecule comprising a nucleotide sequence encoding the novel insect-specific proteins in recombinant form is normally applied in the form of entomocidal compositions and can be applied to the crop area or plant to be treated, simultaneously or in succession, with further biologically active compounds. These compounds may be both fertilizers or micronutrient donors or other preparations that influence plant growth. They may also be selective herbicides, insecticides, fungicides, bactericides, nematicides, molluscicides or mixtures of several of these preparations, if desired together with further carriers, surfactants or application-promoting adjuvants customarily employed in the art of formulation.

The active ingredient according to the invention may be used in unmodified form or together with any suitable agriculturally acceptable carrier. Such carriers are adjuvants conventionally employed in the art of agricultural formulation, and are therefore formulated in known manner to emulsifiable concentrates, coatable pastes, directly sprayable or dilutable solutions, dilute emulsions, wettable powders, soluble powders, dusts, granulates, and also encapsulations, for example, in polymer substances. Like the nature of the compositions, the methods of application, such as spraying, atomizing, dusting, scattering or pouring, are chosen in accordance with the intended objective and the prevailing circumstances. Advantageous rates of application are normally from about 50 g to about 5 kg of active ingredient (a.i.) per hectare ("ha", approximately 2.471 acres), preferably from about 100 g to about 2kg a.i./ha. Important rates of application are about 200 g to about 1 kg a.i./ha and 200g to 500g a.i./ha.
For seed dressing advantageous application rates are 0.5 g to 1000 g a.i.per 100 kg seed, preferably 3 g to 100 g a.i. per 100 kg seed or 10 g to 50 g a.i.per 100 kg seed.

Suitable carriers and adjuvants can be solid or liquid and correspond to the substances ordinarily employed in formulation technology, e.g. natural or regenerated mineral substances, solvents, dispersants, wetting agents, tackifiers, binders or fertilizers. The formulations, i.e. the entomocidal compositions, preparations or mixtures containing the recombinant *Bacillus spp strain, such as Bacillus cereus or Bacillus thuringiensis* strain containing at least one DNA molecule comprising a nucleotide sequence encoding the novel insect-specific proteins in recombinant form as an active ingredient or combinations thereof with other active ingredients, and, where appropriate, a solid or liquid adjuvant, are prepared in known manner, e.g., by homogeneously mixing and/or grinding the active ingredients with extenders, e.g., solvents, solid carriers, and in some cases surface-active compounds (surfactants).

Suitable solvents are: aromatic hydrocarbons, preferably the fractions containing 8 to 12 carbon atoms, e.g. xylene mixtures or substituted naphthalenes, phthalates such as dibutyl phthalate or dioctyl phthalate, aliphatic hydrocarbons such as cyclohexane or paraffins, alcohols and glycols and their ethers and esters, such as ethanol, ethylene glycol monomethyl or monoethyl ether, ketones such as cyclohexanone, strongly polar solvents such as N-methyl-2-pyrrolidone, dimethylsulfoxide or dimethylformamide, as well as vegetable oils or epoxidised vegetable oils such as epoxidised coconut oil or soybean oil; or water.

The solid carriers used, e.g., for dusts and dispersible powders, are normally natural mineral fillers such as calcite, talcum, kaolin, montmorillonite or attapulgite. In order to improve the physical properties it is also possible to add highly dispersed silicic acid or highly dispersed absorbent polymers. Suitable granulated adsorptive carriers are porous types, for example pumice, broken brick, sepiolite or bentonite; and suitable nonsorbent carriers are materials such as calcite or sand. In addition, a great number of pregranulated materials of inorganic or organic nature can be used, e.g. especially dolomite or pulverized plant residues.

Depending on the nature of the active ingredients to be formulated, suitable surface-active compounds are non-ionic, cationic and/or anionic surfactants having good emulsifying, dispersing and wetting properties. The term "surfactants" will also be understood as comprising mixtures of surfactants. Suitable anionic surfactants can be both water-soluble soaps and
water-soluble synthetic surface-active compounds. Suitable soaps are the alkali metal salts, alkaline earth metal salts or unsubstituted or substituted ammonium salts of higher fatty acids (C₁₀ -C₂₂), e.g. the sodium or potassium salts of oleic or stearic acid, or of natural fatty acid mixtures which can be obtained, e.g. from coconut oil or tallow oil. Further suitable surfactants are also the fatty acid methyltaurin salts as well as modified and unmodified phospholipids.

More frequently, however, so-called synthetic surfactants are used, especially fatty sulfonates, fatty sulfates, sulfonated benzimidazole derivatives or alkylarylsulfonates. The fatty sulfonates or sulfates are usually in the forms of alkali metal salts, alkaline earth metal salts or unsubstituted or substituted ammonium salts and generally contain a C₈ -C₂₂ alkyl radical which also includes the alkyl moiety of acyl radicals, e.g. the sodium or calcium salt of lignosulfonic acid, of dodecylsulfate, or of a mixture of fatty alcohol sulfates obtained from natural fatty acids. These compounds also comprise the salts of sulfuric acid esters and sulfonic acids of fatty alcohol/ethylene oxide adducts. The sulfonated benzimidazole derivatives preferably contain 2 sulfonic acid groups and one fatty acid radical containing about 8 to 22 carbon atoms. Examples of alkylarylsulfonates are the sodium, calcium or triethanolamine salts of dodecylbenzenesulfonic acid, dibutylnaphthalenesulfonic acid, or of a naphthalenesulfonic acid/formaldehyde condensation product. Also suitable are corresponding phosphates, e.g. salts of the phosphoric acid ester of an adduct of p-nonylphenol with 4 to 14 moles of ethylene oxide.
Non-ionic surfactant are preferably polyglycol ether derivatives of aliphatic or cycloaliphatic alcohols, or saturated or unsaturated fatty acids and alkylphenols, said derivatives containing 3 to 30 glycol ether groups and 8 to 20 carbon atoms in the (aliphatic) hydrocarbon moiety and 6 to 18 carbon atoms in the alkyl moiety of the alkylphenols.

Further suitable non-ionic surfactants are the water-soluble adducts of polyethylene oxide with polypropylene glycol, ethylenediaminopolypropylene glycol and alkylpolypropylene glycol containing 1 to 10 carbon atoms in the alkyl chain, which adducts contain 20 to 250 ethylene glycol ether groups and 10 to 100 propylene glycol ether groups. These compounds usually contain 1 to 5 ethylene glycol units per propylene glycol unit. Representative examples of non-ionic surfactants are nonylphenolpolyethoxyethanols, castor oil polyglycol ethers, polypropylene/polyethylene oxide adducts, tributylphenoxypolyethoxyethanol, polyethylene glycol and octylphenoxypolyethoxyethanol. Fatty acid esters of polyoxyethylene sorbitan, such as polyoxyethylene sorbitan trioleate, are also suitable non-ionic surfactants.

Cationic surfactants are preferably quaternary ammonium salts which contain, as N-substituent, at least one C₈ -C₂₂ alkyl radical and, as further substituents, lower unsubstituted or halogenated alkyl, benzyl or hydroxyl-lower alkyl radicals. The salts are preferably in the form of halides, methylsulfates or ethylsulfates, e.g., stearyltrimethylammonium chloride or benzyldi-(2-chloroethyl)ethylammonium bromide.

The surfactants customarily employed in the art of formulation are described, e.g., in "McCutcheon's Detergents and Emulsifiers Annual", MC Publishing Corp. Ridgewood, N.J., 1979; Dr. Helmut Stache, "Tensid Taschenbuch" (Handbook of Surfactants), Carl Hanser Verlag, MunichNienna.

Another particularly preferred characteristic of an entomocidal composition of the present invention is the persistence of the active ingredient when applied to plants and soil. Possible causes for loss of activity include inactivation by ultra-violet light, heat, leaf exudates and pH. For example, at high pH, particularly in the presence of reductant, δ-endotoxin crystals are solubilized and thus become more accessible to proteolytic inactivation. High leaf pH might also be important, particularly where the leaf surface can be in the range of pH 8-10. Formulation of an entomocidal composition of the present invention can address these problems by either including additives to help prevent loss of the active ingredient or encapsulating the material in such a way that the active ingredient is protected from inactivation. Encapsulation can be accomplished chemically (McGuire and Shasha, J Econ Entomol 85: 1425-1433, 1992) or biologically (Barnes and Cummings, 1986; EP-A 0 192 319). Chemical encapsulation involves a process in which the active ingredient is coated with a polymer while biological encapsulation involves the expression of the δ-endotoxin genes in a microbe. For biological encapsulation, the intact microbe containing at least one DNA molecule comprising a nucleotide sequence encoding the novel insect-specific proteins in recombinant form is used as the active ingredient in the formulation. The addition of UV protectants might effectively reduce irradiation damage. Inactivation due to heat could also be controlled by including an appropriate additive.

Preferred within the present application are formulations comprising living microorganisms as active ingredient either in form of the vegetative cell or more preferable in form of spores, if available. Suitable formulations may consist, for example, of polymer gels which are crosslinked with polyvalent cations and comprise these microorganisms. This is described, for example, by D.R. Fravel et al. in Phytopathology, Vol. 75, No. 7, 774-777, 1985 for alginate as the polymer material. It is also known from this publication that carrier materials can be co-used. These formulations are as a rule prepared by mixing solutions of naturally occurring or synthetic gel-forming polymers, for example alginates, and aqueous salt solutions of polyvalent metal ions such that individual droplets form, it being possible for the microorganisms to be suspended in one of the two or in both reaction solutions. Gel formation starts with the mixing in drop form. Subsequent drying of these gel particles is possible. This process is called ionotropic gelling. Depending on the degree of drying, compact and hard particles of polymers which are structurally crosslinked via polyvalent cations and comprise the microorganisms and a carrier present predominantly uniformly distributed are formed. The size of the particles can be up to 5 mm.

Compositions based on partly crosslinked polysaccharides which, in addition to a microorganism, for example, can also comprise finely divided silicic acid as the carrier material, crosslinking taking place, for example, via Ca⁺⁺ ions, are described in EP-A1-0 097 571. The compositions have a water activity of not more than 0.3. W.J. Cornick et al. describe in a review article [New Directions in Biological Control:
Alternatives for Suppressing Agricultural Pests and Diseases, pages 345-372, Alan R. Liss, Inc. (1990)] various formulation systems, granules with vermiculite as the carrier and compact alginate beads prepared by the ionotropic gelling process being mentioned. Such compositions are also disclosed by D.R.Fravel in Pesticide Formulations and Application Systems: 11 th Volume, ASTM STP 1112 American Society for Testing and Materials, Philadelphia, 1992, pages 173 to 179 and can be used to formulate the recombinant microorganisms according to the invention.

The entomocidal compositions of the invention usually contain from about 0.1 to about 99%, preferably about 0.1 to about 95%, and most preferably from about 3 to about 90% of the active ingredient, from about 1 to about 99.9%, preferably from about 1 to about 99%, and most preferably from about 5 to about 95% of a solid or liquid adjuvant, and from about 0 to about 25%, preferably about 0.1 to about 25%, and most preferably from about 0.1 to about 20% of a surfactant.

In a preferred embodiment of the invention the entomocidal compositions usually contain 0.1 to 99%, preferably 0.1 to 95%, of a recombinant *Bacillus spp strain,* such as *Bacillus cereus or Bacillus thuringiensis* strain containing at least one DNA molecule comprising a nucleotide sequence encoding the novel insect-specific proteins in recombinant form, or combination thereof with other active ingredients, 1 to 99.9% of a solid or liquid adjuvant, and 0 to 25%, preferably 0.1 to 20%, of a surfactant.

Whereas commercial products are preferably formulated as concentrates, the end user will normally employ dilute formulations of substantially lower concentration. The entomocidal compositions may also contain further ingredients, such as stabilizers, antifoams, viscosity regulators, binders, tackifiers as well as fertilizers or other active ingredients in order to obtain special effects.

The invention now being generally described, the same will be better understood by reference to the following detailed examples that are provided for the purpose of illustration and are not to be considered limiting of the invention unless so specified.

A standard nomenclature has been developed based on the sequence identity of the proteins encompassed by the present invention. The gene and protein names for the detailed examples which follow and their relationship to the names used in the parent application [US application serial no 314594/08] are shown below.

| *Gene* / *Protein Name under Standard Nomenclature* | *Gene* / *Protein Name in Parent* | *Description of Protein* |
|---|---|---|
| VIP3A(a) | -- | VIP from strain AB88 as disclosed in SEQ ID NO:1 of the present application |
| VIP3A(b) | -- | VIP from strain AB424 as disclosed in SEQ ID NO:4 of the present application |

### EXPERIMENTAL

### Formulation Examples

The active ingredient used in the following formulation examples are *Bacillus cereus* strain AB78 having Accession No. NRRL B-21058; *Bacillus thuringiensis* strains having Accession Nos. NRRL B-21060, NRRL B-21224, NRRL B-21225, NRRL B-21226, NRRL B-21227, and NRRL B-21439; and *Bacillus spp* strains having Accession Nos NRRL B-21228, NRRL B-21229, and NRRL B-21230. All the mentioned strains are natural isolates comprising the insect-specific proteins according to the invention.
Alternatively, the isolated insect-specific proteins are used as the active ingredient alone or in combination with the above-mentioned *Bacillus* strains.

| A1. Wettable powders | | | |
|---|---|---|---|
| | a) | b) | c) |
| *Bacillus thuringiensis* spores | 25% | 50% | 75% |
| sodium lignosufonate | 5% | 5% | -- |
| sodium laurylsulfate | 3% | -- | 5% |
| sodium diisobutylnaphthalenesulfonate | -- | 6% | 10% |
| octylphenol polyethylene glycol ether (7-8 moles of ethylene oxid) | -- | 2% | -- |
| highly dispersed silicid acid | 5% | 10% | 10% |
| kaolin | 62% | 27% | -- |

The spores are thoroughly mixed with the adjuvants and the mixture is thoroughly ground in a suitable mill, affording wettable powders which can be diluted with water to give suspensions of the desired concentrations.

| A2. Emulsifiable concentrate | |
|---|---|
| *Bacillus thuringiensis* spores | 10% |
| octylphenol polyethylene glycol ether (4-5 moles ethylene oxide) | 3% |
| clacium dodecylbenzensulfonate | 3% |
| castor oil polyglycol ether (36 moles of ethylene oxide) | 4% |
| cyclohexanone | 30% |
| xylene mixture | 50% |

Emulsions of any required concentration can be obtained from this concentrate by dilution with water.

| A3. Dusts | | |
|---|---|---|
| | a) | b) |
| *Bacillus thuringiensis* spores | 5% | 8% |
| talcum | 95% | -- |
| kaolin | -- | 92% |

Ready for use dusts are obtained by mixing the active ingredient with the carriers and grinding the mixture in a suitable mill.

| A4. Extruder Granulate | |
|---|---|
| *Bacillus thuringiensis* spores | 10% |
| sodium lignosulfonate | 2% |
| carboxymethylcellulose | 1% |
| kaolin | 87% |

The active ingredient or combination is mixed and ground with the adjuvants and the mixture is subsequently moistened with water. The mixture is extruded, granulated and the dried in a stream of air.

| A5. Coated Granule | |
|---|---|
| *Bacillus thuringiensis* spores | 3% |
| polyethylene glycol (mol wt 200) | 3% |
| kaolin | 94% |

The active ingredient or combination is uniformly applied in a mixer to the kaolin moistened with polyethylene glycol. Non-dusty coated granulates are obtained in this manner.

| A6. Suspension Concentrate | |
|---|---|
| *Bacillus thuringiensis* spores | 40% |
| ethylene glycol | 10% |
| nonylphenol polyethylene glycol ether (15 moles of ethylene oxide) | 6% |
| sodium lignosulfonate | 10% |
| carboxymethylcellulose | 1% |
| 37% aqueous formaldehyde solution | 0.2% |
| silicone oil in the form of a 75% aqueous solution | 0.8% |
| water | 32% |

The active ingredient or combination is intimately mixed with the adjuvants giving a suspension concentrate from which suspensions of any desired concentration can be obtained by dilution with water.

### EXAMPLE 1. BACTERIAL CULTURE

A subculture of Bc strain AB78 was used to inoculate the following medium, known as TB broth:

| | |
|---|---|
| Tryptone | 12 g/l |
| Yeast Extract | 24 g/l |
| Glycerol | 4 ml/l |
| KH₂PO₄ | 2.1 g/l |
| K₂HPO₄ | 14.7 g/l |
| pH 7.4 | |

The potassium phosphate was added to the autoclaved broth after cooling. Flasks were incubated at 30°C on a rotary shaker at 250 rpm for 24 h-36 h, which represents an early to mid-log growth phase.

The above procedure can be readily scaled up to large fermentors by procedures well known in the art.

During vegetative growth, usually 24-36 h. after starting the culture, which represents an early to mid-log growth phase, AB78 bacteria were centrifuged from the culture supernatant. The culture supernatant containing the active protein was used in bioassays.

### EXAMPLE 2. INSECT BIOASSAYS

*B. cereus* strain AB78 was tested against various insects as described below.

Western, Northern and Southern corn rootworm, *Diabrotica virgifera virgifera, D. longcornis barberi* and *D. undecempunctata howardi*, respectively: dilutions were made of AB78 culture supernatant grown 24-36 h., mixed with molten artificial diet (Marrone et al. (1985) J. of Economic Entomology 78:290-293) and allowed to solidify. Solidified diet was cut and placed in dishes. Neonate larvae were placed on the diet and held at 30 C. Mortality was recorded after 6 days.

*E. coli* clone bioassay: *E. coli cells* were grown overnight in broth containing 100 µg/ml ampicillin at 37°C. Ten ml culture was sonicated 3X for 20 sec each. 500 µl of sonicated culture was added to molten western corn rootworm diet.

Colorado potato beetle, Leptinotarsa decemlineata: dilutions in Triton X-100 (to give final concentration of 0.1% TX-100) were made of AB78 culture supernatant grown 24-36 h. Five cm² potato leaf pieces were dipped into these dilutions, air dried, and placed on moistened filter paper in plastic dishes. Neonate larvae were placed on the leaf pieces and held at 30°C. Mortality was recorded after 3-5 days.

Yellow mealworm, *Tenebrio molitor*. dilutions were made of AB78 culture supernatant grown 24-36 h., mixed with molten artificial diet (Bioserv #F9240) and allowed to solidify. Solidified diet was cut and placed in plastic dishes. Neonate larvae were placed on the diet and held at 30°C. Mortality was recorded after 6-8 days.

European corn borer, black cutworm, tobacco budworm, tobacco hornworm and beet armyworm; *Ostrinia nubilalis, Agrotis ipsilon, Heliothis virescens, Manduca sexta* and *Spodoptera exigua,* respectively: dilutions, in TX-100 (to give final concentration of 0.1% TX-100), were made of AB78 culture supernatant grown 24-36 hrs. 100 µl was pipetted onto the surface of 18 cm² of solidified artificial diet (Bioserv #F9240) and allowed to air dry. Neonate larvae were then placed onto the surface of the diet and held at 30°C. Mortality was recorded after 3-6 days.
Northern house mosquito, *Culex pipiens*:-dilutions were made of AB78 culture supernatant grown 24-36 h. 100 µl was pipetted into 10 ml water in a 30 ml plastic cup. Third instar larvae were added to the water and held at room temperature. Mortality was recorded after 24-48 hours. The spectrum of entomocidal activity of AB78 is given in Table 14.

**TABLE 14**

| **Activity of AB78 culture supernatant against various insect species** | | |
|---|---|---|
| Insect species tested to date | Order | Activity |
| Western corn rootworm (*Diabrotica virgifera virgifera*) | Col | +++ |
| Northern corn rootworm (*Diabrotica longicornis barberi*) | Col | +++ |
| Southern corn rootworm (*Diabrotica undecimpunctata howardi)* | Col | - |
| Colorado potato beetle (*Leptinotarsa decemlineata*) | Col | - |
| Yellow mealworm (*Tenebrio molitor*) | Col | - |
| European corn borer (*Ostrinia nubilalis*) | Lep | - |
| Tobacco budworm (Heliothis virescens) | Lep | - |
| Tobacco hornworm (*Manduca sexta*) | Lep | - |
| Beet armyworm (Spodoptera exigua) | Lep | - |
| Black cutworm (Agrotis ipsilon) | Lep | - |
| Northern house mosquito (*Culex pipiens*) | Dip | - |

The newly discovered *B. cereus* strain AB78 showed a significantly different spectrum of insecticidal activity as compared to known coleopteran active δ-endotoxins from Bt. In particular, AB78 showed more selective activity against beetles than known coleopteran-active Bt strains in that it was specifically active against *Diabrotica* spp. More specifically, it was most active against *D. virgifera virgifera* and *D. longicornis barberi* but not *D. undecimpunctata howardi*.

A number of *Bacillus* strains were bioassayed for activity during vegetative growth (Table 15) against western corn rootworm. The results demonstrate that AB78 is unique in that activity against western corn rootworm is not a general phenomenon.

**TABLE 15**

| **Activity of culture supernatants from various *Bacillus spp.* against western corn rootworm** | |
|---|---|
| *Bacillus* strain | Percent WCRW mortality |
| *B. cereus* AB78 (Bat.1) | 100 |
| *B. cereus* AB78 (Bat.2) | 100 |
| *B. cereus* (Carolina Bio.) | 12 |
| B. cereus ATCC 11950 | 12 |
| *B. cereus* ATCC 14579 | 8 |
| *B. mycoides* (Carolina Bio.) | 30 |
| *B. popilliae* | 28 |
| *B. thuringiensis* HD135 | 41 |
| *B. thuringiensis* HD191 | 9 |
| *B. thuringiensis* GC91 | 4 |
| *B. thuringiensis isrealensis* | 24 |
| Water Control | 4 |

Specific activity of AB78 against western corn rootworm is provided in Table 16.

**TABLE 16**

| **Activity of AB78 culture supernatant against neonate western corn rootworm** | |
|---|---|
| Culture supernatant concentration (µl/ml) | Percent WCRW mortality |
| 100 | 100 |
| 25 | 87 |
| 10 | 80 |
| 5 | 40 |
| 2.5 | 20 |
| 1 | 6 |
| 0 | 0 |

The LC₅₀ was calculated to be 6.2 µl of culture supernatant per ml of western corn rootworm diet.

The cell pellet was also bioassayed and had no activity against WCRW. Thus, the presence of activity only in the supernatant indicates that this VIP is an exotoxin.

### EXAMPLE 3. COSMID CLONING OF TOTAL DNA FROM B. CEREUS STRAIN AB78

The VIP1A(a) gene was cloned from total DNA prepared from strain AB78 as follows:
Isolation of AB78 DNA was as follows:
1. Grow bacteria in 10 ml L-broth overnight. (Use 50 ml sterile centrifuge tube)
2. Add 25 ml of fresh L-broth and ampicillin (30 µg/ml).
3. Grow cells 2-6 h. at 30°C with shaking.
4. Spin cells in a 50 ml polypropylene orange cap tube in IEC benchtop clinical centrifuge at 3/4 speed.
5. Resuspend cell pellet in 10 ml TES (TES = 50 mM TRIS pH 8.0, 100 mM EDTA, 15 mM NaCl).
6. Add 30 mg lysozyme and incubate 2 hrs at 37°C.
7. Add 200 µl 20% SDS and 400 µl Proteinase K stock (20 mg/ml). Incubate at 37°C.
8. Add 200 µl fresh Proteinase K. Incubate 1 hr. at 55°C. Add 5 ml TES to make 15 ml final volume.
9. Phenol extract twice (10 ml phenol, spin at room temperature at 3/4 speed in an IEC benchtop clinical centrifuge). Transfer supernatant (upper phase) to a clean tube using a wide bore pipette.
10. Extract once with 1:1 vol. phenol:chloroform/isoamyl alcohol (24:1 ratio).
11. Precipitate DNA with an equal volume of cold isopropanol; Centrifuge to pellet DNA.
12. Resuspend pellet in 5 ml TE.
13. Precipitate DNA with 0.5 ml 3M NaOAc pH 5.2 and 11 ml 95% ethanol. Place at -20°C for 2 h.
14. "Hook" DNA from tube with a plastic loop, transfer to a microfuge tube, spin, pipette off excess ethanol, dry in vacuo.
15. Resuspend in 0.5 ml TE. Incubate 90 min. at 65°C to help get DNA back into solution.
16. Determine concentration using standard procedures.

### Cosmid Cloning of AB78

All procedures, unless indicated otherwise, were performed according to Stratagene Protocol, Superoos 1 Instruction Manual, Cat. No. 251301.

Generally, the steps were as follows:
A. Sau 3A partial digestion of the AB78 DNA.
B. Preparation of vector DNA
C. Ligation and packaging of DNA
D. Tittering the cosmid library
   1. Start a culture of HB101 cells by placing 50 ml of an overnight culture in 5 mls of TB with 0.2% maltose. Incubate 3.5 hrs. at 37°C.
   2. Spin out cells and resuspend in 0.5 ml 10 mM MgSO₄.
   3. Add together:
      100 I cells
      100 l diluted packaging mixture
      100 l 10 mM MgSO₄
      30 l TB
   4. Adsorb at room temperature for 30 minutes with no shaking.
   5. Add 1 ml TB and mix gently. Incubate 30 minutes at 37°C.
   6. Plate 200 l onto L-amp plates. Incubate at 37°C overnight.

At least 400 cosmid clones were selected at random and screened for activity against western corn rootworm as described in Example 2. DNA from 5 active clones and 5 non-active clones were used in Southern hybridizations. Results demonstrated that hybridization using the above described oligonucleotide probe correlated with western corn rootworm activity (Table 18).

Cosmid clones P3-12 and P5-4 have been deposited with the Agricultural Research Service Patent Culture Collection (NRRL) and given Accession Nos. NRRL B-21061 and NRRL B-21059 respectively.

**TABLE 18**

| **Activity of AB78 cosmid clones against western corn rootworm.** | |
|---|---|
| Clone | Mean percent mortality (N=4) |
| Clones which hybridize with probe | |
| P 1-73 | 47 |
| P1-83 | 64 |
| P2-2 | 69 |
| P3-12 | 85 |
| P5-4 | 97 |
| | |

| Clones which do not hybridize with probe | |
|---|---|
| P1-2 | 5 |
| P3-8 | 4 |
| P3-9 | 12 |
| P3-18 | 0 |
| P4-6 | 9 |
| | |

### EXAMPLE 4. PURIFICATION OF VIPS FROM STRAIN AB88:

Bacterial liquid culture was grown overnight [for 12h] at 30°C in TB media. Cells were centrifuged at 5000 x g for 20 minutes and the supernatant retained. Proteins present in the supernatant were precipitated with ammonium sulfate (70% saturation), centrifuged [at 5000 x g for 15 minutes] and the pellet retained. The pellet was resuspended in the original volume of 20 mM Tris pH 7.5 and dialyzed overnight against the same buffer at 4°C. AB88 dialysate was more turbid than comparable material from AB78. The dialysate was titrated to pH 4.5 using 20 mM sodium citrate (pH 2.5) and, after 30 min incubation at room temperature, the solution was centrifuged at 3000 x g for 10 min. The protein pellet was redissolved in 20 mM Bis-Tris-Propane pH 9.0.

AB88 proteins have been separated by several different methods following clarification including isoelectric focusing (Rotofor, BioRad, Hercules, CA), precipitation at pH 4.5, ion-exchange chromotography, size exclusion chromatography and ultrafiltration.
Proteins were separated on a Poros HQ/N anion exchange column (PerSeptive Biosystems, Cambridge, MA) using a linear gradient from 0 to 500 mM NaCl in 20 mM Bis-Tris-Propane pH 9.0 at a flow rate of 4 ml/min. The insecticidal protein eluted at 250 mM NaCl.

European corn borer (ECB)-active protein remained in the pellet obtained by pH 4.5 precipitation of dialysate. When preparative IEF was done on the dialysate using pH 3-10 ampholytes, ECB insecticidal activity was found in all fractions with pH of 7 or greater. SDS-PAGE analysis of these fractions showed protein bands of MW ∼60 kDa and ∼80 kDa. The 60 kDa and 80 kDa bands were separated by anion exchange HPLC on a Poros-Q column (PerSeptive Biosystems, Cambridge, MA). N-terminal sequence was obtained from two fractions containing proteins of slightly differing MW, but both of approximately 60 kDa in size. The sequences obtained were similar to each other and to some δ-endotoxins.

When the ECB-active pH 4.5 pellet was further separated by anion exchange on a Poros-Q column, activity was found only in fractions containing a major band of ∼60 kDa.

Black cutworm-active protein also remained in the pellet when AB88 dialysate was brought down to pH 4.5. In preparative IEF using pH 3-10 ampholytes, activity was not found in the ECB-active IEF fractions; instead, it was highest in a fraction of pH 4.5-5.0. Its major components have molecular weights of ∼35 and -80 kDa.

The pH 4.5 pellet was separated by anion exchange HPLC to yield fractions containing only the 35 kDa material and fractions containing both 35 kDa and 80 kDa bands.

### EXAMPLE 5. CHARACTERIZATION OF AB88 VIP.

Fractions containing the various lepidopteran active vegetative proteins were generated as described in Example 4. Fractions with insecticidal acitivity were separated in 8 to 16% SDS-polyacrylamide gels and transferred to PVDF membranes [LeGendre et al, (1989) in: A Practical Guide to Protein and Peptide Purification for Microsequencing, ed Matsudaria PT (Academic Press Inc, New Yorkl]. Biological analysis of fractions demonstrated that different VIPs were responsible for the different lepidopteran species activity.

The *Agrotis ipsilon* activity is due to an 80 kDa and/or a 35 kDa protein, either delivered singly or in combination. These proteins are not related to any δ-endotoxins from Bt as evidenced by the lack of sequence homology of known Bt δ-endotoxin sequences. The vip3A(a) insecticidal protein from strain AB88 is present mostly (at least 75% of the total) in supernatants of AB88 cultures.

Also, these proteins are not found in the AB88 δ-endotoxin crystal. N-terminal sequences of the major δ-endotoxin proteins were compared with the N-terminal sequences of the 80 kDa and 35 kDa VIP and revealed no sequence homology. The N-terminal sequence of the vip3A(a) insecticidal protein posses a number of positively charged residues (from Asn2 to Asn7) followed by a hydrophobic core region (from Thr8 to Ile34). Unlike most of the known secretion proteins, the vip3A(a) insecticidal protein from strain AB88 is not N-terminally processed during export.

The *Ostrinia nubilalis* activity is due to a 60 kDa VIP and the *Spodoptera frugiperda* activity is due to a VIP of unknown size.

*Bacillus thuringiensis* strain AB88 has been deposited in the Agricultural Research Service, Patent Culture Collection (NRRL), Northern Regional Research Center, 1815 North University Street, Peoria, Illinois 61604, USA and given the Accession No. NRRL B-21225.

### EXAMPLE 6. ISOLATION AND BIOLOGICAL ACTIVITY OF B. THURINGIENSIS AB424

A *B. thuringiensis* strain, designated AB424, was isolated from a moss covered pine cone sample by standard methods known in the art. A subculture of AB424 was grown and prepared for bioassay as described in Example 1.

Biological activity was evaluated as described in Example 2. The results are as follows:

| Insect species tested | Percent mortality |
|---|---|
| *Ostrinia nubilalis* | 100 |
| *Agrotis ipsilon* | 100 |
| *Diabrotica virgifera virgifera* | 0 |

Strain AB424 has been deposited in the Agricultural Research Service, Patent Culture Collection (NRRL), Northern Regional Research Center, 1815 North University Street, Peoria, Illinois 61604, USA, and given Accession No. NRRL B-21439.

### EXAMPLE 7. CLONING OF THE VIP3A(a) and VIP3A(b) GENES WHICH ENCODE PROTEINS ACTIVE AGAINST BLACK CUTWORM.

Total DNA from isolates AB88 and AB424 was isolated [Ausubel et al (1988), in: Current Protocols in Molecular Biology (John Wiley & Sons, NY)] and digested with the restriction enzymes *Xba*l [library of 4.0 to 5.0 Kb size-fractionated *XbaI* fragments of *B thuringiensis* AB88 DNA] and *EcoR*I [library of 4.5 to 6.0 Kb size-fractionated *EcoRI* fragments *B thuringiensis* AB424 DNA] respectively, ligated into pBluescript vector previously linearized with the same enzymes and dephosphorylated, and transformed into *E. coli* DH5α strain. Recombinant clones were blotted onto nitrocellulose filters which were subsequently probed with a ³² P labeled 33-bases long oligonucleotide corresponding to the 11-N terminal amino acids of the 80 kDa protein active against *Agrotis ipsilon* (black cutworm). Hybridization was carried out at 42°C in 2 x SSC/0.1% SDS (1 x SSC = 0.15 m NaCl/0.015 M sodium citrate, pH 7.4) for 5 min and twice at 50°C in 1 x SSC/0.1 SDS for 10 min. Four out of 400 recombinant clones were positive. Insect bioassays of the positive recombinants exhibited toxicity to black cutworm larvae comparable to that of AB88 or AB424 supernantants.

Plasmid pCIB7104 contains a 4.5 Kb *XbaI* fragment of AB88 DNA. Subclones were constructed to define the coding region of the insecticidal protein.

*E coli* pCIB7105 was constructed by cloning the 3.5 Kb *XbaI-AccI* fragment of pCIB7104 into pBluescript.

Plasmid pCIB7106 contained a 5.0 Kb *EcoRI* fragment of AB424 DNA. This fragment was further digested with *HincII* to render a 2.8 kb *EcoRI-HincII* insert (pCIB7107), which still encoded a functional insecticidal protein.

The nucleotide sequence of pCIB7104, a positive recombinant clone from AB88, and of pCIB7107, a positive recombinant clone from AB424, was determined by the dideoxy termination method of Sanger et al., Proc. Natl. Acad. Sci. USA, 74: 5463-5467 (1977), using PRISM Ready Reaction Dye Deoxy Terminator Cycle Sequencing Kits and PRISM Sequenase® Terminator Double-Stranded DNA Sequencing Kit and analysed on an ABI 373 automatic sequencer.

The clone pCIB7104 contains the VIP3A(a) gene whose coding region is disclosed in SEQ ID NO:1 and the encoded protein sequence is disclosed in SEQ ID NO:2. A synthetic version of the coding region designed to be highly expressed in maize is given in SEQ ID NO:3. A fusion between synthetic and native sequences is disclosed in SEQ ID NO: 6. Any number of synthetic genes can be designed based on the amino acid sequence given in SEQ ID NO:2.

The clone pCIB7107 contains the VIP3A(b) gene whose coding region is disclosed in SEQ ID NO:4 and the encoded protein is disclosed in SEQ ID NO:5. Both pCIB7104 and pCIB7107 have been deposited with the Agricultural Research Service Patent Culture Collection (NRRL) and given Accession Nos. NRRL B-21422 and B-21423, respectively.

The VIP3A(a) gene contains an open reading frame (ORF) that extends form nucleotide 732 to 3105. This ORF encodes a peptide of 791 amino acids corresponding to a molecular mass of 88,500 daltons. A Shine-Dalgarno (SD) sequence is located 6 bases before the first methionine and its sequence identifies a strong SD for *Bacillus*.

The VIP3A(b) gene is 98% identical to VIP3A(a).

When blost of total DNA isolated from AB88 *B thuringiensis* cells were probed with a 33.base fragment that spans the N-terminal region of the VIP3A-insecticidal protein, single bands could be observed in different restriction digests. This result was confirmed by using larger probes spanning the coding region of the gene. A search of the GenBank data base revealed no homology to known proteins.

### EXAMPLE 8. EXPRESSION OF THE VIP3A INSECTICIDAL PROTEINS

The time course for expression of the VIP3A(a) insecticidal protein was analyzed by western blot. Samples from *Bacillus thuringiensis* Ab88 clutures were taken throughout ist growth curve and sporulation. The VIP3A(a) insecticidal protein can be detected in the supernatants of AB88 cultures during logarithmic phase, as early as 15 h after initiating the culture. It reached its maximum level during early stages of stationary phase and remained at high levels during and after sporulation. Similar results were obtained when supernatants of AB424 *Bacillus cereus* cultures were used. The levels of VIP3A(a) insecticidal protein reflected the expression of the VIP3A(a) gene as determined by Northern blot. The initiation of the sporulation was determined by direct microscopic observations and by analyzing the presence of δ-endotoxins in cell pellets. Cry-I type prtoeins could be detected late in the stationary phase , during and after sporulation.

### EXAMPLE 9. IDENTIFICATION OF NOVEL VIP3-LIKE GENES BY HYBRIDIZATION

To identify *Bacillus* containing genes related to the VIP3A(a) from isolate AB88, a collection of *Bacillus* isolates was screened by hybridization. Cultures of 463 *Bacillus* strains were grown in microtiter wells until sporulation. A 96-pin colony stampel was used to transfer the cultures to 150 mm plates containing L-agar. Inoculated plates were kept at 30°C for 10 hours, then at 4°C overnight. Colonies were blotted onto nylon filters and probed with a 1.2Kb *Hin*dIII VIP3A(a) derived fragment. Hybridization was performed overnight at 62°C using hybridization conditions of Maniatis *et al*. Molecular Cloning: A Laboratory Manual (1982). Filters were washed with 2xSSC/0.1% SDS at 62°C and exposed to X-ray film.

Of the 463 *Bacillus* strains screened, 60 contain VIP3-like genes that could detected by hybridization. Further characterization of some of them (AB6 and AB426) showed that their supernatants contain a BCW insecticidal protein similar to the Vip3 protein that are active against black cutworm.

### EXAMPLE 10. CHARACTERIZATION OF A B. thuringiensis STRAIN M2194 CONTAINING A CRYPTIC VIP3-LIKE GENE

A *B. thuringiensis* strain, designated M2194, was shown to contain VIP3-like gene(s) by colony hybridization as described in Example 8. The M2194 VIP3 like gene is considered cryptic since no expression can be detected throughout the bacterial growth phases either by immunoblot analysis using polyclonal antibodies raised against the VIP3A(a) protein isolated from AB88 or by bioassay as described in Example 2.
Antiserum against purified VIP3A(a) insecticidal protein was produced in rabbits. Nictrocellulose-bound protein (50 µg) was dissolved in DMSO and emulsified with Freund's complete adjuvant (Difco). Two rabbits were given subcutaneous injections each month for three month. They were bled 10 days after the second and third injection and the serum was recovered from the blood sample [Harlow et al (1988) in : Antibodies: A Laboratory Manual (Cold Spring Harbor Lab Press, Plainview, NY)].

The M2194 VIP3-like gene was cloned into pKS by following the protocol described in Example 3, which created pCIB7108. *E. coli* containing pCIB7108 which comprises the M2194 VIP3 gene were active against black cutworm demonstrating that the gene encodes a functional protein with insecticidal activity. The plasmid pCIB7108 has been deposited with the Agricultural Research Service Patent Culture Collection (NRRL) and given Accession No. NRRL B-21438.

### EXAMPLE 11. INSECTICIDAL ACITIVITY OF VIP3A PROTEINS

The activity spectrum of VIP3A insecticidal proteins was qualitatively determined in insect bioassays in which recombinant *E coli* carrying the VIP*A genes were fed to larvae. In these assays, cells carrying the VIP3A(a) and VIP3A(b) genes were insecticidal to *Agrotis ipsilon, Spodoptera frugiperda, Spodoptera exigua, Heliothis virescens* and *Helicoverpa zea.* Under the same expermimental conditions, bacterial extracts containing VIP3A proteins did not show any activity against *Ostrinia nubilalis.*

| Effect of VIP*A insecticidal proteins on *Agrotis ipsilon* larvae | |
|---|---|
| Treatment | (%) Mortality |
| TB medium | 5 |
| AB88 Supernatant | 100 |
| Ab424 Supernatant | 100 |
| Buffer | 7 |
| *E coli* pKS | 10 |
| *E coli* pCIB7104 (AB88) | 100 |
| *E coli* pCIB7105 (AB88) | 100 |
| *E coli* pCIB7106 (AB424) | 100 |
| *E coli* pCIB7107 (AB424) | 100 |

| Effect of VIP3A insecticidal proteins on lepidopteran insect larvae | | |
|---|---|---|
| Treatment | Insect | (%) Mortality |
| *E coli* pKS | BCW | 10 |
| | FAW | 5 |
| | BAW | 10 |
| | TBW | 8 |
| | CEW | 10 |
| | ECB | 5 |
| | | |
| *E coli* pCIB7105 | | |
| *E coli* pCIB7107 | BCW | 100 |
| | FAW | 100 |
| | BAW | 100 |
| | TBW | 100 |
| | CEW | 50 |
| | ECB | 10 |
| BCW = Black Cut Worm; FAW = Fall Army Worm; BAW = Beet Army Worm; TBW = Tobacco Bud Worm; CEW = Corn Ear Worm; ECB = European Corn Borer | | |

### EXAMPLE 12. ISOLATION AND BIOLOGICAL ACTIVITY OF OTHER BACILLUS SP.

Other *Bacillus* species have been isolated which produce proteins with insecticidal activity during vegetative growth. These strains were isolated from environmental samples by standard methodologies. Isolates were prepared for bioassay and assayed as described in Examples 1 and 2 respectively. Isolates which produced insecticidal proteins during vegetative growth with activity against *Agrotis ipsilon* in the bioassay are tabulated below. No correlation was observed between the presence of a δ-endotoxin crystal and vegetative insecticidal protein production.

| Bacillus isolate | Presence of δ-endotoxin crystal | Percent mortality |
|---|---|---|
| AB6 | + | 100 |
| AB53 | - | 80 |
| AB88 | + | 100 |
| AB195 | - | 60 |
| AB211 | - | 70 |
| AB217 | - | 83 |
| AB272 | - | 80 |
| AB279 | - | 70 |
| AB289 | + | 100 |
| AB292 | + | 80 |
| AB294 | - | 100 |
| AB300 | - | 80 |
| AB359 | - | 100 |

Isolates AB289, AB294 and AB359 have been deposited in the Agricultural Research Service, Patent Culture Collection (NRRL), Northern Regional Research Center, 1815 North University Street, Peoria II 61604, USA and given the Accession Numbers NRRL B-21227, NRRL B-21229, and NRRL B-21226 respectively.

*Bacillus* isolates which produce insecticidal proteins during vegetative growth with activity against *Diabrotica virgifera virgifera* are tabulated below.

| Bacillus isolate | Presence of δ-endotoxin crystal | Percent mortality |
|---|---|---|
| AB52 | - | 50 |
| AB59 | - | 71 |
| AB68 | + | 60 |
| AB78 | - | 100 |
| AB122 | - | 57 |
| AB218 | - | 64 |
| AB256 | - | 64 |

Isolates AB59 and AB256 have been deposited in the Agricultural Research Service, Patent Culture Collection (NRRL), Northern Regional Research Center, 1815 North University Street, Peoria Illinois 61604, USA, and given the Accession Numbers NRRL B-21228 and NRRL B-21230, respectively.

The following deposits have been made at Agricultural Research Service, Patent Culture Collection (NRRL), Northern Regional Research Center, 1815 North University Street, Peoria, Illinois 61604, USA:

| Strain designation | Deposition Number | Deposition Date |
|---|---|---|
| 1. *E. coli* PL2 | NRRL B-21221 | March 09, 1994 |
| 2. *E. coli* PL2 | NRRL B-21221N | September 02, 1994 |
| 3. *E. coli* pCIB6022 | NRRL B-21222 | March 09, 1994 |
| 4. *E. coli* pCIB6023 | NRRL B-21223 | March 09, 1994 |
| 5. *E. coli* pCIB6023 | NRRL B-21223N | September 02, 1994 |
| 6. *Bacillus thuringiensis* HD73-78VIP | NRRL B-21224 | March 09, 1994 |
| 7. *Bacillus thuringiensis* AB88 | NRRL B-21225 | March 09, 1994 |
| 8. *Bacillus thuringiensis* AB359 | NRRL B-21226 | March 09, 1994 |
| 9. *Bacillus thuringiensis* AB289 | NRRL B-21227 | March 09, 1994 |
| 10. *Bacillus* sp. AB59 | NRRL B-21228 | March 09, 1994 |
| 11. *Bacillus* sp. AB294 | NRRL B-21229 | March 09, 1994 |
| 12. *Bacillus* sp. AB256 | NRRL B-21230 | March 09, 1994 |
| 13. *E. coli* P5-4 | NRRL B-21059 | March 18, 1993 |
| 14. *E. coli* P3-12 | NRRL B-21061 | March 18, 1993 |
| 15. *Bacillus cereus* AB78 | NRRL B-21058 | March 18, 1993 |
| 16. *Bacillus thuringiensis* AB6 | NRRL B-21060 | March 18, 1993 |
| 17. *E. coli* pCIB6202 | NRRL B-21321 | September 02, 1994 |
| 18. *E. coli* pCIB7100 | NRRL B-21322 | September 02, 1994 |
| 19. *E. coli* pCIB7101 | NRRL B-21323 | September 02, 1994 |
| 20. E. *coli* pCIB7102 | NRRL B-21324 | September 02, 1994 |
| 21. E. *coli* pCIB7103 | NRRL B-21325 | September 02, 1994 |
| 22. *E. coli* pCIB7104 | NRRL B-21422 | March 24, 1995 |
| 23. E. *coli* pCIB7107 | NRRL B-21423 | March 24, 1995 |
| 24. *E. coli* pCIB7108 | NRRL B-21438 | May 05, 1995 |
| 25. *Bacillus thuringiensis* AB424 | NRRL B-21439 | May 05, 1995 |

## Claims

1. A DNA molecule encoding as vegetative insecticidal protein isolatable during the vegetative growth phase of *Bacillus spp.,* wherein said protein is isolatable from liquid culture media, and wherein said protein is encoded by a nucleotide sequence that hybridizes to a nucleotide sequence of SEQ ID NOs: 1, 3 or 4 at 65 °C in a buffer comprising 7 % SDS and 0.5 M sodium phosphate.

2. The DNA molecule according to claim 1 encoding a protein as defined by SEQ ID NO: 2 or SEQ ID NO: 5.

3. The DNA molecule according to claim 2 having the nucleotide sequence given in SEQ ID NO: 1, SEQ ID NO: 3, or SEQ ID NO: 4.

4. The DNA molecule according to claim 1 which comprises a nucleotide sequence that has been wholly or partially optimized for expression in a plant by utilizing plant preferred codons.

5. The DNA molecule according to claim 4 having the nucleotide sequence given in SEQ ID NO: 3.

6. The DNA molecule according to claim 1 which comprises a nucleotide sequence that has been wholly or partially optimized for expression in a microorganism by utilizing host preferred codons.

7. A DNA molecule according to claim 1 obtainable by a process comprising
a) obtaining a DNA molecule comprising a nucleotide sequence encoding a vegetative insecticidal protein; and
b) hybridizing said DNA molecule with an oligonucleotide probe comprising a contiguous portion of the coding sequence for the said insect-specific protein at least 10 nucleotides in length obtainable from a DNA molecule defined in SEQ ID NO: 1, SEQ ID NO: 3, or SEQ ID NO: 4; and
c) isolating said hybridized DNA.

8. An expression cassette comprising a DNA molecule according to any one of claims 1-7 operably linked to plant expression sequences including the transcriptional and translational regulatory signals necessary for expression of the associated DNA constructs in a host organism and optionally further regulatory sequences.

9. An expression cassette according to claim 8, **characterized in that** the host organism is a plant.

10. A vector molecule comprising an expression cassette according to claim 8.

11. A host organism comprising a DNA molecule according to any one of claims 1 to 7, an expression cassette comprising said DNA molecule, or a vector molecule comprising said expression cassette stably incorporated into the genome of the host organism.

12. The host organism of claim 11 selected from the group consisting of plant and insect cells, bacteria, yeast, baculoviruses, protozoa, nematodes and algae.

13. The host organism according to claim 11 which is a microorganism transformed with an expression cassette according to any one of claims 8 or 9 or a vector molecule according to claim 10, **characterized in that** said microorganism is preferably a microorganism that multiplies on plants.

14. The host organism according to claim 13, **characterized in that** the microorganism is
a. a root colonizing bacterium or a Pseudomonas strain;
b. *Bacillus thuringiensis* AB424 deposited under the Accession Number NRRL B-21439.

15. A transgenic plant including parts as well as progeny and seed thereof comprising a DNA molecule according to any one of claims 1 to 7 or an expression cassette according to claim 9, stably incorporated into the plant genome.

16. The plant according to claim 15 expressing a protein of SEQ ID NO: 2 or SEQ ID NO: 5.

17. The plant according to claim 16 further expressing a second distinct insect control principle such as a Bt δ-endotoxin.

18. The plant according to any one of claims 15-17 which is selected from the group consisting of maize, sorghum, wheat, sunflower, cotton, rice, soybean, barley and oil seed rape.

19. The plant according to claim 18 which is a maize plant.

20. The plant according to claim 18 which is a cotton plant.

21. The plant according to any one of claims 15 to 20 which is a hybrid plant.

22. Seed of a plant according to any one of claims 15 to 20, which expresses a protein encoded by a nucleotide sequence of claim 1, treated with a seed protecting coating.

23. A method for isolating a DNA molecule according to claim 1, said method comprising:
a) obtaining a DNA molecule comprising a nucleotide sequence encoding a vegetative insecticidal protein;
b) hybridizing said DNA molecule with an oligonucleotide probe comprising a contiguous portion of the coding sequence for the said insect-specific protein at least 10 nucleotides in length obtainable from a DNA molecule defined by SEQ ID NO: 1, SEQ ID NO: 3, or SEQ ID NO: 4 and
c) isolating hybridized DNA.

24. A method of increasing insect target range **characterized in that** an expression cassette according to claim 8 is expressed in a plant together with at least one second insecticidal protein that is different from the vegetative insecticidal protein encoded by said expression cassette

25. The method according to claim 24 **characterized in that** the second insecticidal protein is selected from the group consisting of *Bt* δ-endotoxins, protease inhibitors, lectins, α-amylases and peroxidises.

26. A method of protecting plants against damage caused by an insect pest comprising planting a plant according to any one of claims 15 to 21 transgenic for a DNA molecule according to any one of claims 1 to 7 encoding and expressing a vegetative insecticidal protein encoded by a nucleotide sequence of claim 1.

27. A method of producing a plant or plant cell expressing a vegetative insecticidal protein comprising transforming the said plant or plant cell with an expression cassette according to claim 9 or a vector molecule according to claim 10.

## Patentansprüche

1. DNA-Molekül, das für ein vegetatives insektizides Protein, das während der vegetativen Wachstumsphase von *Bacillus spp.* isolierbar ist, codiert, wobei das Protein aus flüssigen Kulturmedien isolierbar ist und wobei das Protein von einer Nukleotidsequenz codiert wird, die bei 65°C in einem Puffer, der 7% SDS und 0,5 M Natriumphosphat umfasst, mit einer Nukleotidsequenz von SEQ ID NO: 1, 3 oder 4 hybridisiert.

2. DNA-Molekül nach Anspruch 1, das für ein Protein gemäß SEQ ID NO: 2 oder SEQ ID NO: 5 codiert.

3. DNA-Molekül nach Anspruch 2 mit der in SEQ ID NO: 1, SEQ ID NO: 3 oder SEQ ID NO: 4 angegebenen Nukleotidsequenz.

4. DNA-Molekül nach Anspruch 1, das eine Nukleotidsequenz umfasst, die durch Verwendung von von Pflanzen bevorzugten Codons ganz oder teilweise für die Expression in einer Pflanze optimiert worden ist.

5. DNA-Molekül nach Anspruch 4 mit der in SEQ ID NO: 3 angegebenen Nukleotidsequenz.

6. DNA-Molekül nach Anspruch 1, das eine Nukleotidsequenz umfasst, die durch Verwendung von vom Wirt bevorzugten Codons ganz oder teilweise für die Expression in einem Mikroorganismus optimiert worden ist.

7. DNA-Molekül nach Anspruch 1, das durch ein Verfahren erhältlich ist, das Folgendes umfasst:
a) Gewinnen eines DNA-Moleküls, das eine Nukleotidsequenz, die für ein vegetatives insektizides Protein codiert, umfasst; und
b) Hybridisieren des DNA-Moleküls mit einer Oligonukleotidsonde, die einen durchgehenden Abschnitt der Codiersequenz für das insektenspezifische Protein mit einer Länge von mindestens 10 Nukleotiden, erhältlich von einem in SEQ ID NO: 1, SEQ ID NO: 3 oder SEQ ID NO: 4 definierten DNA-Molekül, umfasst; und
c) Isolieren der hybridisierten DNA.

8. Expressionskassette, umfassend ein DNA-Molekül nach einem der Ansprüche 1-7 in operativer Verknüpfung mit pflanzlichen Expressionssequenzen einschließlich den für die Expression der assoziierten DNA-Konstrukte in einem Wirtsorganismus erforderlichen Transkriptions- und Translationsregulationssignalen sowie gegebenenfalls weiteren Regulationssequenzen.

9. Expressionskassette nach Anspruch 8, **dadurch gekennzeichnet, dass** es sich bei dem Wirtsorganismus um eine Pflanze handelt.

10. Vektormolekül, umfassend eine Expressionskassette nach Anspruch 8.

11. Wirtsorganismus, umfassend ein DNA-Molekül nach einem der Ansprüche 1 bis 7, Expressionskassette, umfassend das DNA-Molekül, oder Vektormolekül, umfassend die Expressionskassette, stabil in das Genom des Wirtsorganismus integriert.

12. Wirtsorganismus nach Anspruch 11, ausgewählt aus der Gruppe bestehend aus Pflanzen- und Insektenzellen, Bakterien, Hefe, Baculoviren, Protozoen, Nematoden und Algen.

13. Wirtsorganismus nach Anspruch 11, bei dem es sich um einen mit einer Expressionskassette nach einem der Ansprüche 8 oder 9 oder mit einem Vektormolekül nach Anspruch 10 transformierten Mikroorganismus handelt, **dadurch gekennzeichnet, dass** es sich bei dem Mikroorganismus vorzugsweise um einen Mikroorganismus, der sich auf Pflanzen vermehrt, handelt.

14. Wirtsorganismus nach Anspruch 13, **dadurch gekennzeichnet, dass** es sich bei dem Mikroorganismus um
a. ein wurzelkolonisierendes Bakterium oder einen Pseudomonas-Stamm;
b. *Bacillus thuringiensis* AB424, der unter der Eintragsnummer NRRL B-21439 hinterlegt ist,
handelt.

15. Transgene Pflanze einschließlich Teile sowie Nachkommen und Samen davon, umfassend ein DNA-Molekül nach einem der Ansprüche 1 bis 7 oder eine Expressionskassette nach Anspruch 9, stabil in das Pflanzengenom integriert.

16. Pflanze nach Anspruch 15, die ein Protein mit der SEQ ID NO: 2 oder SEQ ID NO: 5 exprimiert.

17. Pflanze nach Anspruch 16, die weiterhin ein zweites, unterschiedliches Insektenbekämpfungsprinzip, wie ein *Bt*-δ-Endotoxin exprimiert.

18. Pflanze nach einem der Ansprüche 15-17, ausgewählt aus der Gruppe bestehend aus Mais, Sorgum, Weizen, Sonnenblume, Baumwolle, Reis, Sojabohne, Gerste und Raps.

19. Pflanze nach Anspruch 18, bei der es sich um eine Maispflanze handelt.

20. Pflanze nach Anspruch 18, bei der es sich um eine Baumwollpflanze handelt.

21. Pflanze nach einem der Ansprüche 15 bis 20, bei der es sich um eine Hybridpflanze handelt.

22. Saatgut einer Pflanze nach einem der Ansprüche 15 bis 20, die ein von einer Nukleotidsequenz nach Anspruch 1 codiertes Protein exprimiert, behandelt mit einer das Saatgut schützenden Beschichtung.

23. Verfahren zum Isolieren eines DNA-Moleküls nach Anspruch 1, wobei das Verfahren Folgendes umfasst:
a) Gewinnen eines DNA-Moleküls, das eine Nukleotidsequenz, die für ein vegetatives insektizides Protein codiert, umfasst; und
b) Hybridisieren des DNA-Moleküls mit einer Oligonukleotidsonde, die einen durchgehenden Abschnitt der Codiersequenz für das insektenspezifische Protein mit einer Länge von mindestens 10 Nukleotiden, erhältlich von einem in SEQ ID NO: 1, SEQ ID NO: 3 oder SEQ ID NO: 4 definierten DNA-Molekül, umfasst; und
c) Isolieren der hybridisierten DNA.

24. Verfahren zur Erweiterung des Zielinsektenspektrums, **dadurch gekennzeichnet, dass** eine Expressionskassette nach Anspruch 8 gemeinsam mit mindestens einem zweiten insektiziden Protein, das sich von dem von der Expressionskassette codierten vegetativen insektiziden Protein unterscheidet, in einer Pflanze exprimiert wird.

25. Verfahren nach Anspruch 24, **dadurch gekennzeichnet, dass** das zweite insektizide Protein aus der Gruppe *Bt*-δ-Endotoxine, Proteasehemmer, Lektine, α-Amylasen und Peroxidasen ausgewählt ist.

26. Verfahren zum Schützen von Pflanzen gegen durch ein Schadinsekt verursachten Schaden, umfassend das Pflanzen einer Pflanze nach einem der Ansprüche 15 bis 21, die für ein DNA-Molekül nach einem der Ansprüche 1 bis 7, das für ein vegetatives insektizides Protein, das von einer Nukleotidsequenz nach Anspruch 1 codiert wird, codiert und dieses exprimiert, transgen ist.

27. Verfahren zur Herstellung einer Pflanze oder Pflanzenzelle, die ein vegetatives insektizides Protein exprimiert, umfassend das Transformieren der Pflanze oder Pflanzenzellen mit einer Expressionskassette nach Anspruch 9 oder mit einem Vektormolekül nach Anspruch 10.

## Revendications

1. Molécule d'ADN codant pour une protéine insecticide végétative pouvant être isolée pendant la phase de croissance végétative de *Bacillus spp.,* ladite protéine pouvant être isolée d'un milieu de culture liquide, et ladite protéine étant codée par une séquence nucléotidique qui s'hybride à une séquence nucléotidique SEQ ID NO:1, 3 ou 4 à 65°C dans un tampon contenant 7 % de SDS et du phosphate de sodium 0,5 M.

2. Molécule d'ADN selon la revendication 1, codant pour une protéine telle que définie par SEQ ID NO:2 ou SEQ ID NO:5.

3. Molécule d'ADN selon la revendication 2, ayant la séquence nucléotidique présentée dans SEQ ID NO:1, SEQ ID NO:3 ou SEQ ID NO:4.

4. Molécule d'ADN selon la revendication 1, qui comprend une séquence nucléotidique qui a été en totalité ou en partie optimisée pour une expression dans une plante par utilisation de codons préférés par les plantes.

5. Molécule d'ADN selon la revendication 4, ayant la séquence nucléotidique présentée dans SEQ ID NO:3.

6. Molécule d'ADN selon la revendication 1, qui comprend une séquence nucléotidique qui a été en totalité ou en partie optimisée pour une expression dans un microorganisme par utilisation de codons préférés par l'hôte.

7. Molécule d'ADN selon la revendication 1, pouvant être obtenue par un procédé comprenant :
a) l'obtention d'une molécule d'ADN comprenant une séquence nucléotidique codant pour une protéine insecticide végétative ; et
b) l'hybridation de ladite molécule d'ADN avec une sonde oligonucléotidique comprenant une portion contiguë de la séquence codante pour ladite protéine spécifique d'insecte, ayant une longueur d'au moins 10 nucléotides, pouvant être obtenue à partir d'une molécule d'ADN définie dans SEQ ID NO:1, SEQ ID NO:3 ou SEQ ID NO:4 ; et
c) l'isolement dudit ADN hybridé.

8. Cassette d'expression comprenant une molécule d'ADN selon l'une quelconque des revendications 1 à 7 liée d'une manière opérationnelle à des séquences d'expression dans les plantes, comprenant des signaux régulateurs de transcription et de traduction, nécessaires à l'expression des constructions d'ADN associées dans un organisme hôte, et en option des séquences régulatrices supplémentaires.

9. Cassette d'expression selon la revendication 8, **caractérisée en ce que** l'organisme hôte est une plante.

10. Molécule vecteur comprenant une cassette d'expression selon la revendication 8.

11. Organisme hôte comprenant une molécule d'ADN selon l'une quelconque des revendications 1 à 7, une cassette d'expression comprenant ladite molécule d'ADN, ou une molécule vecteur comprenant ladite cassette d'expression incorporée d'une manière stable dans le génome de l'organisme hôte.

12. Organisme hôte de la revendication 11, choisi dans le groupe consistant en les cellules de plantes et d'insectes, les bactéries, les levures, les baculovirus, les protozoaires, les nématodes et les algues.

13. Organisme hôte selon la revendication 11, qui est un microorganisme transformé par une cassette d'expression selon l'une quelconque des revendications 8 ou 9 ou une molécule vecteur selon la revendication 10, **caractérisé en ce que** ledit microorganisme est de préférence un microorganisme qui se multiplie sur les plantes.

14. Organisme hôte selon la revendication 13, **caractérisé en ce que** le microorganisme est
a. une bactérie de colonisation des racines ou une souche de Pseudomonas ;
b. *Bacillus thuringiensis* AB424, déposé sous le Numéro d'Accession NRRL B-21439.

15. Plante transgénique, y compris les parties et la descendance et les graines de cette dernière, comprenant une molécule d'ADN selon l'une quelconque des revendications 1 à 7 ou une cassette d'expression selon la revendication 9, incorporée d'une manière stable dans le génome de la plante.

16. Plante selon la revendication 15, exprimant une protéine ayant la séquence SEQ ID NO:2 ou SEQ ID NO:5.

17. Plante selon la revendication 16, qui exprime en outre un deuxième principe distinct de maîtrise des insectes, tel que l'endotoxine-δ *Bt*.

18. Plante selon l'une quelconque des revendications 15 à 17, qui est choisie dans le groupe consistant en le maïs, le sorgho, le blé, le tournesol, le coton, le riz, le soja, l'orge et le colza oléagineux.

19. Plante selon la revendication 18, qui est une plante de maïs.

20. Plante selon la revendication 18, qui est une plante de coton.

21. Plante selon l'une quelconque des revendications 15 à 20, qui est une plante hybride.

22. Graine d'une plante selon l'une quelconque des revendications 15 à 20, qui exprime une protéine codée par une séquence nucléotidique de la revendication 1, traitée avec un enrobage de protection des graines.

23. Procédé pour isoler une molécule d'ADN selon la revendication 1, ledit procédé comprenant :
a) l'obtention d'une molécule d'ADN comprenant une séquence nucléotidique codant pour une protéine insecticide végétative ;
b) l'hybridation de ladite molécule d'ADN avec une sonde oligonucléotidique comprenant une portion contiguë de la séquence codante pour ladite protéine spécifique d'insecte, ayant une longueur d'au moins 10 nucléotides, pouvant être obtenue à partir d'une molécule d'ADN définie dans SEQ ID NO:1, SEQ ID NO:3 ou SEQ ID NO:4 ; et
c) l'isolement dudit ADN hybridé.

24. Procédé pour augmenter la gamme cible d'insectes, **caractérisé en ce qu'**une cassette d'expression selon la revendication 8 est exprimée dans une plante en même temps qu'au moins une deuxième protéine insecticide, qui est différente de la protéine insecticide végétative codée par ladite cassette d'expression.

25. Procédé selon la revendication 24, **caractérisé en ce que** la deuxième protéine insecticide est choisie dans le groupe consistant en les endotoxines-δ *Bt*, les inhibiteurs des protéases, les lectines, les α-amylases et les peroxydases.

26. Procédé pour protéger les plantes contre les dommages provoqués par un insecte ravageur, comprenant la plantation d'une plante selon l'une quelconque des revendications 15 à 21, transgénique pour une molécule d'ADN selon l'une quelconque des revendications 1 à 7, codant pour une protéine insecticide végétative codée par une séquence nucléotidique de la revendication 1 et exprimant cette protéine.

27. Procédé de production d'une plante ou d'une cellule végétale exprimant une protéine insecticide végétative, comprenant la transformation de ladite plante ou de ladite cellule végétale avec une cassette d'expression selon la revendication 9 ou une molécule vecteur selon la revendication 10.
